# FASCICULE DE BREVET EUROPEEN

(11) **EP 0 718 299 B1**
(45) Date de publication et mention de la délivrance du brevet: **05.04.2000**
(21) Numéro de dépôt: 95402865.0
(22) Date de dépôt: 19.12.1995
(51) Int. Cl.: C07D 487/04, C07D 495/14, A61K 31/40

(54) **Ethers d'oximes tricycliques, leur procédé de préparation et les compositions pharmaceutiques qui les contiennent**
Trizyklische Oximether, Verfahren zu ihrer Herstellung und diese enthaltende pharmazeutische Zusammensetzungen
Tricyclic oxime ethers process for their preparation and pharmaceutical compositions containing them

(30) Priorité: 22.12.1994 FR 9415431
(43) Date de publication de la demande: 26.06.1996
(73) Titulaire: ADIR ET COMPAGNIE, 92415 Courbevoie Cédex (FR)
(72) Inventeur: Rault, Sylvain, F-14370 Moult (FR); Robba, Max, F-75004 Paris (FR); Lancelot, Jean-Charles, F-14400 Le Bourg (FR); Prunier, Hervé, F-14000 Caen (FR); Renard, Pierre, F-78000 Versailles (FR); Pfeiffer, Bruno, F-95600 Eaubonne (FR); Guardiola-Lemaitre, Béatrice, F-92210 Saint-Cloud (FR); Rettori, Marie-Claire, F-92400 Courbevoie (FR)

(56) Documents cités:
- HETEROCYCLES, vol. 23, no. 6, 1985 pages 1471-1477, C. LAPORTE-WOJCIK ET AL 'Thienopyrrolizines. Synthesis of 4-imino (and 4-amino)-4H-thieno(2,3-b)pyrrolizines'
- TETRAHEDRON LETTERS., vol. 26, no. 19, 1985 OXFORD GB, pages 2305-2308, S. RAULT ET AL 'A convenient synthesis of new aminopyrroloindoles via an imminium salt'
- JOURNAL OF PHARMACEUTICAL SCIENCES, vol. 58, no. 1, 1969 pages 138-141, F. J. VILLANI ET AL 'Oximino ethers: Dialkylaminoalkyl derivatives'
- EUROPEAN JOURNAL OF MEDICINAL CHEMISTRY.CHIMICA THERAPEUTICA., vol. 26, no. 9, 1991 CHATENAY-MALABRY FR, pages 939-946, S. RAULT ET AL 'Synthèse et étude préliminaire de l'activité psychotrope d'alkylamino et iminopyrrolo(1,2-a)indoles'

## Description

La présente invention concerne de nouveaux éthers d'oximes tricycliques, leurs procédés de préparation et les compositions pharmaceutiques qui les contiennent.

On connait dans la littérature quelques exemples d'éthers d'oximes mono ou polycycliques parmi lesquels on peut notamment citer les brevets BAYER EP 544168 et EP 544169 qui revendiquent des éthers d'oximes répondant aux formules générales suivantes :

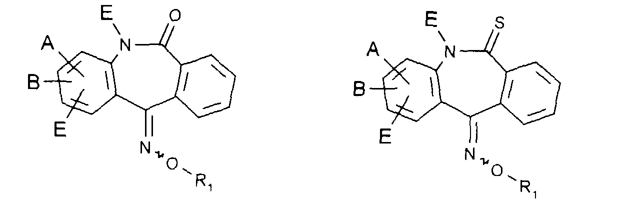

Ces composés sont décrits comme étant inhibiteurs de transcriptase reverse utilisables pour le traitement des affections à retrovirus comme le SIDA.

L'art antérieur décrit par ailleurs des dérivés iminopyrroloindoliques pour leurs propriétés psychostimulantes (European Journal of Medicinal Chemistry, 1991, 26, pp. 939-946).

La demanderesse a découvert de nouveaux éthers d'oximes à structure tricyclique entièrement originale qui se caractérisent de manière inattendue par une très forte affinité pour les récepteurs 5HT_{2C} et/ou 5HT₃ ainsi que pour les sites de recapture de la sérotonine. Cette haute affinité est associée à une remarquable sélectivité vis à vis des autres récepteurs sérotoninergiques.

Le potentiel et l'intérêt thérapeutique de composés se liant fortement aux récepteurs 5HT_{2C} et/ou 5HT₃ sont bien connus et l'on peut à ce sujet se référer utilement aux revues suivantes :
- G.J. KILPATRICK "5HT₃ receptors" (Medicinal Research Reviews, 1990, 10(4), p 441-475).
- D.HOYER et al "VII International Union of Pharmacology Classification of Receptor for 5-hydroxytryptamine (Serotonin)" (Pharmacological Reviews, 1994, 46 (2) pp 157-203).

Il en est de même pour les composés inhibant la recapture de la sérotonine, voir à ce sujet la revue de S.Z. LANGER et D. GRAHAM intitulée "Inhibitors of serotonin uptake" tirée de l'ouvrage "SEROTONIN from cell biology to pharmacology and therapeutics" (P.M. VANHOUTTE et al. (eds.) 1993 Klurver Academic Publisher. Pays Bas).

L'intérêt des composés de la présente invention est d'autant plus grand qu'ils agissent puissamment et simultanément au niveau de ces différents sites d'action ce qui leur confère un grand intérêt en thérapeutique.

La présente invention concerne plus particulièrement les composés de formule (I) : 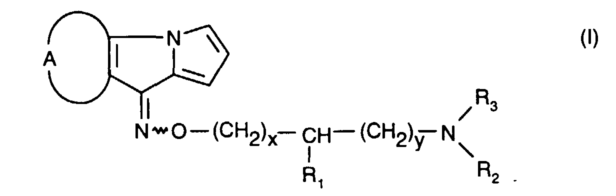 dans laquelle
- R₁ est choisi parmi :
   - hydrogène,
   - alkyle,
   - alcényle,
   - cycloalkyle,
   - cycloalkylalkyle dont la chaîne alkyle linéaire ou ramifiée comporte de 1 à 4 atomes de carbone,
   - hydroxy,
   - alkoxy,
   - phenyle éventuellement substitué,
   - phénylalkyle éventuellement substitué dont la chaîne alkyle linéaire ou ramifiée comporte de 1 à 4 atomes de carbone,
   - phénoxy éventuellement substitué, et
   - phénylalkoxy éventuellement substitué dont la chaîne alkyle linéaire ou ramifiée comporte de 1 à 4 atomes de carbone,
   ou bien R₁ forme avec R₂ et la chaîne 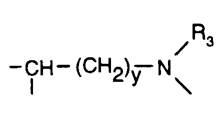 qui les porte un système cyclique azoté comportant de 5 à 8 sommets,
- R₂ et R₃ sont choisis chacun indépendamment l'un dé l'autre parmi :
   - hydrogène,
   - alkyle,
   - alcényle,
   - cycloalkyle,
   - indanyle éventuellement substitué,
   - cycloalkylalkyle dont la chaîne alkyle linéaire ou ramifiée comporte de 1 à 4 atomes de carbone,
   - phényle éventuellement substitué, et
   - phénylalkyle éventuellement substitué dont la chaîne alkyle linéaire ou ramifiée comporte de 1 à 4 atomes de carbone,
   ou bien R₂ et R₃ forment ensemble avec l'atome d'azote qui les porte un système hétérocyclique choisi parmi : 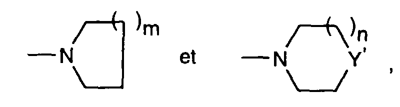 avec :
   m nombre entier pouvant prendre les valeurs 0, 1, 2, 3, ou 4,
   n nombre entier pouvant prendre les valeurs 0, 1, ou 2,
   Y' étant choisi parmi l'oxygène, le soufre et le groupement

      〉N-R₄,

      R₄ étant choisi parmi :
      - hydrogène,
      - alkyle, et
      - -(CH₂)_{σ}- phényle éventuellement substitué, avec σ nombre entier pouvant prendre les valeurs 0, 1, 2, 3, ou 4,
- x et y représentent des nombres entiers identiques ou différents pouvant prendre chacun indépendamment l'un de l'autre les valeurs 0, 1, 2, 3, ou 4,
- A représente :
   * soit un groupement de formule (α) : 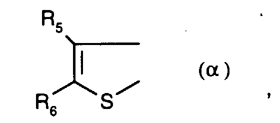 et forme ainsi avec le système hétérocyclique qui le porte un thiéno[2,3-d]pyrrolo[1,2-a]pyrrole de formule générale (I_{α}) : 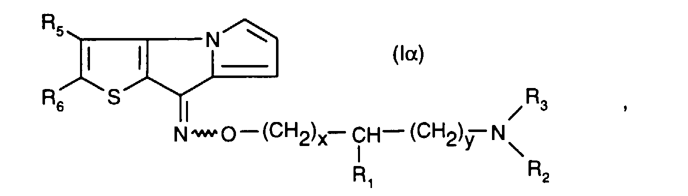 dans laquelle R₁, R₂, R₃, x, et y sont tels que définis précédemment et R₅ et R₆, identiques ou différents représentent indépendamment l'un de l'autre un groupement choisi parmi :
      - hydrogène,
      - alkyle,
      - hydroxy,
      - alkoxy,
      - halogène,
      - trifluorométhyle,
      - alkoxycarbonyle,
      - -(CH₂)ₚ- phényle éventuellement substitué avec p nombre entier pouvant prendre les valeurs 0, 1, 2, 3, ou 4, et
      - -O-(CH₂)ₚ'-phényle éventuellement substitué avec p' nombre entier pouvant prendre les valeurs 0, 1, 2, 3, ou 4,
   * soit un groupement de formule (β) : 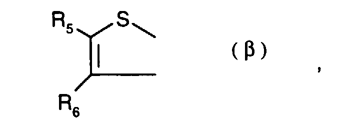 et forme ainsi avec le système hétérocyclique qui le porte un thiéno[3,2-d]pyrrolo[1,2-a]pyrrole de formule générale (I_{β}) : 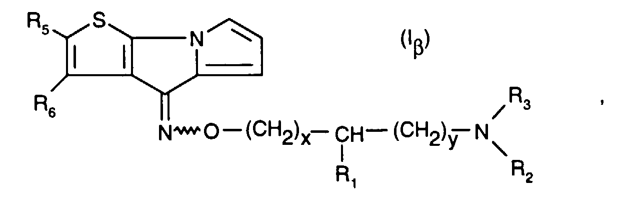 dans laquelle R₁, R₂, R₃, R₅, R₆, x et y sont tels que définis précédemment,
   * soit un groupement de formule (γ) : 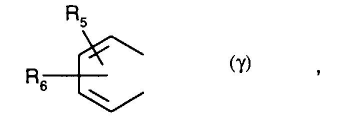 et forme ainsi avec le système hétérocyclique qui le porte un pyrrolo[1,2-a]indole de formule générale (Iγ) : 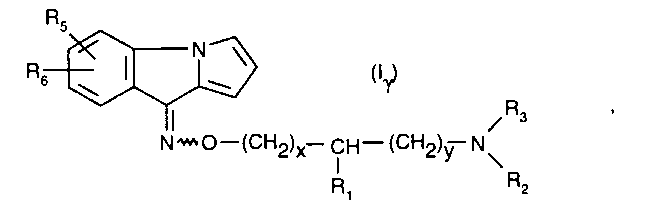
dans laquelle R₁, R₂, R₃, R₅, R₆, x et y sont tels que définis précédemment,
- leurs isomères cis ou trans au niveau de l'éther d'oxime,
- leur énantiomères ou diastéréoisomères, et
- leurs hydrates et/ou sels d'addition à un acide ou une base, pharmaceutiquement acceptables,
étant entendu que, sauf précision contraire :
- les termes "alkyle" et "alkoxy" représentent des groupements linéaires ou ramifiés possédant de 1 à 6 atomes de carbone,
- le terme "alcényle" représente un groupement carboné insaturé linéaire ou ramifié possédant de 2 à 6 atomes de carbone,
- le terme "cycloalkyle" représente un système carboné cyclique comportant de 3 à 8 sommets,
- les expressions "indanyle éventuellement substitué", "phényle éventuellement substitué", "phénoxy éventuellement substitué", "phénylalkyle éventuellement substitué", "-(CH₂)_{σ}-phényle éventuellement substitué", "-(CH₂)ₚ-phényle éventuellement substitué", "-O-(CH₂)_{p'}-phényle éventuellement substitué" et "phénylalkoxy éventuellement substitué" signifient que le radical phényle peut être éventuellement substitué par un ou plusieurs substituants identiques ou différents choisis parmi alkyle, alkoxy, hydroxy, halogène, trifluorométhyle, nitrile et nitro.

Parmi les acides que l'on peut utiliser pour former un sel d'addition pharmaceutiquement acceptable avec les composés de l'invention, on peut citer, à titre d'exemple et de façon non limitative, les acides chlorhydrique, sulfurique, phosphorique, tartrique, malique, maléique, succinique, fumarique, oxalique, méthanesulfonique, éthanesulfonique, camphorique, et citrique.

Parmi les bases que l'on peut utiliser pour former un sel d'addition pharmaceutiquement acceptable avec les composés de l'invention, on peut citer, à titre d'exemple et de façon non limitative, les hydroxydes de sodium, de potassium, de calcium, ou d'aluminium, les carbonates de métaux alcalins ou alcalinoterreux, et les bases organiques comme la triéthylamine, la benzylamine, la diéthanolamine, la tert-butylamine, la dicyclohexylamine, et l'arginine.

La présente invention s'étend également au procédé de préparation des composés de formule générale (I), caractérisé en ce que l'on cyclise avec de l'oxychlorure de phosphore en présence ou en absence d'un solvant, comme la N,N-diméthyl formamide (DMF), un composé de formule (II) : 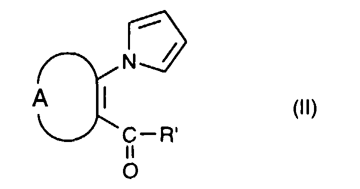 dans laquelle A est tel que défini dans la formule (I) et R' représente un radical hydroxy, alkoxy ou 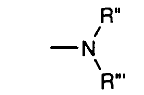 avec R" et R‴ différents de H, pouvant être des alkyles ou former ensemble avec l'atome d'azote qui les porte une amine cyclique choisie parmi pyrrolidine, pipéridine ou morpholine,
de manière à obtenir après cyclisation une cétone tricyclique de formule (III) : 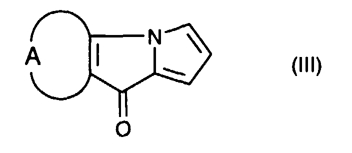 dans laquelle A est tel que défini précedemment, que l'on fait ensuite réagir avec de l'hydroxylamine de manière à obtenir l'oxime de formule (IV) : 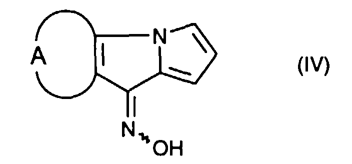 dans laquelle A est tel que défini précédemment et que l'on peut, si nécessaire et si on le désire, séparer en ses isomères E et Z, avant de le faire réagir avec un composé de formule (V) : 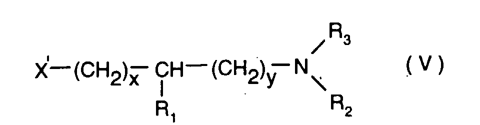 dans laquelle R_{1,} R₂, R₃, x et y sont tels que définis dans la formule (I) et X' représente un groupement partant pouvant être un halogène ou un tosylate, de manière à accéder au composé de formule (I) : 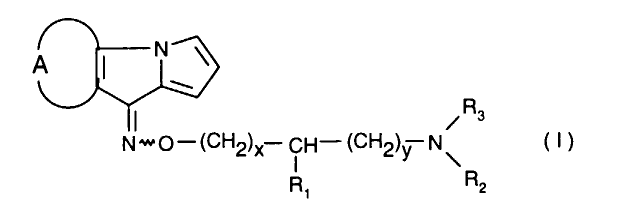 dans laquelle A, R₁, R₂, R₃, x et y sont tels que définis précédemment et que l'on peut si nécessaire et si on le désire séparer en ses isomères Z et E et en ses éventuels énantiomères ou diastéréoisomères, et/ou salifier avec un acide ou avec une base pharmaceutiquement acceptables.

La présente invention concerne également un procédé de préparation des composés de formule (I) caractérisé en ce que l'on cyclise avec de l'oxychlorure de phosphore en présence ou en absence d'un solvant, comme la DMF, un composé de formule ( II ) : 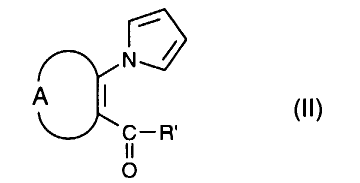 dans laquelle A est tel que défini dans la formule (I) et R' représente un radical hydroxy, alkoxy ou 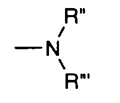 avec R" et R"' différents de H pouvant être des alkyles ou former ensemble, avec l'atome d'azote qui les porte une amine cyclique choisie parmi pyrrolidine, pipéridine ou morpholine,
de manière à obtenir après cyclisation une cétone tricyclique de formule (III) : 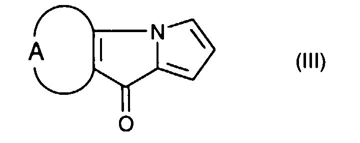 dans laquelle A est tel que défini précédemment que l'on fait ensuite réagir avec une amine de formule (VI) : 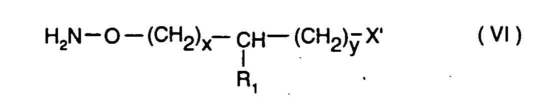 dans laquelle R_{1,} x et y sont tels que définis dans la formule (I) et X' représente un groupement partant comme un halogène ou un tosylate, de manière à obtenir un composé de formule (VII) : 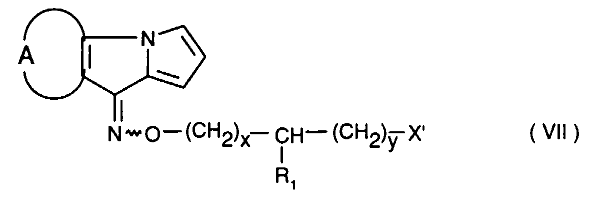 dans laquelle A, R₁, X', x et y sont tels que définis précédemment que l'on peut :
a) soit faire réagir avec une amine de formule (VIII) : 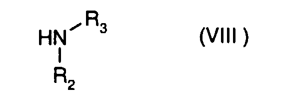 dans laquelle R₂ et R₃ sont tels que définis dans la formule (I) de manière à accéder au composé de formule (I) : 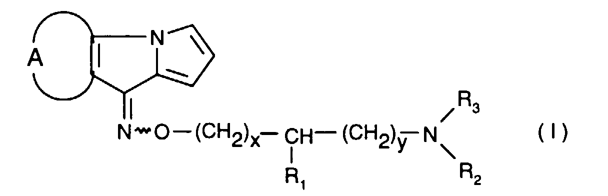 dans laquelle A, R₁, R₂, R₃, x et y sont tels que définis précédemment et que l'on peut si nécessaire et si on le désire séparer en ses isomères Z et E et en ses éventuels énantiomères ou diastéréoisomères et/ou salifier avec un acide ou une base pharmaceutiquement acceptable,
b) soit faire réagir avec du phtalimidure de potassium de manière à obtenir le phtalimide de formule (IX) : 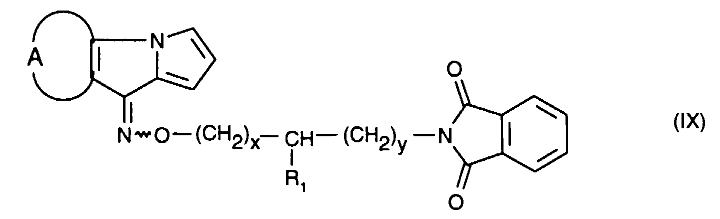 dans laquelle A, R₁, x et y sont tels que définis précédemment, que l'on hydrolyse ensuite en présence d'hydrazine pour accéder au composé de formule (I_{A}) : 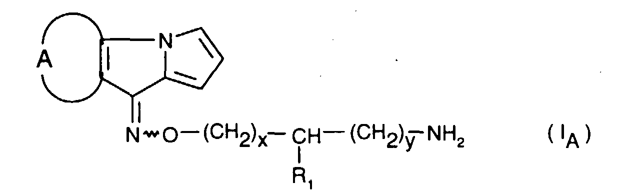 dans laquelle A, R₁, x et y sont tels que définis précédemment, cas particulier des composés de formule (I) pour lesquels R₂=R₃=H, que l'on peut si nécessaire et si on le désire séparer en ses isomères Z et E et en ses éventuels énantiomères ou diastéréoisomères, et/ou salifier avec un acide ou une base pharmaceutiquement acceptable,
composé de formule (I_{A}) que l'on peut si on le désire alkyler avec un composé de formule (X) :

X'-R₅ (X)

dans laquelle X' est tel que défini précédemment et R₅ possède la même signification que R₂ avec la réserve que R₅ ne peut représenter ni un hydrogène ni un phényle éventuellement substitué, de manière à accéder au composé de formule (I_{B}) : 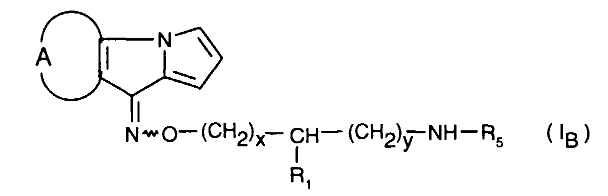 dans laquelle A, R₁, R₅, x et y sont tels que définis précédemment, cas particulier des composés de formule (I) que l'on peut si nécessaire et si on le désire séparer en ses isomères Z et E et en ses éventuels enantiomères ou diastéréoisomères et/ou salifier avec un acide ou avec une base pharmaceutiquement acceptables.

La présente invention concerne également un procédé de préparation des composés de formule (I_{C}) : 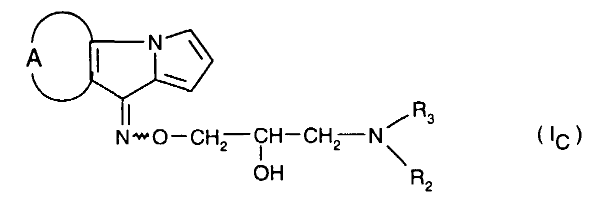 cas particulier des composés de formule générale (I) pour lesquels A, R₂ et R₃ sont tels que définis dans la formule (I) avec x=y=1 et R₁ représentant un groupement hydroxy, caractérisé en ce que l'on cyclise avec de l'oxychlorure de phosphore en présence ou en absence d'un solvant comme la DMF un composé de formule (II) : 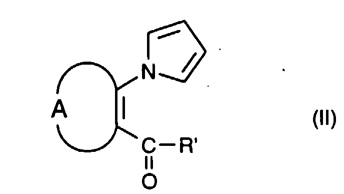 dans laquelle A est tel que défini précédemment et R' représente un radical hydroxy, alkoxy ou 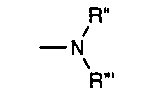 avec R" et R"' différents de H pouvant être des alkyles ou former ensemble, avec l'atome d'azote qui les porte, une amine cyclique choisie parmi pyrrolidine, pipéridine ou morpholine,
de manière à obtenir après cyclisation une cétone tricyclique de formule générale (III) : 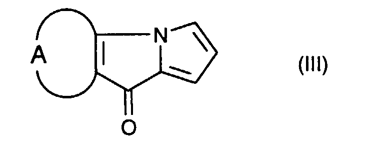 dans laquelle A est tel que défini précédemment, que l'on fait ensuite réagir avec de l'hydroxylamine de manière à obtenir l'oxime de formule générale (IV) : 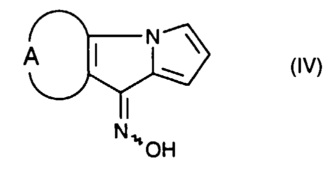 dans laquelle A est tel que défini précédemment et que l'on peut, si nécessaire et si on le désire, séparer en ses isomères E et Z avant de le faire réagir, après métallation par un hydrure métallique comme l'hydrure de sodium avec une épihalohydrine de formule générale (XI) : 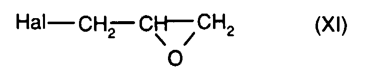 dans laquelle Hal représente un atome d'halogène, pour obtenir après réaction du composé intermédiaire ainsi formé avec une amine de formule générale (VIII) : 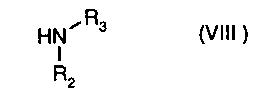 dans laquelle R₂ et R₃ sont tels que définis précédemment, un composé de formule (I_{C}) tel que défini précédemment, que l'on peut si nécessaire et si on le désire séparer en ses isomères Z et E et en ses éventuels énantiomères ou diastéréoisomères, et/ou transformer avec un acide ou avec une base en sels pharmaceutiquement acceptables.

Les composés de formule générale (I), (I_{A}), (I_{B}), (I_{C}) ainsi que les intermédiaires de synthèses utilisés dans les procédés précédemment décrits peuvent si nécessaire être purifiés suivant une ou plusieurs méthodes de purification choisies parmi la cristallisation, la chromatographie sur colonne de silice, la HPLC sur phase chirale ou non, l'extraction, la filtration et le passage sur charbon et/ou résine.

Les matières premières utilisées dans les procédés précédemment décrits sont, soit commerciales, soit aisément accessibles à l'homme de métier selon des procédés décrits dans la littérature ou explicités dans les préparations décrites ci-après.

Les composés de formule (I) possèdent des propriétés pharmacologiques très intéressantes.

La demanderesse a découvert que les composés de l'invention possèdent, et de manière très sélective, une très forte affinité pour les récepteurs sérotoninergiques 5HT₃ et/ou 5HT_{2C} ainsi que pour les sites de recapture de la sérotonine.

Outre cette très haute affinité clairement établie *in vitro* par des études de liaison aux récepteurs (determination de l'affinité), l'activité des composés de la présente invention a été également établie *in vivo* dans des modèles animaux comportementaux (exemple B et C de la présente demande).

Les composés de l'invention sont donc susceptibles d'être utilisés dans la prévention et le traitement de l'anxiété, de la dépression, du stress, des psychoses, des troubles obsessionnels compulsifs de la schizophrénie, des troubles du système nerveux central, de la migraine, des troubles de la mémoire, des troubles du comportement et de la prise alimentaire, de l'alcoolisme, de la douleur, ainsi que dans la prévention et le traitement du vomissement et des désordres de la vidange gastrique.

L'invention sétend aussi aux compositions pharmaceutiques renfermant comme principe actif au moins un des composés de formule (I) sous forme pure ou sous forme d'un mélange d'isomères ou un de ses sels d'addition avec un acide ou une base, pharmaceutiquement acceptables, en combinaison avec un ou plusieurs excipients ou véhicules pharmaceutiquement acceptables.

Parmi les compositions selon l'invention, on pourra citer, à titre d'exemples et de façon non limitative, celles qui conviennent pour l'administration orale, parentérale, oculaire, per ou transcutanée, nasale, rectale, perlinguale, ou respiratoire, et notamment les préparations injectables, les aérosols, les gouttes oculaires ou nasales, les comprimés, les comprimés sublinguaux, les gélules, les capsules, les tablettes, les glossettes, les suppositoires, les crèmes, les pommades, et les gels.

Les préparations ainsi obtenues se présentent généralement sous forme dosée et peuvent contenir selon les affections traitées, le poids, l'âge, et le sexe du malade de 0,01 à 100 mg de principe actif en prises de une à trois fois par jour, de préférence de 0,01 à 5 mg de principe actif, particulièrement de 0,1 à 5 mg, par exemple 1 mg.

Les exemples qui suivent illustrent l'invention et ne la limitent en aucune façon.

### Préparation 1 : 3-(pyrrol-1-yl)thiophén-2-carboxylate de méthyle

Ajouter à température ambiante par petites portions 20 g (0,127 moles) de 3-aminothiophén-2-carboxylate de méthyle à une solution de 16,80 g (0,127 moles) de 2,5-diméthoxy tétrahydrofurane dans 100 ml d'acide acétique.
Après 30 minutes d'agitation à température ambiante puis 90 minutes à 90°C l'acide acétique est éliminé sous pression réduite et le résidu repris par 100 ml de soude 2N et extrait à l'éther éthylique.
Après lavage à l'eau et décoloration sur charbon animal, la phase étherée est mise à sec pour fournir le composé du titre
Rendement : 68 %
Point de fusion : 60°C
Infra rouge : ν cm⁻¹(KBr) : 1700 (CO)

### Préparation 2 : acide 3-(pyrrol-1-yl)thiophén-2-carboxylique

Une solution de 20 g (0,096 moles) de 3-(pyrrol-1-yl)thiophén-2-carboxylate de méthyle dans un mélange de 150 ml de méthanol et 100 ml d'une solution acqueuse de soude 6N est chauffée au reflux sous agitation pendant 3 heures.
Le méthanol est éliminé sous pression réduite et le milieu réactionnel dilué avec 100 ml d'eau acidifiée et extrait par 500 ml d'éther. La phase éthérée est décantée, séchée puis concentrée sous vide, et le composé du titre recristallisé dans l'éther éthylique.
Rendement : 74 %
Point de fusion : 170°C
Infra rouge : ν cm⁻¹(KBr) : 1675 (CO), 2980 et 2520 (OH)

### Préparation 3 : 4-méthyl-3-(pyrrol-1-yl)thiophén-2-carboxylate de méthyle.

43,8 g (0,292 moles) de chlorhydrate de 4-chloropyridine et 38,59 g (0,292 mole) de 2,5-diméthoxy tetrahydrofurane sont agités 10 minutes à température ordinaire dans 600 ml de dioxane. 50 g (0,292 mole) de 4-méthyl-3-aminothiophén-2-carboxylate de méthyle sont ajoutés et la suspension est portée au reflux 3 heures. Le dioxane est éliminé sous pression réduite, le résidu est repris par 1 litre d'eau puis extrait par 1 litre d'éther éthylique. La phase étherée est lavée à l'eau, décantée, séchée sur sulfate de magnésium, décolorée au charbon animal et concentrée sous pression réduite, pour donner le composé du titre.
Rendement : 91 %
Point de fusion : 76°C
Infra rouge : ν cm⁻¹(KBr) : 1715 (CO)

### Préparations 4 à 8 :

En procédant de la même façon que pour les préparations 1 et 3 mais en utilisant les thiophène-carboxylates de méthyle convenablement substitués, on obtient les composés correspondant aux préparations 4 à 8.

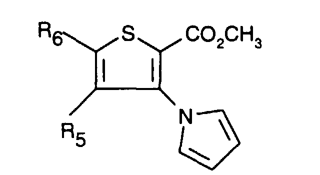

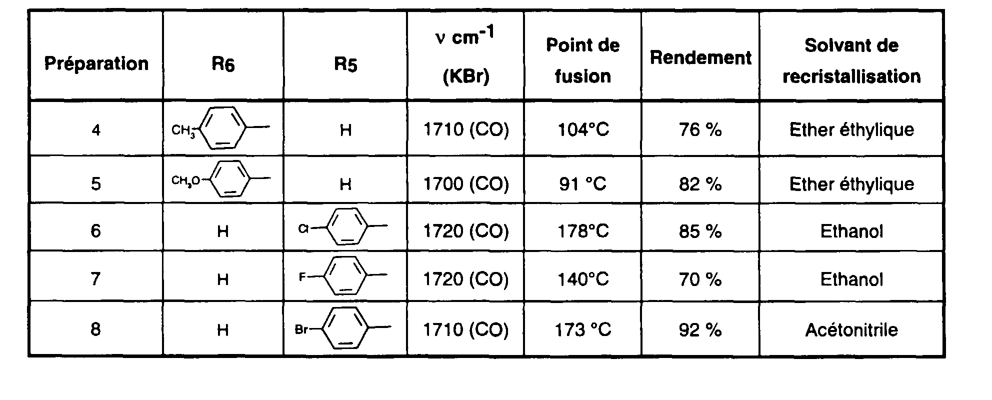

### Préparation 9 : 4,5-diméthyl-2-(pyrrol-1-yl)thiophén-3-carboxylate d'éthyle

En procédant comme pour la préparation 1, on obtient le composé du titre sous forme d'huile.
Rendement : 91 %
Infra rouge : ν cm⁻¹(KBr) : 1720 (C=O) 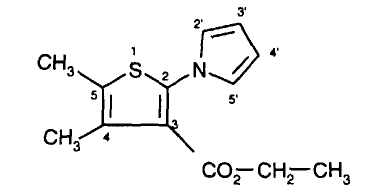 RMN ¹H : δ (ppm) (DMSO d₆) : 1,47 triplet (3H, CH₂CH₃) ; 2,57 singulet (3H, CH₃₍₄₎) ; 2,67 singulet (3H, CH₃₍₅₎) ; 4,47 quadruplet (2H, CH₂) ; 6,50 doublet de doublet (2H, H_{3'}, H_{4'}) ; 7,20 doublet de doublet (2H, H_{2'} H_{5'}).

### Préparation 10 : acide 4,5-diméthyl-2-(pyrrol-1-yl)thiophén-3-carboxylique

En procédant comme pour la préparation 2, on obtient le composé du titre.
Rendement : 84 %
Point de fusion : 156°C
Infra rouge : ν cm⁻¹(KBr) : 2530 (OH), 1670 (C=O) 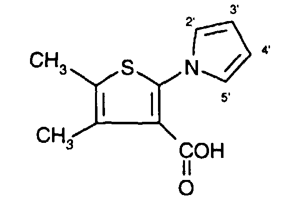 RMN ¹H : δ (ppm) (DMSO d₆) : 2,28 singulet (3H, CH₃₍₄₎) ; 2,50 singulet (3H, CH₃₍₅₎) ; 6,13 doublet de doublet (2H, H_{3'}, H_{5'}) ; 6,84 doublet de doublet (2H, H_{2'}, H_{5'}).

### Préparation 11 : N-[3-(pyrrol-1-yl)thiophén-2-ylcarbonyl]pyrrolidine

Porter au reflux pendant 7 heures une suspension de 10 g (0,048 mole) de 3-(pyrrol-1-yl) thiophén-2-carboxylate de méthyle dans 40 ml de pyrrolidine. La solution est versée dans 800 ml d'eau froide sous agitation et le précipité formé, essoré, lavé à l'eau froide, puis recristallisé dans l'éther éthylique.
Rendement : 88 %
Point de fusion : 110°C (éther éthylique)
Infra rouge : ν cm⁻¹ (KBr) : 1600 (C=O)

### Préparation 12 : N-[3-(pyrrol-1-yl)thiophén-2-ylcarbonyl]morpholine

En procédant de la même façon que pour la préparation 11, mais en utilisant la morpholine à la place de la pyrrolidine, on obtient le composé du titre sous forme de cristaux blancs.
Rendement: 84 %
Point de fusion : 100°C (éther éthylique)
Infra rouge : ν cm⁻¹ (KBr) : 1615 (C=O)

### Préparation 13 : N-[3-(pyrrol-1-yl)thiophén-2-ylcarbonyl]pipéridine

En procédant de la même façon que pour la préparation 11, mais en utilisant la pipéridine à la place de la pyrrolidine, on obtient le composé du titre sous forme de cristaux jaunes.
Rendement : 80 %
Point de fusion : 126°C (éther éthylique)
Infra rouge : ν cm⁻¹ (KBr) : 1625 (C=O)

### Préparation 14 : N-[3-(pyrrol-1-yl)-4-méthylthiophén-2-ylcarbonyl]pyrrolidine.

En procédant de la même façon que pour la préparation 11 mais en utilisant le 4-méthyl-3-(pyrrol-1-yl)thiophén-2-carboxylate de méthyle, on obtient le composé du titre sous forme de cristaux blancs.
Rendement : 83 %
Point de fusion : 178°C
Infra rouge : ν cm⁻¹(KBr) : 1610 (C=O)

### Préparation 15 à 19 :

En procédant de la même façon que pour la préparation 11 mais en remplaçant le 3-(pyrrol1-yl)thiophén-2-carboxylate de méthyle par les esters correspondant aux préparations 4 à 8, on obtient les composés correspondants aux préparations 15 à 19.

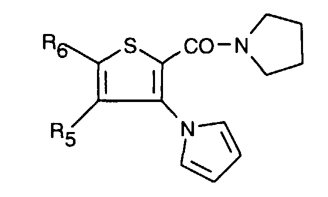

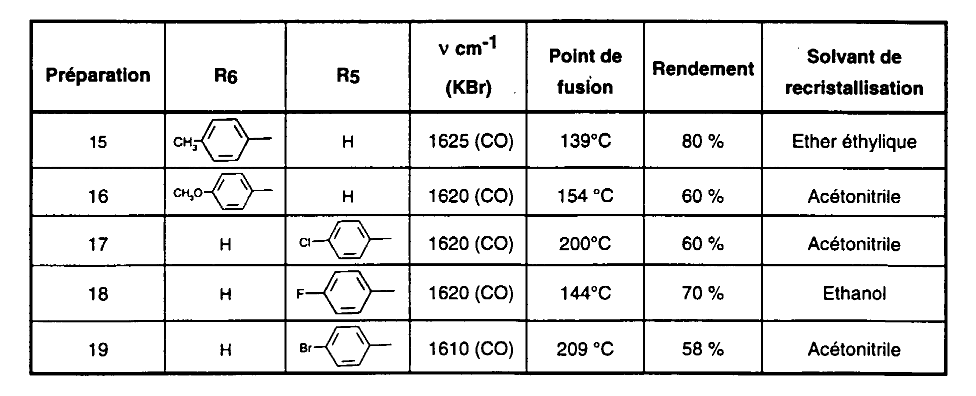

### Préparation 20 : N-[4,5-diméthyl-2-(pyrrol-1-yl)thiophén-3-ylcarbonyl]pyrrolidine

### Stade A : Chlorure de 4,5-diméthyl-2-(pyrrol-1-yl)thiophén-3-carbonyle.

4,45 g (20,1 mml) d'acide 4,5-diméthyl-2-(pyrrol-1-yl)thiophén-3-carboxylique (préparation 10) sont dissous dans 150 ml de benzène à 10°C puis 5,85 g (28,1 mmol) de pentachlorure de phosphore sont ajoutés doucement.
Après une heure d'agitation à 10°C puis 2 heures à température ambiante le milieu réactionnel est filtré et le benzène éliminé sous pression réduite.
Le résidu est repris par 500 ml de n-hexane puis filtré et le n-hexane éliminé sous pression réduite. Le chlorure d'acide obtenu est engagé directement dans le stade B.

### Stade B : N-[4,5-diméthyl-2-(pyrrol-1-yl)thiophén-3-yl-carbonyl]pyrrolidine

Le chlorure d'acide obtenu au stade A est dissous dans 25 ml de benzène a 0°C puis 2,92 ml de triéthylamine et 13,5 ml de pyrrolidine sont additionnés successivement goutte à goutte. Le milieu réactionnel est agité une heure puis diluée avec 50 ml d'eau, acidifié avec de l'acide chlorhydrique 10 N et extrait avec 300 ml d'éther éthylique.
La phase éthérée est lavée à l'eau, séchée sur sulfate de magnésium, décolorée au charbon animal et concentré sous pression réduite pour donner le composé du titre.
Rendement : 74%
Point de fusion : 100°C
Infra rouge : ν cm⁻¹(KBr) : 1615 (C=O) 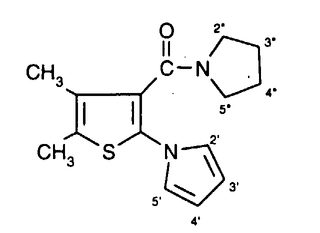 RMN ¹H : δ (ppm) (DMSO d₆) : 1,70 multiplet (4H, H_{3"}, H_{4"}) ; 2,00 singulet (3H, CH₃₍₄₎) ; 2,33 singulet (3H, CH₃₍₅₎) ; 3,33 multiplet (4H, H_{2"}, H_{3"}) ; 6,20 triplet (2H, H_{3'}, H_{4'}) ; 6,87 triplet (2H, H_{2'}, H_{5'}).

### Préparation 21 : N-[2-(pyrrol-1-yl)thiophén-3-ylcarbonyl]pyrrolidine

Ajouter goutte à goutte 50 ml de pyrrolidine à une solution d'azoture de 2-(pyrrol-1-yl)thiophén-3-yl carbonyl (Heterocycles, (1983), 20, 477) dans 500 ml de dichlorométhane à température ambiante. Le milieu réactionnel est agité 12 heures à température ambiante puis lavé par trois fois 150 ml d'acide chlorhydrique 1N puis 150 ml d'eau. La phase organique est ensuite séchée sur chlorure de calcium puis mise à sec pour foumir le composé du titre sous forme d'huile
Rendement : 73 %
Infra rouge : ν cm⁻¹(KBr) : 1610 (C=O)
RMN ¹H : δ (ppm) (DMSO d₆) : 1,66 multiplet (4H) ; 2,80 multiplet (2H) ; 3,30 multiplet (2H) ; 6,20 multiplet (2H, H_{3'}, H_{4'}) ; 6,90 multiplet (2H, H_{2'}, H_{5'}) ; 7,03 doublet (1H, H₄) ; 7,31 doublet (1H, H₅)

### Préparation 22 : acide 5-chloro 2-(pyrrol-1-yl)benzoïque

18,6 ml (0,143 mol) de 2,5-diméthoxy tétrahydrofurane sont agités 10 minutes avec 21,5 g (0,143 mol) de chlorhydrate de 4-chloropyridine dans 600 ml de 1,4-dioxane. 24,6 g (0,143 mol) d'acide 2-amino-4-chlorobenzoïque sont ajoutés et le mélange est porté au reflux 4 heures. Après élimination du dioxane sous pression réduite, le résidu est repris par 1 I d'eau et extrait par 800 ml d'éther éthylique. La phase éthérée est lavée à l'eau puis extraite avec 500 ml d'une solution aqueuse saturée en hydrogénocarbonate de sodium. La phase aqueuse est acidifiée par de l'acide chlorhydrique 10 N puis extraite avec 800 ml d'éther éthylique. La phase éthérée est lavée à l'eau, décantée, séchée sur sulfate de magnésium, décolorée au charbon animal et concentrée sous pression réduite pour fournir le composé du titre sous forme d'une poudre grise.
Rendement : 95%
Point de fusion : 178 °C
Infra rouge : ν cm⁻¹(KBr) : 3000 (COOH), 1680 (C=O)

### Préparation 23 : N-[5-chloro-2-(pyrrol-1-yl)benzoyl]pyrrolidine

### Stade A: Chlorure de 5-chloro-2-(pyrrol-1-yl)benzoyle

10 g (45,1 mmol) d'acide 5-chloro-2-pyrrol-1-ylbenzoïque sont dissous dans 300 ml de benzène à 10°C puis 13,15 g (63,1 mmol ; 1,4 éq.) de pentachlorure de phosphore sont ajoutés doucement. Après 1 heure d'agitation à 10°C puis 2 heures à température ambiante, le milieu réactionnel est filtré et le benzène éliminé sous pression réduite. Le résidu est repris par 500 ml de n-hexane puis filtré et le n-hexane éliminé sous pression réduite. On obtient le chlorure d'acide sous forme d'une huile rouge engagée directement dans le stade B.
Infra rouge : ν cm⁻¹ : 3100 (CH), 1755 (C=O)

### Stade B : N-[5-chloro-2-(pyrrol-1-yl)benzoyl]pyrrolidine

Le chlorure d'acide obtenu au stade A est dissous dans 50 ml de benzène à 0°C et 6,5 ml de triéthylamine puis 30 ml de pyrrolidine sont additionnés goutte à goutte.
Le milieu réactionnel est agité 1 heure puis dilué avec 100 ml d'eau, acidifié à pH=4 avec de l'acide chlorhydrique 10 N, et extrait avec 300 ml d'éther éthylique.
La phase éthérée est lavée à l'eau, séchée sur sulfate de magnésium, décolorée au charbon animal et concentrée sous pression réduite pour donner le composé du titre sous forme d'huile.
Rendement : 79 %
Infra rouge : ν cm⁻¹ (KBr) : 3100 (=C-H), 1620 (C=O)

### Préparation 24 : acide 5-méthyl-2-(pyrrol-1-yl)benzoïque.

En procédant de la même façon que pour la préparation 22, mais en utilisant les réactifs appropriés, on obtient le composé du titre
Rendement : 77 %
Point de fusion : 124°C
Infra rouge : ν cm⁻¹ (KBr) : 3430 (OH), 1675 (C=O)

### Préparation 25 : N-[5-méthyl-2-(pyrrol-1-yl)benzoyl]pyrrolidine.

En procédant de la même façon que pour la préparation 23, stades A et B mais en partant de l'acide 5-méthyl-2-(pyrrol-1-yl)benzoïque on obtient le composé du titre.
Rendement : 42% sur deux étapes
Point de fusion : 69°C
Infra rouge : ν cm⁻¹: 2930-2880 (=C-H), 1630(C=O)

### Préparation 26 : acide 2-(pyrrol-1-yl)benzoïque

- Méthode A :
   En procédant de la même façon que pour les préparations 22 et 24 mais en utilisant les réactifs appropriés, on obtient le composé du titre.
   Rendement : 91,8 %
   Point de fusion : 98°C
   Infra rouge : ν cm⁻¹(KBr) : 3460 (OH), 1680 (C=O)
- Méthode B :
   *Stade A : 2-(pyrrol-1-yl)benzoate de méthyle*
      17,14 mol de 2,5-diméthoxytétrahydrofurane, 19,84 g de chlrorhydrate de 4-chloropyridine dans 600 ml de 1,4-dioxane sont agités 10 minutes à 25°C, puis 17,12 mol d'anthranilate de méthyle sont ajoutés et la suspension chauffée au reflux pendant 3 heures.
      Après élimination du dioxane sous pression réduite, le résidu est repris dans 1 litre d'eau et extrait à l'éther éthylique.
      La phase éthérée est lavée à l'eau, décantée, séchée sur sulfate de magnésium, décolorée sur charbon animal puis concentrée sous pression réduite pour donner le produit du titre sous forme d'huile.
      Rendement: 92,7 %
      Infra rouge : ν cm⁻¹(KBr) : 2940 (=C-H), 1710(CO₂CH₃)
   *Stade B : acide 2-(pyrrol-1-yl)benzoïque*
      24 g de l'ester obtenu au stade précédant sont dissous dans un mélange de 75 ml de méthanol et 75 ml d'une solution aqueuse de soude à 30 %.
      Après 5 heures de chauffage au reflux, le méthanol est éliminé sous pression réduite et le résidu repris à l'eau, acidifié avec de l'acide chlorhydrique 10 N et le précipité isolé par filtration.
      Le précipité est repris dans de l'éther éthylique puis la phase éthérée est lavée à l'eau, séchée sur sulfate de magnésium, décolorée sur charbon animal puis concentré sous pression réduite pour fournir le composé du titre avec un rendement de 65%.
      Rendement global : 60%

### Préparation 27 : acide 4-chloro-2-(pyrrol-1-yl)benzoïque

En procédant de la même façon que pour les préparations 22 et 24 mais en utilisant les réactifs appropriés, on obtient le composé du titre.
Rendement: 88,2 %
Point de fusion : 139°C
Infra rouge : ν cm⁻¹(KBr) 1670 (C=O)

### Préparation 28 : 8H-thiéno[2,3-d]pyrrolo[1,2-a]pyrrol-8-one

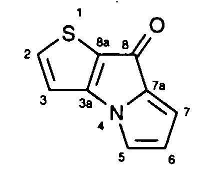

Une suspension de 0,04 moles de N-[3-(pyrrol-1-yl)thiophén-2-ylcarbonyl]pyrrolidine (préparation 11) dans 90 ml d'oxychlorure de phosphore est portée au reflux pendant 1 heure. Après refroidissement, le sel d'imminium formé est isolé par filtration, lavé à l'éther de pétrole puis à l'éther éthylique, séché puis dissous dans l'eau. La phase aqueuse est alcalinisée avec de la soude 2N goutte à goutte puis après 1 heure d'agitation le précipité formé est isolé par filtration, lavé à l'eau, séché puis recristallisé dans l'éther éthylique.
Rendement : 77 %
Point de fusion : 112°C
Infra rouge : ν cm⁻¹(KBr) : 1670 (C=O)

Ce composé peut également être obtenu en partant des N-[3-(pyrrol-1-yl)thiophén-2-ylcarbonyl] morpholine (préparation 12) et N-[3-(pyrrol-1-yl)thiophén-2-ylcarbonyl]pipéridine (préparation 13).

### Préparations 29 à 34

En procédant de la même façon que pour la préparation 28, mais en remplaçant la N-[3-(pyrrol-1-yl) thiophén-2-ylcarbonyl]pyrrolidine par l'amide appropriée, on obtient les composés correspondants aux préparations 29 à 34.

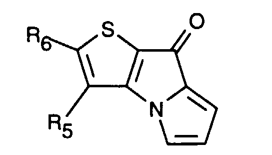

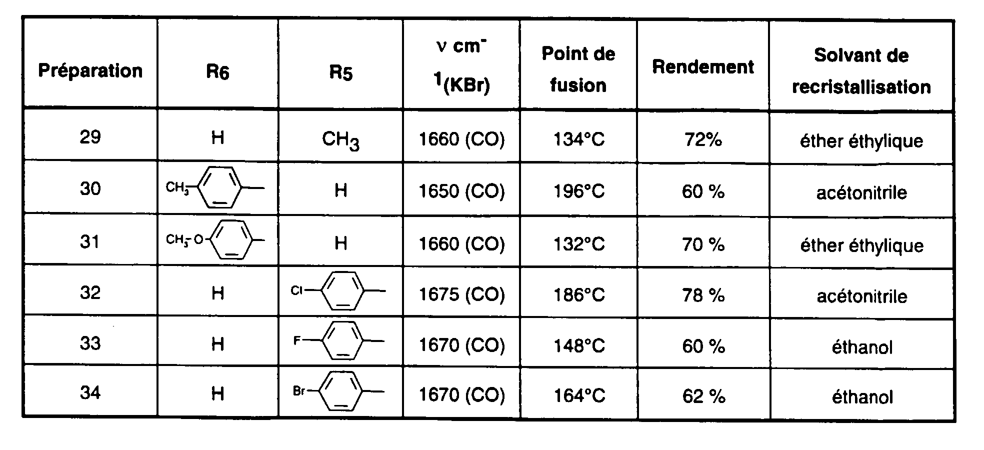

### Préparation 35 : 8-hydroxy-iminothiéno[2,3-d]pyrrolo[1,2-a]pyrrole

Chauffer à reflux pendant 90 minutes une solution de 0,011 mol de 8*H*-thiéno[2,3-d]pyrrolo[1,2-a] pyrrol-8-one (préparation 28) et 0,0132 mol de chlorhydrate d'hydroxylamine dans 50 ml de pyridine. Après refroidissement et élimination de la pyridine sous pression réduite, le résidu est repris par 150 ml d'eau et extrait à l'éther éthylique. La solution éthérée est séchée sur sulfate de magnésium puis concentrée sous pression réduite pour founir le composé du titre sous forme d'un mélange 70% de forme Z, 30% de forme E.
Rendement : 82,8%
Point de fusion : 172°C
Infra rouge : ν cm⁻¹ (KBr) : 3360 (OH)

### Préparation 36 : 3-méthyl-8-hydroxy-iminothiéno[2,3-d]pyrrolo[1,2-a]pyrrole

En procédant comme pour la préparation 35 on obtient le composé du titre sous forme d'un mélange 70% de forme Z, 30% de forme E.
Rendement : 92%
Point de fusion : 198°C (mélange 70%Z, 30%E)
Infra rouge : ν cm⁻¹(KBr) : 1635 (C=N)

Le 3-méthyl-8-hydroxy-iminothiéno[2,3-d]pyrrolo[1,2-a]pyrrole peut être obtenu sous forme d'isomère Z pur par recristallisation du mélange d'isomère Z et E dans l'acétonitrile.
Rendement : 33%
Point de fusion : 204°C (isomère Z pur)
RMN¹H : (DMSOd₆) : δ(ppm) : 2,03 singulet (3H,CH₃) ; 5,86 doublet de doublet (1H,H₆) ; 6,16 doublet (1H, H₇) ; 6,96 doublet (1H, H₅) ; 7,16 singulet (1H, H₂) ; 11,49 singulet (1H, =N-OH)

### Préparation 37 à 41 :

En procédant comme pour la préparation 35, mais en partant des composés des préparations 30 à 34, on obtient les composés des préparations 37 à 41.

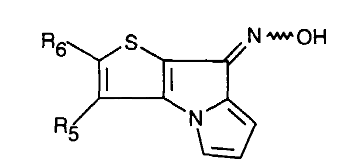

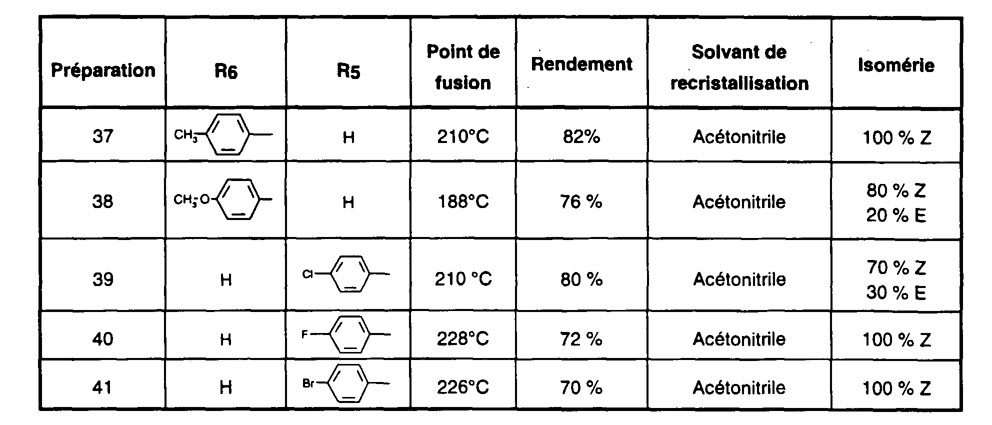

### Préparation 42 : 7-chloropyrrolo[1,2-a]indol-9-one

Une solution de 8,5 g (30,9 mmol) de N-[5-chloro-2-(pyrrol-1-yl)benzoyl]pyrrolidine (préparation 23) dans 70 ml d'oxychlorure de phosphore est chauffée à 100°C pendant 35 minutes. Après refroidissement, le précipité formé est isolé par filtration, lavé avec de l'éther de pétrole pour éliminer l'excès d'oxychlorure de phosphore, puis redissous dans 30 ml d'eau. La solution aqueuse est alcalinisée avec de la soude à 10% goutte à goutte et le précipité formé filtré et extrait par 100 ml d'acétate d'éthyle. La phase organique est lavée à l'eau, séchée sur sulfate de magnésium, décolorée au charbon animal et concentrée sous pression réduite pour fournir 1,76 g du produit du titre.
Rendement : 27 %
Point de fusion : 92°C
Infra rouge : ν cm⁻¹ (KBr) : 3090 (CH), 1675 (C=O) 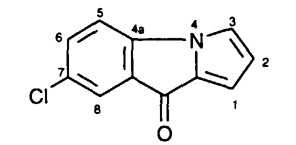 RMN ¹H : δ(ppm) : (DMSO d₆) : 6,33 doublet de doublet (1H, H₂) ; 6,80 doublet de doublet (1H, H₁) ; 7,53-7,47-7,40 multiplet (4H, H₃, H₅, H₆, H₈)

### Préparation 43 : 7-méthylpyrrolo[1,2-a]indol-9-one.

Ajouter à 0°C 1,36 ml (14,9 mmol) d'oxychlorure de phosphore à 1,14 ml (14,9 mmol) de diméthylformamide puis additionner une solution de 3 g (14,9 mmol) d'acide 5-méthyl-2-pyrrol-1-yl benzoïque (préparation 25) dans 30 ml de diméthylformamide.
Après une nuit d'agitation à température ambiante, hydrolyser le milieu réactionnel en le versant sur 50 ml d'eau, extraire avec 200 ml d'éther éthylique et laver la phase éthérée successivement avec de l'eau, une solution aqueuse saturée en bicarbonate puis avec de l'eau.
Après séchage sur sulfate de magnésium et décoloration sur charbon animal, la phase éthérée est mise à sec pour fournir le composé du titre.
Rendement : 35%
Point de fusion : 85°C
Infra rouge : ν cm⁻¹(KBr) : 1670 (C=O)

### Préparations 44 et 45

En procédant de la même façon que pour la préparation 43 on obtient :

### Préparation 44 : pyrrolo[1,2-a]indol-9-one

Rendement : 50%
Point de fusion : 126°C
Infra rouge : ν cm⁻¹(KBr) : 1670 (C=O)

### Préparation 45 : 6-chloropyrrolo[1,2-a]indol-9-one

Rendement : 35 %
Point de fusion : 181°C
Infra rouge : ν cm⁻¹(KBr) : 1670 (C=O)

### Préparation 46 : 7-chloro-9-hydroxy-iminopyrrolo[1,2-a]indole

Ajouter 3,9 g (56,1 mmol) de chlorhydrate d'hydroxylamine à une solution de 4,6 g (22,6 mmol) de 7-chloropyrrolo[1,2-a]indol-9-one dans 80 ml de pyridine puis chauffer le milieu réactionnel au reflux pendant 3 heures. Après refroidissement, la pyridine est éliminée sous pression réduite et le résidu repris dans l'eau et extrait par 300 ml d'éther éthylique. La phase éthérée est lavée avec 100 ml d'acide chlorhydrique 0,5 N, puis avec de l'eau, séchée sur sulfate de magnésium, décolorée au charbon animal puis concentré sous pression réduite pour fournir 4,18 g du produit du titre.
Rendement : 84,6 %
Point de fusion : 211°C
Infra rouge : ν cm⁻¹ (KBr) : 3180, 3060, 2880, 1490 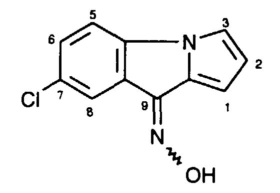 RMN ¹H : δ(ppm) (DMSO d₆) : 6,40 doublet de doublet (1H, H₂) ; 6,70 doublet de doublet (1H, H₁) ; 7,65-7,55 multiplet (4H, H₃, C₆H₃) ; 12,27 singulet (1H, OH)

### Préparations 47 à 49

En procédant comme pour la préparation 46 mais en partant des composés des préparations 43, 44 et 45, on obtient les composés des préparations 47, 48 et 49.

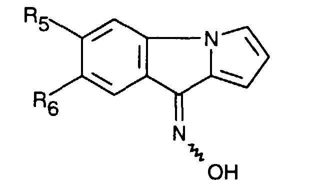

| **Préparation** | **R**_{**5**} | **R**_{**6**} | **Point de fusion** | **Rendement** | **RMN** ^{**1**}**H δ(ppm) (DMSO d6)** |
|---|---|---|---|---|---|
| 47 | H | CH₃ | 187°C | 77% | 2,35 (s, 3H) ; 6,33 (dd, 1H) ; 6,65 (dd, 1H) ; 7,43-7,25 (m, 4H) ; 11,43 (s, OH) |
| 48 | H | H | 186°C | 80% | 6,35 (dd, 1H) ; 6,65 (dd, 1H) ; 7,50-7,23-7,13 (m, 5H) ; 11,97 (m, 1H) |
| 49 | Cl | H | 195°C | 84% | 6,38 (dd, 1H) ; 6,67 (dd, 1H) ; 7,17 (dd, 1H) ; 7,57 (m, 2H) ; 7,77 (d,1H) ; 12,10 (s, 1H) |

### Préparation 50 : 1,2-diméthyl-8H-thiéno[3,2-d]pyrrolo[1,2-a]pyrrol-8-one

Une solution de 7,6 g de N-[4,5-diméthyl-2-(pyrrol-1-yl)thiophén-3-ylcarbonyl]pyrrolidine (préparation 20) dans 50 cm³ d'oxychlorure de phosphore est chauffée à 100 °C pendant 35 minutes. Après refroidissement, le précipité formé est isolé par filtration, lavé avec de l'éther de pétrole pour éliminer l'excès d'oxychlorure de phosphore puis redissous dans 25 ml d'eau. La solution acqueuse est alcalinisée avec de la soude à 10% et le précipité formé filtré et extrait par 100 ml d'acétate d'éthyle. La phase organique est lavée à l'eau, séchée sur sulfate de magnésium, décolorée au charbon animal et concentrée sous pression réduite pour fournir 2,8 g du produit du titre.
Rendement : 49,7 %
Point de fusion : 95°C (décomposition)
Infra rouge : ν cm⁻¹(KBr) : 1670 (C=O) 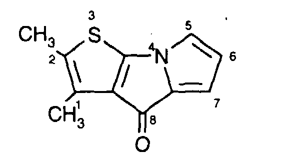 RMN ¹H : δ(ppm) (DMSO d₆) : 2,03 singulet (3H, CH₃₍₁₎) ; 2,20 singulet (3H, CH₃₍₂₎) ; 6,05 doublet de doublet (1H, H₆) ; 6,93 doublet de doublet (1H, H₇) ; 7,20 doublet de doublet (1H, H₅)

### Préparation 51 : 8H-thiéno[3,2-d]pyrrolo[1,2-a]pyrrol-8-one

En procédant comme pour la préparation 50 mais en utilisant la N-[2-(pyrrol-1-yl)thiophén-3-yl carbonyl]pyrrolidine (préparation 21) on obtient le composé du titre.

### Préparation 52 : 1,2-diméthyl-8-hydroxy-iminothiéno[3,2-]pyrrolo[1,2-a]pyrrole

En procédant comme pour la préparation 46 mais en partant de la 1,2-diméthyl-8*H*-thiéno[3,2-d] pyrrolo[1,2-a]pyrrol-8-one (préparation 50) on obtient le composé du titre.
Rendement : 83 %
Point de fusion : décomposition à partir de 180°C 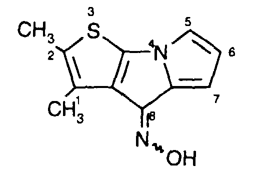 RMN ¹H : δ(ppm) (DMSO d₆) : 2,17 singulet (3H, CH₃₍₁₎); 2,30 singulet (3H, CH₃₍₂₎) ; 6,13 doublet de doublet (1H, H₆) ; 6,53 doublet de doublet (1H, H₇) ; 7,15 doublet de doublet (1H, H₅)

### Préparation 53 : 8-hydroxy-iminothiéno[3,2-d]pyrrolo[1,2-a]pyrrole

En procédant comme pour la préparation 52 mais en partant de la 8*H*-thiéno[2,3-b]pyrrolo[2,1-e] pyrrole-8-one (préparation 51) on obtient le composé du titre
Rendement : 90 %
Point de fusion :
250°C isomère E 190°C isomère Z
Infra rouge : ν cm⁻¹(KBr) : 1635 (C=N) 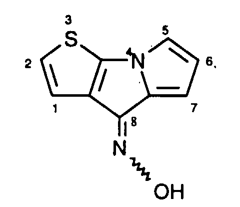 RMN ¹H: δ(ppm)(DMSO d₆) :
Isomère E : 6,15 (1H, H₆) ; 6,60 (1H, H₇) ; 7,03 (2H, H₁, H₂) ; 7,27 (1H, H₅)
Isomère Z: 6,12 (1H, H₆) ; 6,37 (1H, H₇) ; 7,05 (1H, H₂) ; 7,21 (1H, H₁₎ 7,26 (1H, H₅)

### EXEMPLE 1 : 8-[(3-isopropylamino-2-hydroxy-n-prop-1-yloxy)imino]thiéno[2,3-d]pyrrolo [1,2-a]pyrrole

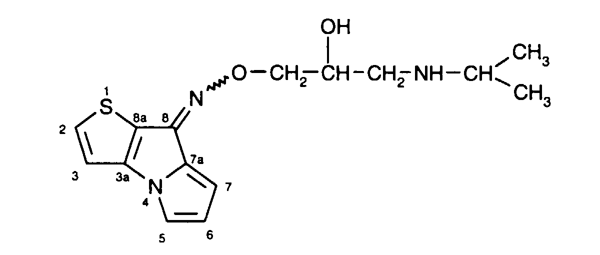

### Stade A : 8-[(2,3-époxyprop-1-yl)oxyimino]thiéno[2,3-d]pyrrolo[2,1-a]pyrrole (Z)

Ajouter 0,8 g d'hydrure de sodium à une solution de 4 g (0,021 mole) de 8-hydroxyiminothiéno[2,3-d] pyrrolo[1,2-a]pyrrole dans 50 ml de toluène et 10 ml de diméthylformamide. Attendre 15 minutes puis ajouter 2,85 g (0,021 mole) d'épibromhydrine. Après 7 heures d'agitation à température ambiante, le milieu réactionnel est versé dans 200 ml d'eau, décanté, séché puis mis à sec sous pression réduite. Le solide obtenu est recristallisé dans le cyclohexane pour donner l'isomère Z.
Rendement : 35 %
Point de fusion : 100°C
Infra rouge : ν cm⁻¹(KBr) :1600 (C=N)

### Stade B : 8-[3-isopropylamino-2-hydroxyprop-1-yloxy)imino]thiéno[2,3-d]pyrrolo[1,2-a]pyrrole

2 g de 8-[(2,3-époxyprop-1-yl)oxyimino]thiéno[2,3-d]pyrrolo[1,2-a]pyrrole (Z) sont additionnés à 5 ml d'une solution aqueuse d'isopropylamine puis agités 10 heures à température ambiante. Après hydrolyse et extraction à l'éther éthylique, l'huile résiduelle obtenue est dissoute dans 30 ml d'isopropanol à 40°C. Après addition d'un équivalent d'acide oxalique, le précipité formé est essoré, lavé à l'éther et recristallisé dans un mélange isopropanol/propanol (60:40)
Rendement : 65% (oxalate)
Point de fusion : oxalate 196°C (mélange 50/50 Z/E)
Infra rouge : ν cm⁻¹ (KBr) : (oxalate) 3120-2600 (NH₂+), 1700 (C=O)

### EXEMPLE 2 : 8-[(3-cycloheptylamino-2-hydroxyprop-1-yloxy)imino]thiéno[2,3-d]pyrrolo [1,2-a]pyrrole

En procédant comme pour l'exemple 1 mais en remplaçant dans le stade B l'isopropylamine par la cycloheptylamine on obtient le composé du titre
Rendement: 51% Point de fusion : base : 118°C : (mélange 50/50 Z/E)
oxalate : 186°C : (mélange 50/50 Z/E)

### EXEMPLE 3 : 8-{[2-(N,N-diméthylamino)éthyloxy]imino}-3-méthylthiéno[2,3-d]pyrrolo [1,2-a]pyrrole (Z)

A 6,99 g de carbonate de sodium dans 200 ml d'acétone anhydre sont ajoutés 1,32 g d'eau puis 3,91 g de chlorhydrate de 1-chloro-2-(N,N-diméthylamino)éthane et 5 g de 8-hydroxyimino-3-méthyl thiéno[2,3-d]pyrrolo[1,2-a]pyrrole (Z) (préparation 36). Le milieu réactionnel est porté au reflux 40 heures puis l'acétone éliminée, le résidu repris par 100 ml d'eau puis extrait par 200 ml d'éther éthylique. La phase éthérée est lavée à l'eau, décantée puis séchée sur sulfate de magnésium puis mis à sec pour donner le produit du titre sous forme d'huile.
Rendement : 77%

### Préparation du sel d'oxalate

4,9 g de l'huile précédemment obtenue sont dissous dans 50 ml d'isopropanol à 40°C puis on additionne 1,68 g d'acide oxalique et l'on porte à reflux pendant 30 minutes. Après refroidissement le précipité est isolé par filtration, lavé à l'éther éthylique puis séché pour donner le produit du titre sous forme d'oxalate.
Rendement : 68%
Point de fusion : 200°C
Infra rouge : ν cm⁻¹ (KBr) : 3006, 2700, 2540, 2500, 1725, 1610. 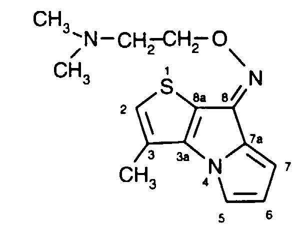 RMN ¹H: δ(ppm) (DMSO d₆) : 2,36 singulet (3H, CH₃) ; 2,80 singulet (6H, 2xCH₃) ; 3,43 multiplet (2H, CH₂) ; 4,60 multiplet (2H, CH₂) ; 6,20 doublet dédoublé (1H, H₆) ; 6,53 doublet dédoublé (1H, H₇) ; 7,30 doublet dédoublé (1H, H₅) ; 7,56 singulet (1H, H₂) ; 10,40 singulet (1H, NHO)

En partant de 8-hydroxyiminothiéno[2,3-d]pyrrolo[1,2-a]pyrrole sous forme d'un mélange 70% Z/30% E on obtient le composé du titre sous forme d'un mélange d'isomères en proportions équivalentes aux proportions de départ.

### EXEMPLES 4 à 38:

En procédant de la même façon que pour l'exemple 3 mais en utilisant l'aminohalogénoalkyle et le 8-hydroxy-iminothiéno[2,3-d]pyrrolo[1,2-a]pyrrole approprié, on obtient les composés des exemples suivants :

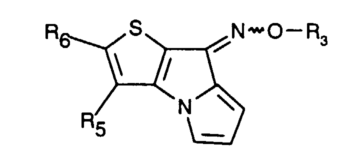

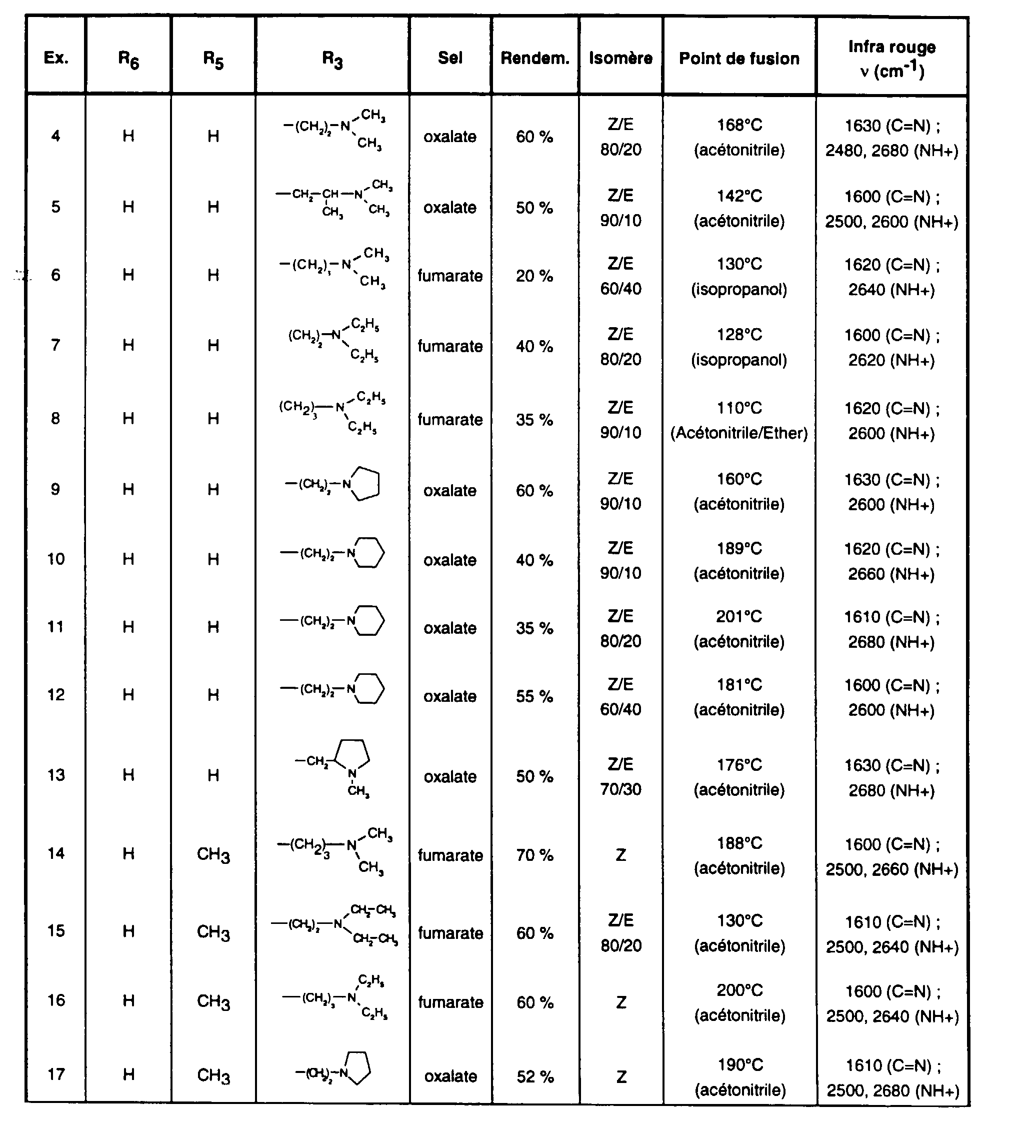

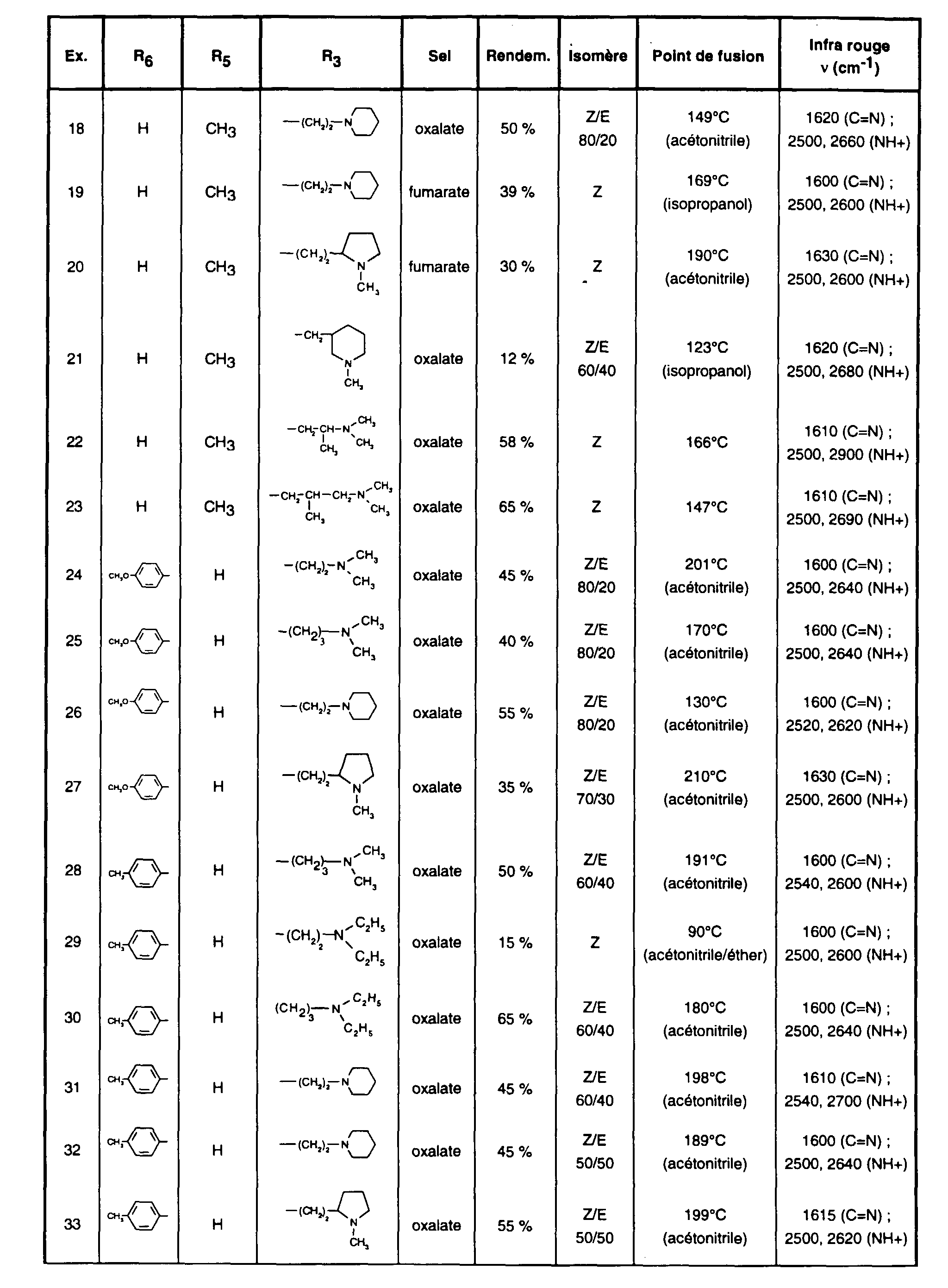

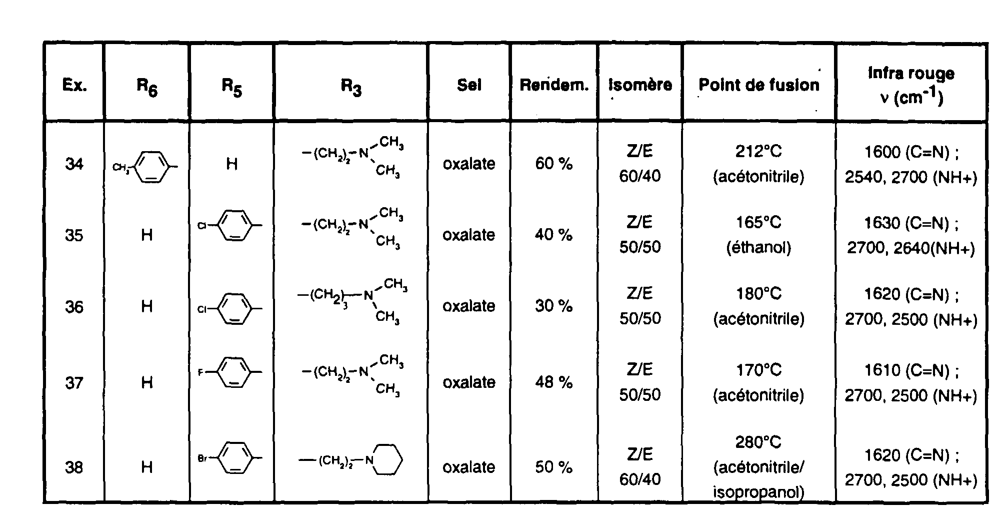

### EXEMPLE 39 : 3-méthyl-8-([3-(N,N-diméthylamino)-1-phénylprop-1-yloxy]imino}thiéno [2,3-d]pyrrolo[1,2-a]pyrrole

### Méthode A:

Ajouter 0,75 g (25 mmol) d'hydrure de sodium à une solution de 1 g (4,89 mmol) de 3-méthyl-8-hydroxy-iminothiéno[2,3-d]pyrrolo[1,2-a]pyrrole (Z) (préparation 36) dans 50 ml de benzène. Après 1 heure de chauffage à reflux, ajouter 1,15 g (4,89 mmole) de chlorhydrate de N,N-diméthyl-3-chloro-3-phénylpropylamine et maintenir le reflux pendant 2 heures. Après refroidissement, le précipité est filtré et le filtrat concentré sous pression réduite puis extrait à l'éther éthylique. Après lavage à l'eau et séchage, la phase organique est mise à sec pour fournir le composé du titre sous forme de base qui est ensuite salifiée dans l'isopropanol par de l'acide oxalique.
Rendement: 6 %
Point de fusion : (oxalate) : 210°C (décomposition)
Infra rouge : ν cm⁻¹ (KBr) (oxalate) : 1710 (C=O) 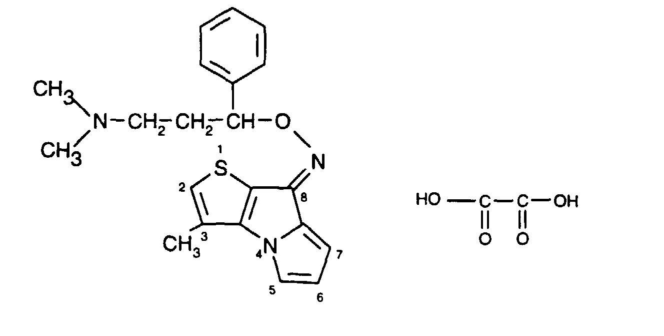 RMN¹H : δ (ppm) (DMSO d₆) : 2,33 multiplet (5H, CH₂ et CH₃) ; 2,70 singulet (6H, N(CH₃)₂) ; 3,00 triplet (2H, CH₂-N) ; 5,30 multiplet (3H, CH, OH, NH⁺) ; 6,10 doublet de doublet (1H, H₆) ; 6,40 doublet de doublet (1H, H₇) ; 7,30 multiplet (6H, H₉ et C₆H₅) ; 7,50 singulet (1H, H₂).

### Méthode B :

Porter au reflux 26 heures 1 g de 3-méthyl-8-hydroxy-iminothiéno[2,3-d]pyrrolo[1,2-a]pyrrole ,2-a]pyrrole (Z), 1,15 g de chlorhydrate de N,N-diméthyl-3-chloro-3-phénylpropylamine et 0,8 g d'hydroxyde de sodium dans 100 ml de méthanol. Après refroidissement et élimination du méthanol sous pression réduite, le résidu est extrait à l'éther éthylique puis traité comme pour la méthode A.
Rendement : 15 %

### EXEMPLE 40 : 3-méthyl-8-[(2-amino-éthyloxy)imino]thiéno[2,3-d]pyrrolo[1,2-a]pyrrole (Z)

### Stade A : 3-méthyl-8-[(2-bromo-éthyloxy)imino]thiéno[2,3-d]pyrrolo[1,2-a]pyrrole (Z)

Ajouter successivement 5 g de bromhydrate de 2-aminoxy-1-bromoéthane, 1,5 mole d'acide acétique glacial et 1,6 ml de pyridine à une suspension de 1,43 g de 3-méthyl-8*H*-thiéno[2,3-d]pyrrolo[1,2-a] pyrrol-2-one (préparation 29) dans 70 ml d'éthanol. Le milieu réactionnel est porté à reflux pendant 3 heures puis l'éthanol est éliminé sous pression réduite et le résidu extrait par 100 ml d'éther éthylique. Après lavage à l'eau, séchage, mise à sec et réempatage dans un mélange éther éthylique/éther de pétrole, 1:1, on obtient le produit du titre sous forme d'une poudre.
Rendement : 23 %
Point de fusion : 92°C
Infra rouge : ν cm⁻¹ (KBr) : 2950, 2900, 1490

### Stade B : 3-méthyl-8-[(2-phtalimido-éthyloxy)imino]thiéno[2,3-d]pyrrolo[1,2-a]pyrrole (Z)

Ajouter 0,98 g de phtalimidure de potassium à une solution de 1,64 g de 8-[(2-bromo-éthyloxy)imino] thiéno[2,3-d]pyrrolo[1,2-a]pyrrole (Z) dans 30 ml de diméthylformamide, puis chauffer le mélange au reflux pendant 2 heures. Après refroidissement, le milieu réactionnel est versé sur 200 ml d'eau puis extrait par 300 ml d'acétate d'éthyle. La phase organique est lavée à l'eau, séchée sur sulfate de magnésium, décolorée au charbon animal puis concentrée et le résidu repris par un minimum d'éther éthylique puis filtré et séché.
Rendement : 55 %
Point de fusion : 157°C (acétonitrile/isopropanol, 8:2)
Infra rouge : ν cm⁻¹ (KBr) : 1710 (C=O)

### Stade C : 3-méthyl-8-[(2-amino-éthyloxy)imino]thiéno[2,3-d]pyrrolo[1,2-a]pyrrole (Z)

Ajouter 0,3 ml (6,1 mmol) d'hydrate d'hydrazine à une solution de 0,85 (2,2 mmol) de 3-méthyl-8-[(2-phtalimido-éthyloxy)imino]thiéno[2,3-d]pyrrolo[1,2-a]pyrrole (Z) dans 50 ml d'un mélange éthanol/isopropanol, 1:1. Le milieu réactionnel est porté au reflux. Après 30 minutes de reflux, ajouter 0,3 ml d'hydrate d'hydrazine et reporter 90 minutes au reflux. Rajouter 0,5 ml d'hydrate d'hydrazine pendant les 90 minutes de reflux. Après refroidissement du milieu réactionnel, le précipité formé est éliminé par filtration et le filtrat concentré sous pression réduite. Le résidu est extrait à l'éther éthylique et la phase éthérée lavée à l'eau, séchée sur sulfate de magnésium, traitée au charbon animal et concentrée pour fournir 200 mg du produit du titre sous forme d'huile (base).

### Préparation du sel d'oxalate :

Reprendre l'huile précédemment obtenue dans 40 ml d'isopropanol puis ajouter 90 mg d'acide oxalique et chauffer à reflux pendant 15 minutes. Après refroidissement, isoler le précipité formé par filtration, et le laver avec de l'isopropanol puis de l'éther éthylique anhydre.
Rendement : 18 %
Point de fusion (oxalate) : 200°C : décomposition (acétonitrile)
Infra rouge : ν cm⁻¹ (KBr) : 1720 cm⁻¹ (C=O) 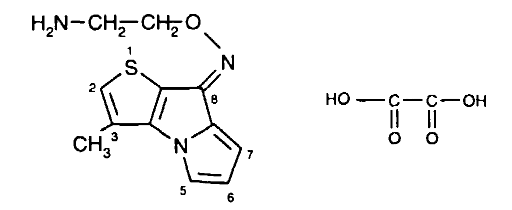 RMN ¹H : δ (ppm) (DMSO d₆) : 2,37 singulet (3H, CH₃) ; 3,20 triplet (2H, CH₂-N) ; 4,37 triplet (2H,O-CH₂) ; 6,20 multiplet (5H, H₆, NH₃⁺ et OH) ; 6,50 doublet de doublet (1H, H₇) ; 7,23 doublet de doublet (1H, H₉) ; 7,50 singulet (1H, H₂).

### EXEMPLE 41 : 3-méthyl-8-{[2-(N-benzylamino)éthyloxy]imino}thiéno[2,3-d]pyrrolo[1,2-a] pyrrole (Z)

Additionner successivement 1 g (3,2 mmole) de 3-méthyl-8-[(2-bromo-éthyloxy)imino]thiéno[2,3-d] pyrrolo[1,2-a]pyrrole (Z), 0,53 g (3,8 mmole) de carbonate de potassium et 0,51 g (4,8 mmole) de benzylamine dans 20 ml de N,N-diméthylformamide. Le milieu réactionnel est porté à reflux pendant 3 heures puis après refroidissement versé sur 100 ml d'eau et extrait à l'éther éthylique. La phase éthérée est lavée à l'eau, séchée sur sulfate de magnésium, décolorée au charbon animal puis concentrée sous pression réduite pour fournir 0,7 g du produit du titre sous forme d'huile (base).

### Préparation du sel d'oxalate

Reprendre l'huile précédemment obtenue dans 80 ml d'isopropanol puis ajouter 0,2 g d'acide oxalique et porter le milieu réactionnel au reflux pendant 30 minutes.'Après refroidissement, isoler le précipité formé par filtration et le laver avec de l'isopropanol puis de l'éther éthylique anhydre.
Rendement : 25 %
Point de fusion (oxalate) : 190°C décomposition (acétonitrile)
Infra-rouge : ν cm⁻¹ (KBr) : 1715 (C=O) 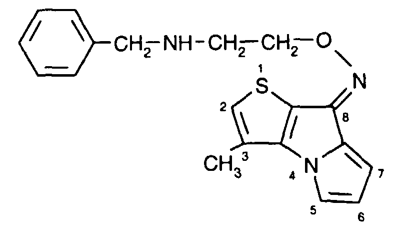 RMN ¹H : δ (ppm) (DMSO d₆) : 2,43 singulet (3H, CH₃) ; 3,47 triplet (2H, CH₂-NH) ; 4,30 singulet (2H, CH₂-φ) ; 4,60 triplet (2H, OCH₂) ; 6,27 doublet de doublet (1H, H₆) ; 6,57 doublet de doublet (1H, H₇) ; 6,77 multiplet (3H, NH₂⁺, OH) ; 7,30 doublet de doublet (1H, H₅) ; 7,47 multiplet (6H, H₂, C₆H₅).

### EXEMPLE 42 : 8-{[2-(N,N-diméthylamino)éthyloxy]imino}thiéno[3,2-d]pyrrolo[1,2-a]pyrrole (Z)

En procédant comme pour l'exemple 3 mais en remplaçant le 8-hydroxy-imino-3-méthylthiéno[2,3-d] pyrrolo[1,2-a]pyrrole (Z) par le 8-hydroxy-iminothiéno[3,2-d]pyrrolo[1,2-a]pyrrole (préparation 53) on obtient le composé du titre sous forme d'oxalate avec un rendement de 70 %.
Point de fusion (oxalate) : > 260°C décomposition (acétonitrile)
Infra-rouge : ν cm⁻¹ (KBr) (oxalate) : 1725 (C=O), 1635 (C=N), 1500, 1090, 960, 900, 835 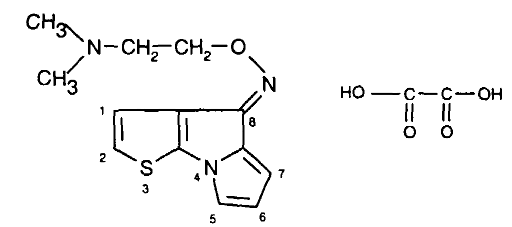 RMN ¹H : δ (ppm) (DMSO d₆) : 2,75 singulet (6H, 2xCH₃) ; 3,4 multiplet (2H, CH₂) ; 4,6 multiplet (2H, CH₂) ; 6,12 doublet de doublet (1H, H₇) ; 6,35 doublet de doublet (1H, H₈) ; 7,05 doublet (1H, H₂) ; 7,20 doublet (1H, H₁) ; 7,25 doublet de doublet (1H, H₆) ; 10,4 singulet (1H, OH).

### EXEMPLE 43 : 8-{[2-(N,N-diméthylamino)éthyloxy]imino}-1,2-diméthylthiéno[3,2-d]pyrrolo [1,2-a]pyrrole

En procédant comme pour l'exemple 42 mais en partant du 1,2-diméthyl-8-hydroxyiminothiéno[3,2-d] pyrrolo[1,2-a]pyrrole (préparation 52), on obtient le composé du titré sous forme d'oxalate avec un
rendement : 38%
Point de fusion (oxalate) : 176°C (acétonitrile)
Infra-rouge : ν cm⁻¹ (KBr) : 3380, 1710 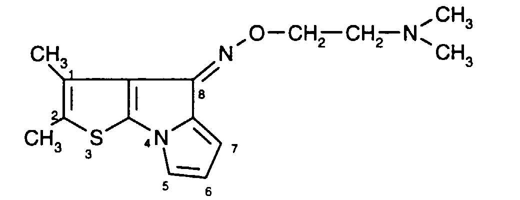 RMN ¹H: δ (ppm) (DMSO d₆) : 2,13 singulet (3H, CH₃₍₁₎) ; 2,30 singulet (3H, CH₃₍₂₎); 2,77 singulet (6H, N(CH₃)₂) ; 3,40 triplet (2H, CH₂N) ; 4,53 triplet (2H, OCH₂) ; 6,20 doublet de doublet (1H, H₆) ; 6,40 multiplet (2H, OH, NH⁺) ; 7,67 doublet de doublet (1H, H₇) ; 7,27 doublet de doublet (1H, H₅).

### EXEMPLES 44 à 47

En procédant comme pour l'exemple 42 on obtient :
**EXEMPLE 44 : 8-[(2-morpholino-éthyloxy)imino]thiéno[3,2-d]pyrrolo[1,2-a]pyrrole**
**EXEMPLE 45 : 8-{[3-(N,N-diméthylamino)-1-phényl-n-prop-1-yloxy]imino}thiéno[3,2-d]pyrrolo [1,2-a]pyrrole**
**EXEMPLE 46 : 8-[(2-amino-éthyloxy)imino]thiéno[3,2-d]pyrrolo[1,2-a]pyrrole**
**EXEMPLE 47 : 1,2-diméthyl 8-[(2-amino-éthyloxy)imino]thiéno[3,2-d]pyrrolo[1,2-a]pyrrole**
**EXEMPLE 48 : 8-[(3-isopropylamino-2-hydroxy-n-prop-1-yloxy)imino]thiéno[3,2-d]pyrrolo [1,2-a]pyrrole**

En procédant comme pour l'exemple 1, on obtient le composé du titre.

### EXEMPLE 49 : 7-chloro-9-{[2-(N,N-diméthylamino)éthyloxy]imino}pyrrolo[1,2-a]indole

Additionner successivement 5 ml d'eau, 0,94 g (6,5 mmol) de chlorhydrate de 1-chloro-2-(N,N-diméthylamino)éthane et 1,19 g (5,44 mmol) de 7-chloro-9-hydroxy-iminopyrrolo[1,2-a]indole (préparation 46) à une solution de 1,56 g (14,7 mmol) de carbonate de sodium dans 100 ml d'acétone, puis chauffer le milieu réactionnel à reflux pendant 36 heures. L'acétone est éliminée sous pression réduite et le résidu repris par 50 ml d'eau et extrait à l'éther éthylique. La phase éthérée est lavée à l'eau, séchée sur sulfate de magnésium, décolorée au charbon animal puis mise à sec pour donner le produit du titre sous forme d'une huile (base).

### Préparation du sel d'oxalate :

L'huile précédemment obtenue est reprise par 50 ml d'isopropanol puis 1 équivalent d'acide oxalique est ajouté et le milieu chauffé 15 minutes à 40°C. Après refroidissement, le précipité formé est isolé par filtration puis séché pour fournir le sel d'oxalate du composé du titre.
Rendement global (oxalate) : 23%
Point de fusion (oxalate) : 176°C (acétonitrile)
Infra rouge : ν cm⁻¹ (KBr) : 3410 (OH) ; 3030 (CH) ; 1710 (C=O) 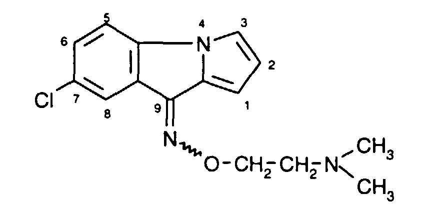 RMN ¹H : δ (ppm) (DMSO d₆) : 2,80 singulet (6H, 2 x CH₃) ; 3,45 multiplet (2H, CH₂-N) ; 4,62 multiplet (2H, O-CH₂); 6,38 doublet de doublet (1H, H₂) ; 6,82 doublet de doublet (1H, H₁) ; 7,55 multiplet (4H, H₃, C₆H₃) ; 10,28 (m, 2H, CO₂H)

### EXEMPLES 50 à 57

En procédant de la même façon que pour l'exemple 49 mais en utilisant l'aminohalogéno-alkyle et le 9-hydroxy-iminopyrrolo[1,2-a]indole appropriés on obtient les composés des exemples suivants :

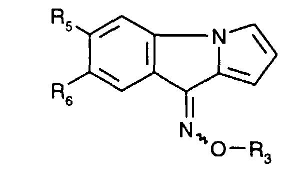

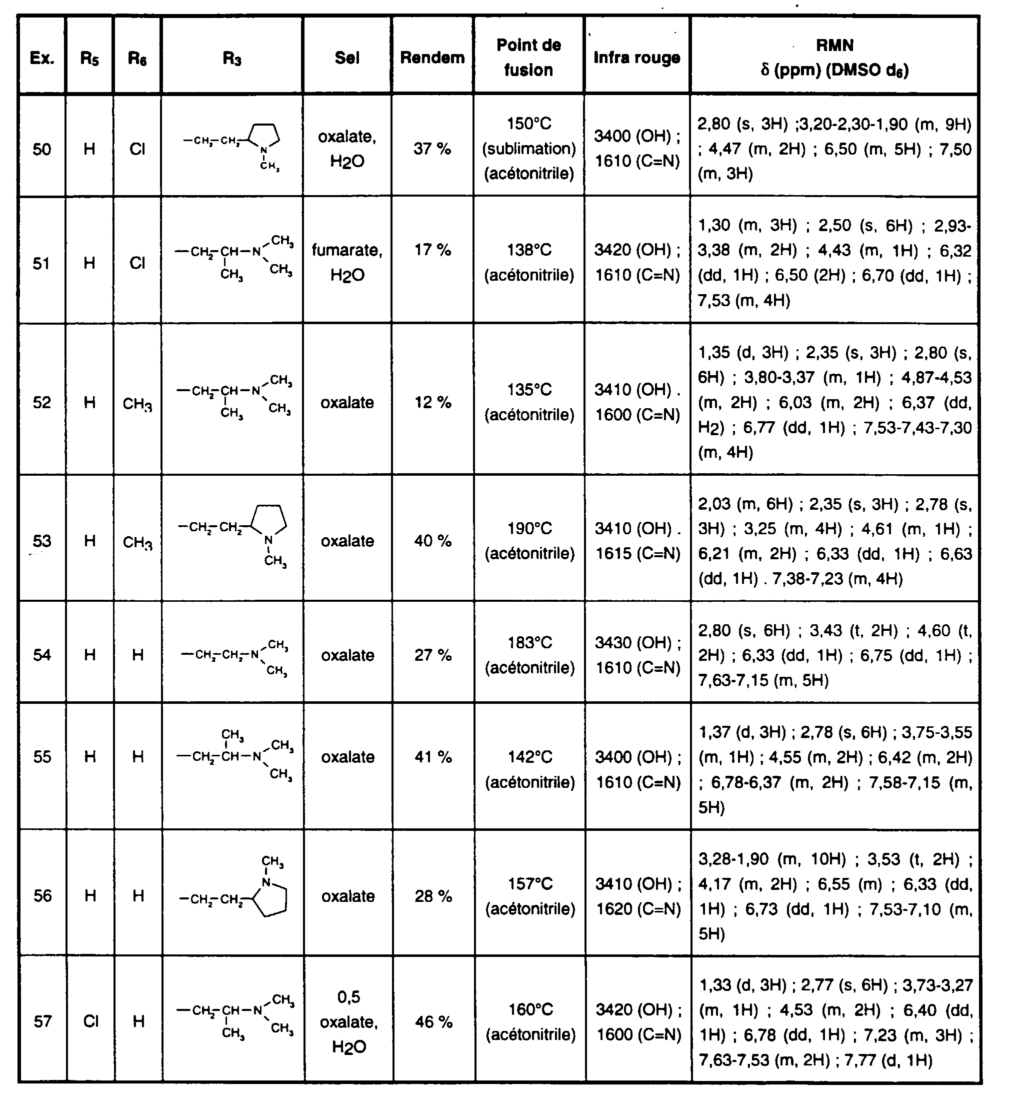

### EXEMPLE 58 : 6-chloro-9-{[3-(N,N-diméthylamino)-1-phényl-n-prop-1-yloxy]imino}pyrrolo[1,2-a] indole

En procédant comme pour l'exemple 39 mais en remplaçant le 3-méthyl-8-hydroxy-imino thiéno[2,3-d] pyrrolo[1,2-a]pyrrole (Z) par le 6-chloro-9-hydroxy-iminopyrrolo[1,2-a]indole, on obtient le composé du titre dont le sel d'oxalate est préparé avec un rendement global de 23 %.
Point de fusion (oxalate) : 120°C décomposition
Infra rouge : ν cm⁻¹ (KBr) :1705 (C=O) 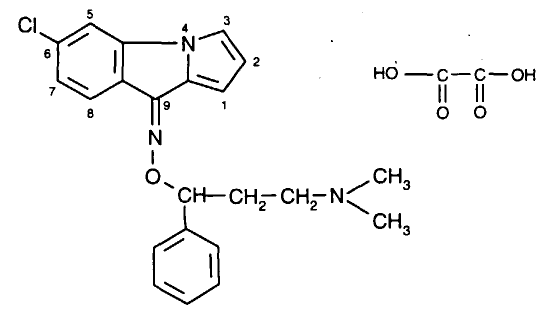 RMN ¹H : δ (ppm) (DMSO d₆) : 2,50 triplet (2H, CH₂-N) ; 2,73 singulet (6H, N(CH₃)₂) ; 3,13 quadruplet (2H, CH₂) ; 5,07 multiplet (2H, OH, NH⁺) ; 5,40 multiplet (1H, CH) ; 6,43 doublet de doublet (1H, H₂) ; 7,87 doublet de doublet (1H, H₁); 7,60-7,33 multiplet (8H, C₆H₃ et C₆H₅) ; 7,77 doublet de doublet (1H, H₃)

### EXEMPLE 59 : 9-[(2-amino-éthyloxy)imino]pyrrolo[1,2-a]indole

### Stade A : 9-[(2-bromo-éthyloxy)imino]pyrrolo[1,2-a]indole

En procédant comme pour le stade A de l'exemple 40 mais en remplaçant la 3-méthyl-8H-thiéno [3,2-b]pyrrolo[2,1-e]pyrrol-2-one par la pyrrolo[1,2-a]indol-9-one, on obtient le 9-[(2-bromoéthyloxy)imino]pyrrolo[1,2-a]indole avec un rendement de 89 %.
Point de fusion : 65°C

### Stade B : 9-[(2-phtalimido-éthyloxy)imino]pyrrolo[1,2-a]indole

En procédant comme pour le stade B de l'exemple 40 mais en remplaçant le 3-méthyl-8-[(2-bromoéthyloxy)imino]thiéno[3,2-b]pyrrolo[2,1-e]pyrrole (Z) par le 9-[(2-bromo-éthyloxy)imino]pyrrolo [1,2-a] indole on obtient le 9-[(2-phtalimido-éthyloxy)imino]pyrrolo[1,2-a]indole avec un rendement de 55%.
Point de fusion : 195°C

### Stade C : 9-[(2-amino-éthyloxy)imino]pyrrolo[1,2-a]indole

En procédant comme pour le stade C de l'exemple 40 mais en remplaçant le 3-méthyl-8-[(2-phtalimido-éthyloxy)imino]thiéno[3,2-b]pyrrolo[2,1-e]pyrrole(Z) par le 9-[(2-phtalimido-éthyloxy)imino] pyrrolo[1,2-a]indole on obtient le produit du titre dont on prépare ensuite le sel d'oxalate.
Rendement global (oxalate) : 48%
Point de fusion (oxalate) : 165°C décomposition (acétonitrile)
Infra rouge : ν cm⁻¹ (KBr) : 3280, 3090, 2940, 1710, 1620

### EXEMPLE 60 : 9-{[2(N-benzylamino)éthyloxy]imino}pyrrolo[1,2-a]indole

En procédant comme pour l'exemple 41 on obtient le composé du titre sous forme de base puis après salification avec de l'acide oxalique sous forme d'oxalate.
Rendement global (oxalate) : 16 %
Point de fusion (oxalate) : 230°C décomposition (acétonitrile)
Infra rouge : ν cm⁻¹ (KBr) : 3200, 2830, 1710, 1620, 1480 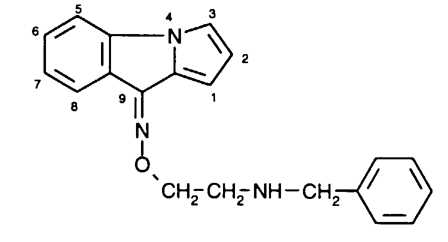 RMN ¹H : δ (ppm) (DMSO d₆) : 3,33 multiplet (2H, CH₂NH) ; 4,20 singulet (2H, CH₂-φ) ; 4,57 multiplet (2H, CH₂O) ; 6,33 doublet de doublet (1H, H₂) ; 6,77 doublet de doublet (1H, H₁); 7,06 multiplet (2H, NH⁺, OH) ; 7,40 multiplet (10H, C₆H₅, H₃, H₅, H₆, H₇, H₈).

### EXEMPLES 61 à 63

En procédant comme pour l'exemple 59 on obtient :
**EXEMPLE 61 : 6-chloro-9-[(2-amino-éthyloxy)imino]pyrrolo[1,2-a]indole**
**EXEMPLE 62 : 7-chloro-9-[(2-amino-éthyloxy)imino]pyrrolo[1,2-a]indole**
**EXEMPLE 63 : 7-méthyl-9-[(2-amino-éthyloxy)imino]pyrrolo[1,2-a]indole**

### EXEMPLE 64 à 67 :

En procédant comme pour l'exemple 49 on obtient :
**EXEMPLE 64 : 9-[(2-morpholino-éthyloxy)imino]pyrrolo[1,2-a]indole**
**EXEMPLE 65 : 9-[(2-pipéridino-éthyloxy)imino]pyrrolo[1,2-a]indole**
**EXEMPLE 66 : 7-chloro-9-{[2-(N,N-diéthylamino)éthyloxy]imino}pyrrolo[1,2-a]indole**
**EXEMPLE 67 : 7-méthyl-9-{[3-(N,N-diméthylamino)-2-méthyl-n-prop-1-yloxy]imino}pyrrolo[1,2-a] indole**

### EXEMPLE 68 et 69

En procédant comme pour l'exemple 1 on obtient :
**EXEMPLE 68 : 9-[(3-isopropylamino-2-hydroxy-n-prop-1-yloxy)imino]pyrrolo[1,2-a]indole**
**EXEMPLE 69 : 7-méthyl-9-[(3-isopropylamino-2-hydroxy-n-prop-1-yloxy)imino]pyrrolo [1,2-a]indole**

### EXEMPLES 70 à 72 :

En procédant comme pour les exemples 41 et 39, on obtient :
**EXEMPLE 70 : 8-{[2-(N-indan-2-ylamino)éthyloxy]imino}thiéno[2,3-d]pyrrolo[1,2-a]pyrrole**
**EXEMPLE 71 : 3-méthyl-8{[3-(N-indan-2-ylamino)-1-phénylprop-1-yloxy]imino}thiéno[2,3-d] pyrrolo[1,2-a]pyrrole**
**EXEMPLE 72 : 8-{[2-(N-méthyl-N-(3-trifluorométhyl)phénylprop-2-yl)éthyloxy]imino)-3-méthylthiéno [2,3-d]pyrrolo[1,2-a]pyrrole**

### EXEMPLES 73 à 80 :

En procédant comme pour l'exemple 49, on obtient :
**EXEMPLE 73 : 9-{[2-(N-benzylamino)n-prop-1-yloxy]imino}pyrrolo[1,2-a]indole**
**EXEMPLE 74 : 9-{[2-(N-benzylamino)-2-isobutyl-éthyloxy]imino}pyrrolo[1,2-a]indole**
**EXEMPLE 75 : 9-{[2-(N-benzylamino)-2-phényléthyloxy]imino}pyrrolo[1,2-a]indole**
**EXEMPLE 76 : 9-{[2-(N-benzyl-N-méthylamino)2-phényléthyloxy]imino}pyrrolo[1,2-a]indole**
**EXEMPLE 77 : 9-{[2-(N-phénylamino)-2-isobutyléthyloxy]imino}pyrrolo[1,2-a]indole**
**EXEMPLE 78 : 9-{[2-(N-phénéthylamino)-n-prop-1-yloxy]imino}pyrrolo[1,2-a]indole**
**EXEMPLE 79 : 9-{[2-(N-méthylamino)-2-phénéthyl]imino}pyrrolo[1,2-a]indole**
**EXEMPLE 80 : 9-{[2-(N-indan-2-ylamino)éthyloxy]imino}pyrrolo[1,2-a]indole**

### ETUDE PHARMACOLOGIQUE DES COMPOSES DE L'INVENTION

### EXEMPLE A : ETUDE DE LIAISON AUX RECEPTEURS DES COMPOSES DE L'INVENTION

### A-1 : Etude de liaison des composés de l'invention aux récepteurs sérotoninergiques 5-HT₃

La liaison des composés de l'invention aux récepteurs 5-HT₃ a été déterminée pour chaque composé par la mesure du déplacement du ³H-zacopride (ligand spécifique des récepteurs 5-HT₃) dans des homogénats d'area postrema de rat.

### PROTOCOLE:

Des aliquotes (50-100 µl) d'une suspension membranaire (2,5-5,0 mg de protéines/cm³) sont mises à incuber pendant 30 min à 25°C dans un tampon Tris-HCl, 25 mM, pH 7,4, en présence de 0,3-0,4 nM de 3^{H}-zacopdde (83 ci/mmol) (volume total : 0,5 cm³). L'incubation est arrêtée par filtration des échantillons au travers de filtres Whatman GF/B qui sont ensuite lavés et séchés. La radioactivité retenue sur les filtres est finalement comptée par spectrométrie en milieu liquide (Aquasol). La liaison non spécifique est estimée dans les mêmes conditions à partir d'échantillons contenant en plus 1 µM d'ondansetron (antagoniste 5-HT₃). Les courbes de déplacement obtenues en ajoutant des concentrations croissantes d'un composé à tester dans le milieu d'incubation sont analysées pour déterminer l'affinité des composés à tester pour les récepteurs 5-HT₃.

### A-2: Etude de la liaison des composés de l'invention au site de recapture de la sérotonine ainsi qu'aux récepteurs sérotoninergiques 5-HT₁, 5-HT_{1A}, 5-HT_{1B}, 5-HT_{1D}, 5-HT₂, 5-HT_{2C}, 5HT₄

La liaison des composés de l'invention est mesurée, selon les méthodes conventionnelles.
- pour le site de recapture de la sérotonine par le déplacement de ³H-paroxétine dans le cerveau de rat dont on a enlevé le cervelet,
- pour les récepteurs 5-HT₁ par le déplacement de 5-OH tryptamine dans le cortex frontal et le striatum de porc,
- pour les récepteurs 5-HT_{1A}, par le déplacement de 8-OH-DPAT dans des homogénats d'hippocampe de rat,
- pour les récepteurs 5-HT_{1B}, par le déplacement de 5-hydroxytryptamine des homogénats de cortex, striatum, et globus pallidus de rat,
- pour les récepteurs 5-HT_{1D}, par le déplacement de 5-OH-tryptamine dans des homogénats de cortex, de striatum et de globus pallidus du rat,
- pour les récepteurs 5-HT₂, par le déplacement d'aminoiodo kétansérine dans des homogénats de cortex frontal de rat,
- pour les récepteurs 5-HT_{2C}, par le déplacement de N-méthyl mésulergine dans des homogénats de cortex frontal et d'hippocampe de rat,
- pour les récepteurs 5-HT₄ par le déplacement de GR 113808 dans le striatum ou l'hippocampe de cobaye.

### CONCLUSION

Il apparaît que les composés de l'invention possèdent une très forte affinité pour les récepteurs sérotoninergiques 5-HT₃ et/ou 5HT_{2C} ainsi que pour les sites de recapture de la sérotonine. Cette forte affinité se double d'une haute sélectivité par rapport aux autres récepteurs sérotoninergiques.

Les valeurs de IC₅₀ obtenues pour les composés de l'invention sont rassemblées dans le tableau (I) suivant:

**- Tableau (I) -**

| *Valeurs de IC*_{*50*} *obtenues pour les sites de recapture de la sérotonine et les récepteurs sérotoninergiques* | | | | | | | | |
|---|---|---|---|---|---|---|---|---|
| ***Ex.*** | ***IC***_{***50***} ***(M)*** | | | | | | | |
| | Sites de recapture de la sérotonine | 5-HT₁ | 5-HT_{1A} | 5-HT_{1B} | 5-HT_{1D} | 5-HT₂ | 5-HT_{2C} | 5-HT₃ |
| 3 | 1,2.10⁻⁷ | 2,2.10⁻⁶ | 5,8.10⁻⁶ | 2,7.10⁻⁶ | 4,8.10⁻⁷ | 3,1.10⁻⁶ | 2,1.10⁻⁶ | 1,1.10⁻¹⁰ |
| 4 | 8,9.10⁻⁷ | 5,5.10⁻⁷ | 4,8.10⁻⁷ | 6,1.10⁻⁷ | 4,1.10⁻⁷ | 5,0.10⁻⁶ | 8,2.10⁻⁷ | 3,5.10⁻⁹ |
| 13 | 6,0.10⁻⁷ | 1,6.10⁻⁵ | > 10⁻⁴ | 3,1.10⁻⁵ | 1,1.10⁻⁵ | 1,4.10⁻⁵ | 1,0.10⁻⁶ | 2,0.10⁻⁹ |
| 14 | 1,1.10⁻⁶ | 1,1.10⁻⁵ | 2,2.10⁻⁵ | 3,2.10⁻⁵ | 7,0.10⁻⁶ | 4,2.10⁻⁵ | 1,8.10⁻⁵ | 2,5.10⁻⁹ |
| 15 | 1,3.10⁻⁷ | 1,1.10⁻⁵ | 2,1.10⁻⁵ | 6,4.10⁻⁶ | 4,0.10⁻⁶ | 1,3.10⁻⁵ | 8,4.10⁻⁷ | 5,0.10⁻⁹ |
| 17 | 3,0.10⁻⁷ | 3,0.10⁻⁶ | 1,6.10⁻⁶ | 3,6.10⁻⁶ | 3,2.10⁻⁶ | 3,7.10⁻⁶ | 7,1.10⁻⁷ | 2,0.10⁻⁹ |
| 18 | 2,6.10⁻⁷ | 5,1.10⁻⁶ | 5,7.10⁻⁶ | 1,4.10⁻⁵ | 7,5.10⁻⁶ | 5,2.10⁻⁶ | 1,2.10⁻⁶ | 4,0.10⁻⁹ |
| 22 | 1,1.10⁻⁷ | 6,9.10⁻⁶ | 9,0.10⁻⁶ | 1,1.10⁻⁵ | 6,4.10⁻⁶ | 1,1.10⁻⁵ | 1,6.10⁻⁶ | 1,0.10⁻⁹ |
| 23 | 5,0.10⁻⁶ | 9,6.10⁻⁶ | 5,2.10⁻⁵ | 2,4.10⁻⁵ | 1,1.10⁻⁵ | 1,4.10⁻⁵ | 2,0.10⁻⁶ | 5,4.10⁻⁹ |
| 39 | 7,2.10⁻⁸ | 1,0.10⁻⁵ | 1,1.10⁻⁵ | >10⁻⁴ | 6,7.10⁻⁶ | 2,2.10⁻⁶ | 6,1.10⁻⁸ | 2,0.10⁻⁷ |
| 40 | 5,8.10⁻⁷ | 9,0.10⁻⁷ | 3,1.10⁻⁶ | 9,2.10⁻⁷ | 2,7.10⁻⁶ | 1,5.10⁻⁵ | 1,8.10⁻⁷ | 2,4.10⁻⁸ |
| 41 | 1,1.10⁻⁷ | 2,7.10⁻⁶ | 9,2.10⁻⁶ | 4,4.10⁻⁶ | 1,1.10⁻⁵ | 4,1.10⁻⁶ | 4,13.10⁻⁷ | 2,9.10⁻⁸ |
| 49 | 1,2.10⁻⁵ | 2,4.10⁻⁶ | 2,1.10⁻⁵ | 1,4.10⁻⁶ | 3,1.10⁻⁶ | 7,6.10⁻⁶ | 1,1.10⁻⁵ | 8,6.10⁻⁸ |
| 52 | 1,3.10⁻⁵ | 2,0.10⁻⁶ | 3,3.10⁻⁶ | 3,8.10⁻⁶ | 8,1.10⁻⁷ | 8,4.10⁻⁶ | 2,3.10⁻⁶ | 4,4.10⁻⁸ |
| 53 | 2,4.10⁻⁵ | 8,3.10⁻⁶ | 1,4.10⁻⁵ | 3,5.10⁻⁵ | 5,5.10⁻⁶ | 2,2.10⁻⁵ | 6,0.10⁻⁶ | 3,7.10⁻⁸ |
| 54 | 9,3.10⁻⁷ | 1,4.10⁻⁷ | 1,1.10⁻⁶ | 1,1.10⁻⁶ | 6,9.10⁻⁷ | 1,4.10⁻⁵ | 1,4.10⁻⁶ | 5,7.10⁻⁹ |
| 55 | 3,2.10⁻⁷ | 2,8.10⁻⁶ | 3,8.10⁻⁶ | 4,4.10⁻⁶ | 1,4.10⁻⁶ | 1,2.10⁻⁵ | 4,1.10⁻⁶ | 5,3.10⁻⁹ |

### EXEMPLE B : ETUDE DE L'ACTIVITE ANXIOLYTIQUE - TEST DIT DES CAGES CLAIRES/ OBSCURES CHEZ LA SOURIS

Les effets anxiolytiques des composés de l'invention ont été étudiés selon le test dit des cages claires/obscures chez la souris (Crawley et al. (1981), Pharmacol. Biochem. Behav., 15, pp 695-699).

### PROTOCOLE

Il s'agit de deux cages de taille égale (20x20x14 cm) en PVC. L'une est fortement éclairée par une lampe de 100 W (lumière "froide"), l'autre est obscurcie. Les deux cages sont séparées l'une de l'autre au moyen d'un petit tunnel opaque (5x7 cm). Les souris sont individuellement introduites au niveau du tunnel et, dès qu'elles ont pénétré pour la première fois dans la cage obscure, on enregistre, au moyen de claviers reliés à un oridinateur, durant 5 minutes, le temps passé par les animaux dans la cage éclairée ainsi que le nombre des transitions entre la cage obscure et la cage éclairée. Chaque groupe expérimental comprend au minimum 15 animaux.

### RESULTATS

Les composés de l'invention administrés par voie intrapéritonéale entrainent par rapport au témoin une augmentation du temps passé par la souris dans la cage éclairée ainsi qu'une augmentation du nombre de transitions entre la cage obscure et la cage éclairée. Cette augmentation significative des deux paramètres étudiés montre la remarquable activité anxiolytique des composés de l'invention.

**- Tableau II -**

| Etude de l'activité anxiolytique (Test des cages claires/obscures) | |
|---|---|
| (Résultats en pourcentage par rapport au groupe témoin) | |
| Exemple 22 | Dose : 15 mg/kg, voie i.p.) |
| Temps passé dans la cage éclairée | + 92% * |
| Nombre de passages | + 76% * |

| | |
|---|---|
| * : p < 0,5 | |

### EXEMPLE C : ETUDE DE L'ACTIVITE ANTIDEPRESSIVE. TEST DIT DE LA NAGE FORCEE CHEZ LA SOURIS.

Les effets antidépresseurs des composés de l'invention ont été étudiés selon le test de la nage forcée chez la souris. (Behavioural despair in mice : a primary screening test for antidepressants, Porsolt R.D. et al, Arch. Int. Pharmacodyn., (1977), 229, pp. 327-336).

### PROTOCOLE

Les animaux sont placés pendant 6 minutes dans un cylindre contenant de l'eau, et duquel il ne peuvent pas s'échapper. La durée d'immobilité des animaux est mesurée pendant les 4 dernières minutes du test. Les composés à tester sont administrés en une seule prise avant le test (l'imipramine étant utilisée en tant que composé de référence).

### RESULTATS

L'immobilité de la souris dans l'eau reflète un état dépressif dû à la situation dans laquelle elle se trouve (situation d'aversion qu'elle ne peut changer). Les composés de l'invention diminuent très significativement par rapport aux témoins la durée d'immobilité des animaux ce qui, dans ce test, est révélateur de propriétés antidépressives.

**- Tableau III -**

| Etude de l'activité antidépressive (Test de la nage forcée) | | |
|---|---|---|
| (Durée d'immobilité exprimée en pourcentage par rapport au groupe témoin) | | |
| Exemple | Dose (mg/kg, voie i.p.) | |
| | 32 | 64 |
| 3 | -39% * | -86% *** |
| 39 | -53% ** | - |

| | | |
|---|---|---|
| * : p < 0,5 | | |
| ** : p < 0,01 | | |
| *** : p < 0,001 | | |

### EXEMPLE D : COMPRIMES DOSES A 5 MG D'OXALATE DE 3-METHYL-8-{[2-(N,N-DIMETHYLAMINO)ETHYLOXY]IMINO}THIENO[3,2-b]PYRROLO[1,2-a]PYRROLE (Z)

Formule de préparation pour 1000 comprimés

| | |
|---|---|
| oxalate de 3-méthyl 8-{[2-(N,N-diméthylamino)éthyloxy]imino}thiéno[2,3-d]pyrrolo[1,2-a]pyrrole (Z) | 5 g |
| Amidon de maïs | 70 g |
| Amidon de blé | 70 g |
| Lactose | 300 g |
| Stéarate de magnésium | 1 g |
| Silice | 1 g |
| Hydroxypropylcellulose | 2 g |

## Revendications

1. Composés de formule générale (I) : 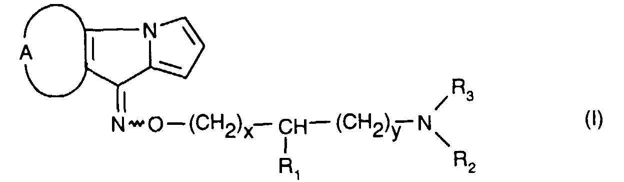 dans laquelle
• R₁ est choisi parmi :
- hydrogène,
- alkyle,
- alcényle,
- cycloalkyle,
- cycloalkylalkyle dont la chaîne alkyle linéaire ou ramifiée comporte de 1 à 4 atomes de carbone,
- hydroxy,
- alkoxy,
- phényle éventuellement substitué,
- phénylalkyle éventuellement substitué dont la chaîne alkyle linéaire ou ramifiée comporte de 1 à 4 atomes de carbone,
- phénoxy éventuellement substitué, et
- phénylalkoxy éventuellement substitué dont la chaîne alkyle linéaire ou ramifiée comporte de 1 à 4 atomes de carbone,
ou bien R₁ forme avec R₂ et la chaîne 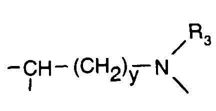 qui les porte un système cyclique azoté comportant de 5 à 8 sommets,
• R₂ et R₃ sont choisis chacun indépendamment l'un de l'autre parmi :
- hydrogène,
- alkyle,
- alcényle,
- cycloalkyle,
- indanyle éventuellement substitué,
- cycloalkylalkyle dont la chaîne alkyle linéaire où ramifiée comporte de 1 à 4 atomes de carbone,
- phényle éventuellement substitué, et
- phénylalkyle éventuellement substitué dont la chaîne alkyle linéaire ou ramifiée comporte de 1 à 4 atomes de carbone,
ou bien R₂ et R₃ forment ensemble avec l'atome d'azote qui les porte un système hétérocyclique choisi parmi :
〉N-R₄ ,
avec :
m nombre entier pouvant prendre les valeurs 0, 1, 2, 3, ou 4,
n nombre entier pouvant prendre les valeurs 0, 1, ou 2,
Y' étant choisi parmi l'oxygène, le soufre et le groupement 〉N-R₄, 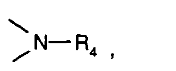
R₄ étant choisi parmi :
- hydrogène,
- alkyle, et
- -(CH₂)_{σ}- phényle éventuellement substitué, avec σ nombre entier pouvant prendre les valeurs 0, 1, 2, 3, ou 4,
• x et y représentent des nombres entiers identiques ou différents pouvant prendre chacun indépendamment l'un de l'autre les valeurs 0, 1, 2, 3, ou 4,
• A représente :
* soit un groupement de formule (α) : 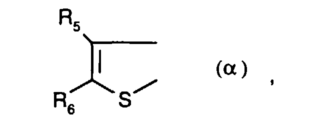 et forme ainsi avec le système hétérocyclique qui le porte un thiéno[2,3-d]pyrrolo[1,2-a] pyrrole de formule générale (I_{α}) : 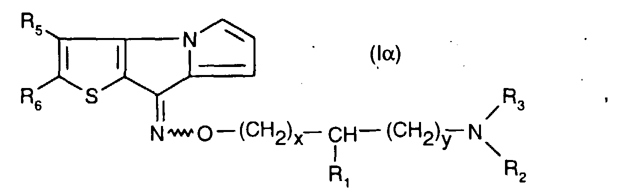 dans laquelle R₁, R₂, R₃, x, et y sont tels que définis précédemment et R₅ et R₆, identiques ou différents représentent indépendamment l'un de l'autre un groupement choisi parmi :
- hydrogène,
- alkyle,
- hydroxy,
- alkoxy,
- halogène,
- trifluorométhyle,
- alkoxycarbonyle,
- -(CH₂)ₚ- phényle éventuellement substitué avec p nombre entier pouvant prendre les valeurs 0, 1, 2, 3, ou 4, et
- -O-(CH₂)_{p'}-phényle éventuellement substitué avec p' nombre entier pouvant prendre les valeurs 0, 1, 2, 3, ou 4,
* soit un groupement de formule (β) : 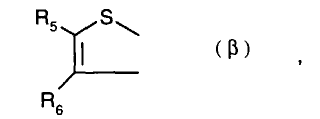 et forme ainsi avec le système hétérocyclique qui le porte un thiéno[3,2-d]pyrrolo[1,2-a] pyrrole de formule générale (I_{β}) : 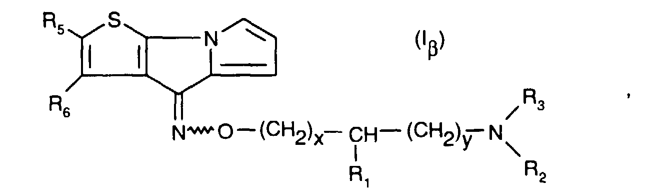 dans laquelle R₁, R₂, R₃, R₅, R₆, x et y sont tels que définis précédemment,
* soit un groupement de formule (γ) : 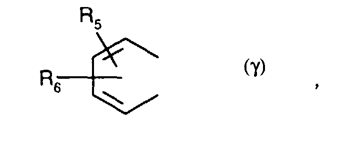 et forme ainsi avec le système hétérocyclique qui le porte un pyrrolo[1,2-a]indole de formule générale (I_{γ}) : 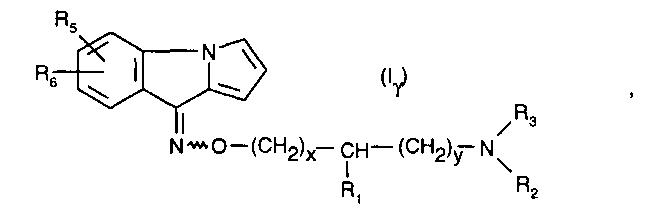 dans laquelle R₁, R₂, R₃, R₅, R₆, x et y sont tels que définis précédemment,
- leurs isomères cis ou trans au niveau de l'éther d'oxime,
- leur énantiomères ou diastéréoisomères, et
- leurs hydrates et/ou sels d'addition à un acide ou à une base, pharmaceutiquement acceptables,
étant entendu que, sauf précision contraire :
- les termes "alkyle", et "alkoxy" représentent des groupements linéaires ou ramifiés possédant de 1 à 6 atomes de carbone,
- le terme "alcényl" représente un groupement carboné insaturé linéaire ou ramifié possédant de 2 à 6 atomes de carbone,
- le terme "cycloalkyle" représente un système carboné cyclique comportant de 3 à 8 sommets,
- les expressions "indanyle éventuellement substitué", "phényle éventuellement substitué", "phénoxy éventuellement substitué", "phénylalkyle éventuellement substitué", "(CH₂)_{σ}-phényle éventuellement substitué", "-(CH₂)ₚ-phényle éventuellement substitué", "-O-(CH₂)_{p'}-phényle éventuellement substitué" et "phénylalkoxy éventuellement substitué", signifient que le radical phényle peut être éventuellement substitué par un ou plusieurs substituants identiques ou différents choisis parmi alkyle, alkoxy, hydroxy, halogène, trifluorométhyle, nitrile et nitro.

2. Composés selon la revendication 1 pour lesquels A représente un groupement de formule (α) : 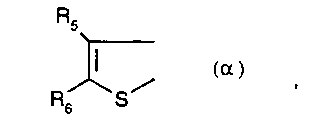 et forme ainsi avec le système hétérocyclique qui le porte un thiéno [2,3-d]pyrrolo[1,2-a]pyrrole de formule générale (I_{α}) : 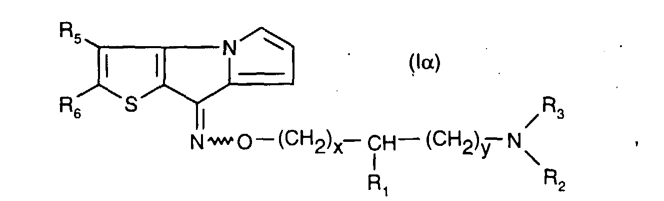 dans laquelle R₁, R₂, R₃, x, et y sont tels que définis dans la revendication 1 et R₅ et R₆, identiques ou différents représentent indépendemment l'un de l'autre un groupement choisi parmi :
- hydrogène,
- alkyle,
- hydroxy,
- alkoxy,
- halogène,
- trifluorométhyle,
- alkoxycarbonyle,
- -(CH₂)ₚ- phényle éventuellement substitué avec p nombre entier pouvant prendre les valeurs 0, 1, 2, 3, ou 4,
- -O-(CH₂)_{p'}- phényle éventuellement substitué avec p' nombre entier pouvant prendre les valeurs 0, 1, 2, 3, ou 4,
- leurs isomères cis ou trans au niveau de l'éther d'oxime,
- leur énantiomères ou diastéréoisomères, et
- leurs hydrates et/ou sels d'addition à un acide ou à une base pharmaceutiquement acceptables,
étant entendu que, sauf précision contraire
- les termes "alkyle", et "alkoxy" représentent des groupements linéaires ou ramifiés possédant de 1 à 6 atomes de carbone,
- les expressions "-(CH₂)ₚ-phényle éventuellement substitué" et "-O-(CH₂)_{p'}-phényle éventuellement substitué" , signifient que le radical phényle peut être éventuellement substitué par un ou plusieurs substituants identiques ou différents choisis parmi alkyle, alkoxy, hydroxy, halogène, trifluorométhyle, nitrile et nitro.

3. Composés selon la revendication 1 pour lesquels A représente un groupement de formule (β) : 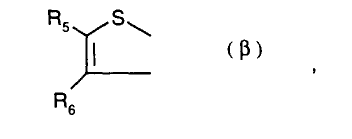 et forme ainsi avec le système hétérocyclique qui le porte un thiéno[3,2-d]pyrrolo[1,2-a] pyrrole de formule générale (I_{β}) : 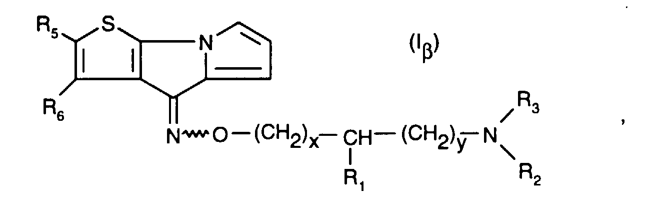 dans laquelle R₁, R₂, R₃, x, et y sont tels que définis dans la revendication 1 et R₅ et R₆, identiques ou différents représentent indépendemment l'un de l'autre un groupement choisi parmi :
- hydrogène,
- alkyle,
- hydroxy,
- alkoxy,
- halogène,
- trifluorométhyle,
- alkoxycarbonyle,
- -(CH₂)ₚ-phényle éventuellement substitué avec p nombre entier pouvant prendre les valeurs 0, 1, 2, 3, ou 4,
- -O-(CH₂)_{p'}-phényle éventuellement substitué avec p' nombre entier pouvant prendre les valeurs 0, 1, 2, 3, ou 4,
- leurs isomères cis ou trans au niveau de l'éther d'oxime,
- leur énantiomères ou diastéréoisomères, et
- leurs hydrates et/ou sels d'addition à un acide ou à une base, pharmaceutiquement acceptables,
étant entendu que, sauf précision contraire :
- les termes "alkyle", et "alkoxy" représentent des groupements linéaires ou ramifiés possédant de 1 à 6 atomes de carbone,
- les expressions "-(CH₂)ₚ-phényle éventuellement substitué" et "-O-(CH₂)_{p'}-phényle éventuellement substitué", signifient que le radical phényle peut être éventuellement substitué par un ou plusieurs substituants identiques ou différents choisis parmi alkyle, alkoxy, hydroxy, halogène, trifluorométhyle nitrile et nitro.

4. Composés selon la revendication 1 pour lesquels A représente un groupement de formule (γ) : 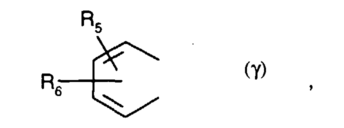 et forme avec le système hétérocyclique qui le porte un pyrrolo[1,2-a]indole de formule générale (I_{γ}) : 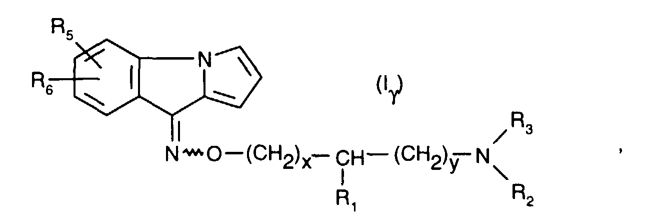 dans laquelle R₁, R₂, R₃, x, et y sont tels que définis dans la revendication 1 et R₅ et R₆, identiques ou différents représentent indépendemment l'un de l'autre un groupement choisi parmi :
- hydrogène,
- alkyle,
- hydroxy,
- alkoxy,
- halogène,
- trifluorométhyle,
- alkoxycarbonyle,
- -(CH₂)ₚ-phényle éventuellement substitué avec p nombre entier pouvant prendre les valeurs 0, 1, 2, 3, ou 4,
- -O-(CH₂)_{p'}-phényle éventuellement substitué avec p' nombre entier pouvant prendre les valeurs 0, 1, 2, 3, ou 4,
- leurs isomères cis ou trans au niveau de l'éther d'oxime,
- leur énantiomères ou diastéréoisomères, et
- leurs hydrates et/ou sels d'addition à un acide ou à une base, pharmaceutiquement acceptables,
étant entendu que, sauf précision contraire :
- les termes "alkyle", et "alkoxy" représentent des groupements linéaires ou ramifiés possédant de 1 à 6 atomes de carbone,
- les expressions "-(CH₂)ₚ-phényle éventuellement substitué" et "-O-(CH₂)_{p'}-phényle éventuellement substitué" signifient que le radical phényle peut être éventuellement substitué par un ou plusieurs substituants identiques ou différents choisis parmi alkyle, alkoxy, hydroxy, halogène, trifluorométhyle nitrile et nitro.

5. Composé selon l'une quelconque des revendications 1 et 2 qui est le 3-méthyl-8-{[3-(N,N-diméthylamino)-1-phényl-n-prop-1-yloxy]imino}thiéno[2,3-d]pyrrolo[1,2-a]pyrrole dont la formule est représentée ci-après, ainsi que ses isomères Z et E et ses énantiomères isolés ou sous forme de mélange et ses sels d'addition à un acide, pharmaceutiquement acceptables.

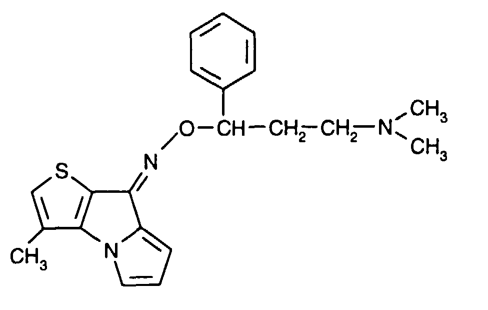

6. Composé selon l'une quelconque des revendications 1 et 2 qui est le 3-méthyl-8-{[2-(N-benzylamino)éthyloxy]imino}thiéno[2,3-d]pyrrolo[1,2-a]pyrrole dont la formule est représentée ci-après, ainsi que ses isomères Z et E isolés ou sous forme de mélange et ses sels d'addition à un acide, pharmaceutiquement acceptables.

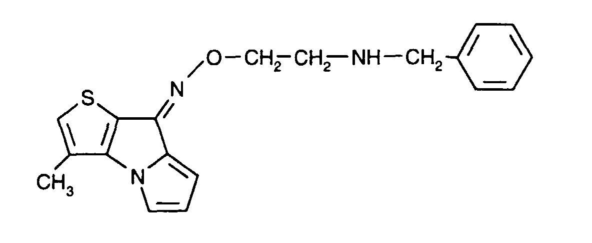

7. Composé selon l'une quelconque des revendications 1 et 2 qui est le 3-méthyl-8-{[2-(N,N-diméthylamino)-n-prop-1-yloxy]imino}thiéno[2,3-d]pyrrolo[1,2-a]pyrrole dont la formule est représentée ci-après, ainsi que ses isomère Z et E et ses énantiomères isolés ou sous forme de mélange et ses sels d'addition à un acide, pharmaceutiquement acceptables.

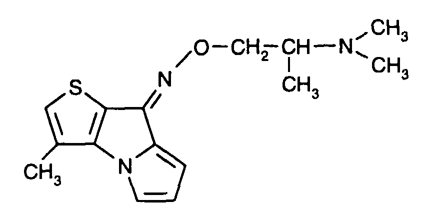

8. Composé selon l'une quelconque des revendications 1 et 3 qui est le 8-{[2-(N,N-diméthylamino)éthyloxy]imino}thiéno[3,2-d]pyrrolo[1,2-a]pyrrole dont la formule est représentée ci-après ainsi que ses isomères Z et E isolés ou sous forme de mélange et ses sels d'addition à un acide, pharmaceutiquement acceptables.

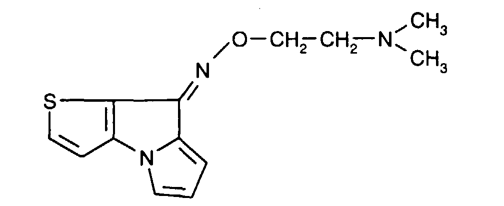

9. Composé selon l'une quelconque des revendications 1 et 4 qui est le 9-{[2-(N,N-diméthylamino)-n-prop-1-yloxy]imino}pyrrolo[1,2-a]indole dont la formule est représentée ci-après, ainsi que ses isomères Z et E et ses énantiomères isolés ou sous forme de mélange et ses sels d'addition à un acide, pharmaceutiquement acceptables.

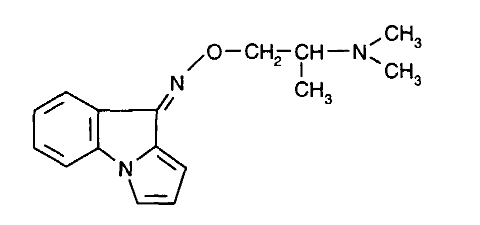

10. Procédé de préparation des composés de formule (I) selon la revendication 1 caractérisé en ce que l'on cyclise avec de l'oxychlorure de phosphore en présence ou en absence d'un solvant, un composé de formule (II) : 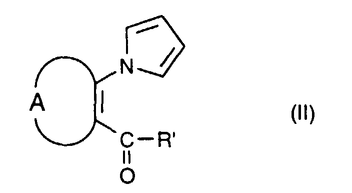 dans laquelle A est tel que défini la revendication 1 et R' représente, un radical hydroxy, alkoxy ou 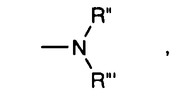 avec R" et R‴ différents de H pouvant être des alkyles ou former ensemble avec l'atome d'azote qui les porte une amine cyclique choisie parmi pyrrolidine, pipéridine ou morpholine, de manière à obtenir après cyclisation une cétone tricyclique de formule (III) : 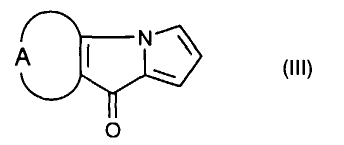 dans laquelle A est tel que défini précedemment, que l'on fait ensuite réagir avec de l'hydroxylamine de manière à obtenir l'oxime de formule (IV) : 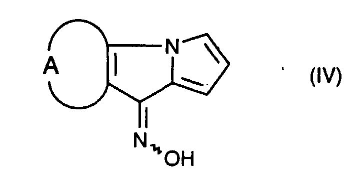 dans laquelle A est tel que défini précédemment et que l'on peut, si nécessaire et si on le désire, séparer en ses isomère E et Z, avant de le faire réagir avec un composé de formule (V) : 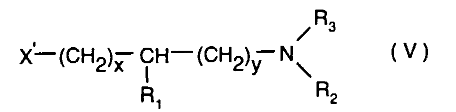 dans laquelle R_{1,} R₂, R₃, x et y sont tels que définis la revendication 1 et X' représente un groupement partant, de manière à accéder au composé de formule (I) : 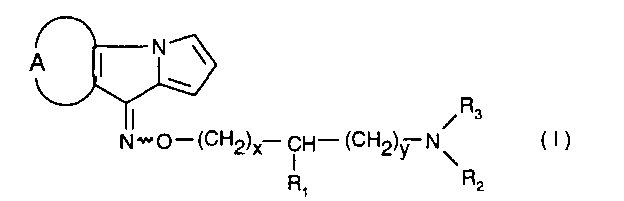 dans laquelle A, R₁, R₂, R₃, x et y sont tels que définis précédemment et que l'on peut si nécessaire et si on le désire séparer en ses isomères Z et E et en ses éventuels énantiomères ou diastéréoisomères, et/ou salifier avec un acide ou avec une base, pharmaceutiquement acceptables.

11. Procédé de préparation des composés de formule (I) selon la revendication 1 caractérisé en ce que l'on cyclise avec de l'oxychlorure de phosphore en présence ou en absence d'un solvant, un composé de formule (II) : 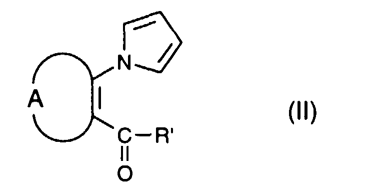 dans laquelle A est tel que défini dans la revendication 1 et R' représente un radical hydroxy, alkoxy ou 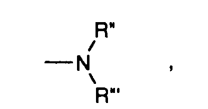 avec R" et R‴ différents de H pouvant être des alkyles ou former ensemble, avec l'atome d'azote qui les porte une amine cyclique choisie parmi pyrrolidine, pipéridine ou morpholine, de manière à obtenir après cyclisation une cétone tricyclique de formule (III) : 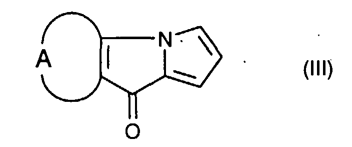 dans laquelle A est tel que défini précédemment, que l'on fait ensuite réagir avec une amine de formule générale (VI) : 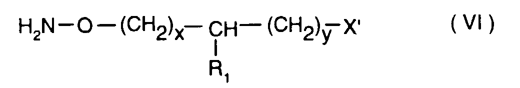 dans laquelle R_{1,} x et y sont tels que définis la revendication 1 et X' représente un groupement partant, de manière à obtenir un composé de formule (VII) : 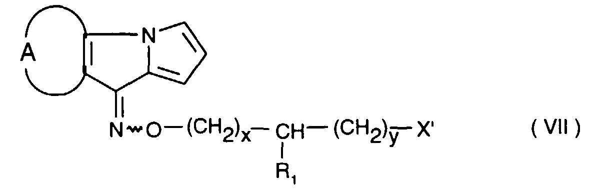 dans laquelle A, R₁, X', x et y sont tels que définis précédemment que l'on peut :
a) soit faire réagir avec une amine de formule générale (VIII) : 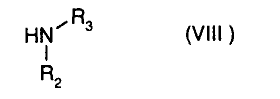 dans laquelle R₂ et R₃ sont tels que définis la revendication 1 de manière à accéder au composé de formule (I) : 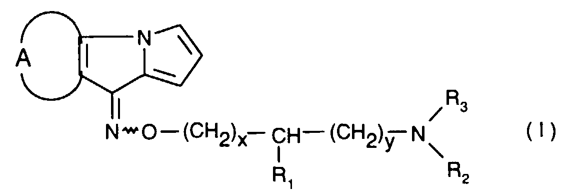 dans laquelle A, R₁, R₂, R₃, x et y sont tels que définis précédemment et que l'on peut si nécessaire et si on le désire séparer en ses isomères Z et E et en ses éventuels énantiomères ou diastéréoisomères et/ou transformer avec un acide ou une base, en sels pharmaceutiquement acceptables,
b) soit faire réagir avec du phtalimidure de potassium de manière à obtenir le phtalimide de formule (IX) : 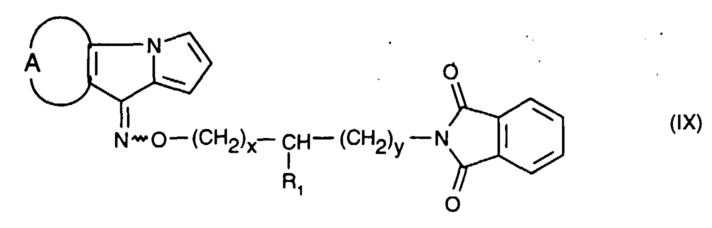 dans laquelle A, R₁, x et y sont tels que définis précedemment, que l'on hydrolyse ensuite en présence d'hydrazine pour accéder au composé de formule (I_{A}) : 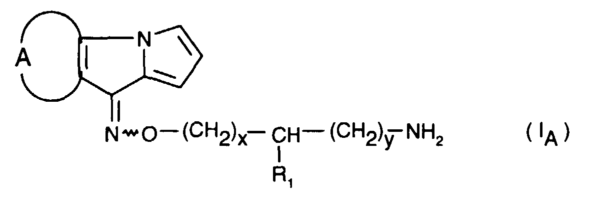 dans laquelle A, R₁, x et y sont tels que définis précédemment, cas particulier des composés de formule (I) pour lesquels R₂=R₃=H, que l'on peut si nécessaire et si on le désire séparer en ses isomères Z et E et en ses éventuels énantiomères ou diastéréoisomères, et/ou transformer avec un acide ou une base, en sels pharmaceutiquement acceptables,
composé de formule générale (I_{A}) que l'on peut si on le désire alkyler avec un composé de formule (X) :
X'-R₅ (X)
dans laquelle X' est tel que défini précédemment et R₅ possède la même signification que R₂ avec la réserve que R₅ ne peut représenter ni un hydrogène ni un phényle éventuellement substitué, de manière à accéder au composé de formule (I_{B}) : 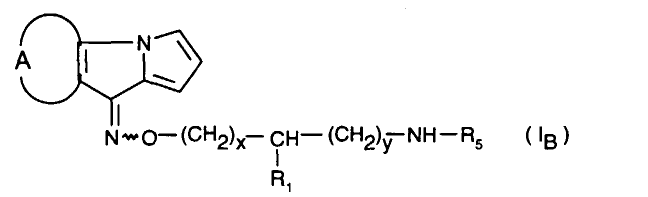 dans laquelle A, R₁, R₅, x et y sont tels que définis précédemment, cas particulier des composés de formule (I) que l'on peut si nécessaire et si on le désire séparer en ses isomères Z et E et en ses éventuels enantiomères ou diastéréoisomères et/ou transformer avec un acide ou une base, en sels pharmaceutiquement acceptables.

12. Procédé de préparation des composés de formule (I_{C}) : 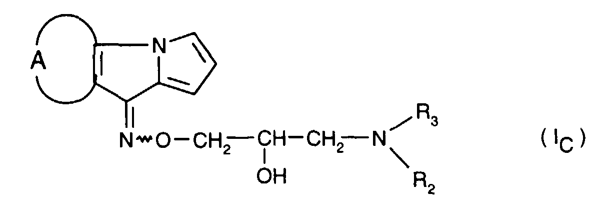 cas particulier des composés de formule générale (I) selon la revendication 1 pour lesquels A, R₂ et R₃ sont tels que définis dans la revendication 1 avec x=y=1 et R₁ représentant un groupement hydroxy, caractérisé en ce que l'on cyclise avec de l'oxychlorure de phosphore en présence ou en absence de solvant, un composé de formule (II) : 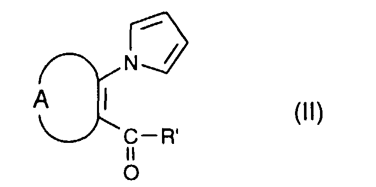 dans laquelle A est tel que défini précédemment et R' représente un radical hydroxy, alkoxy ou 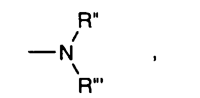 avec R" et R‴ différents de H pouvant être des alkyles ou former ensemble, avec l'atome d'azote qui les porte, une amine cyclique choisie parmi pyrrolidine, pipéridine ou morpholine,
de manière à obtenir après cyclisation une cétone tricyclique de formule (III) : 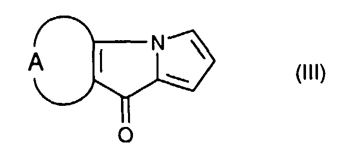 dans laquelle A est tel que défini précédemment, que l'on fait ensuite réagir avec de l'hydroxylamine de manière à obtenir l'oxime de formule générale (IV) : 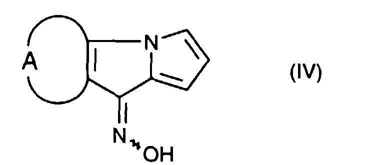 dans laquelle A est tel que défini précédemment et que l'on peut, si nécessaire et si on le désire, séparer en ses isomères E et Z avant de le faire réagir, après métallation par un hydrure métallique, avec une épihalohydrine de formule (XI) : 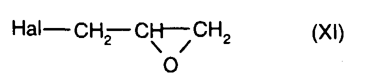 dans laquelle Hal représente un atome d'halogène, pour obtenir après réaction du composé intermédiaire ainsi formé avec une amine de formule (VIII) : 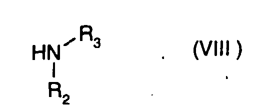 dans laquelle R₂ et R₃ sont tels que définis précédemment, un composé de formule (I_{C}) telle que définie précédemment, que l'on peut si nécessaire et si on le désire séparer en ses isomères Z et E et en ses éventuels énantiomères ou diastéréoisomères, et/ou transformer avec un acide ou une base, en sels pharmaceutiquement acceptables.

13. Composition pharmaceutique contenant comme principe actif au moins un des composés de formule (I) selon la revendication 1, sous forme pure ou sous forme d'un mélange d'isomères ou un de ses sels d'addition avec un acide ou une base, pharmaceutiquement acceptables, en combinaison avec un ou plusieurs excipients ou véhicules pharmaceutiquement acceptables.

14. Composition pharmaceutique selon la revendication 13 utile dans la prévention et le traitement de l'anxiété, de la dépression, du stress, des psychoses, des troubles obsessionnels compulsifs de la schizophrénie, des troubles du système nerveux central, de la migraine, des troubles de la mémoire, des troubles du comportement et de la prise alimentaire, de l'alcoolisme, de la douleur, ainsi que dans la prévention et le traitement du vomissement et des désordres de la vidange gastrique.

## Patentansprüche

1. Verbindungen der allgemeinen Formel (I): 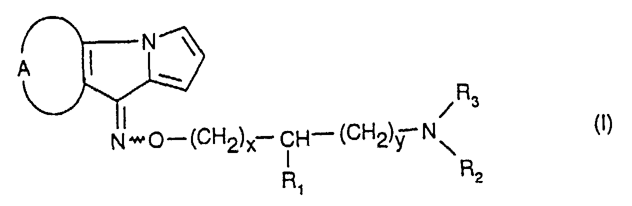 in der
• R₁ ausgewählt ist aus:
- Wasserstoff,
- Alkyl,
- Alkenyl,
- Cycloalkyl,
- Cycloalkylalkyl, dessen geradkettige oder verzweigte Alkylkette 1 bis 4 Kohlenstoffatome aufweist,
- Hydroxy,
- Alkoxy,
- gegebenenfalls substituiertem Phenyl,
- gegebenenfalls substituiertem Phenylalkyl, dessen geradkettige oder verzweigte Alkylkette 1 bis 4 Kohlenstoffatome aufweist,
- gegebenenfalls substituiertem Phenoxy und
- gegebenenfalls substituiertem Phenylalkoxy, dessen geradkettige oder verzweigte Alkylkette 1 bis 4 Kohlenstoffatome aufweist,
oder R₁ zusammen mit R₂ und der sie tragenden Kette 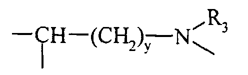 ein Stickstoff-haltiges cyclisches System mit 5 bis 8 Kettengliedern bildet,
• R₂ und R₃ jeweils unabhängig voneinander ausgewählt sind aus:
- Wasserstoff,
- Alkyl,
- Alkenyl,
- Cycloalkyl,
- gegebenenfalls substituiertem Indanyl,
- Cycloalkylalkyl, dessen geradkettige oder verzweigte Alkylkette 1 bis 4 Kohlenstoffatome aufweist,
- gegebenenfalls substituiertem Phenyl und
- gegebenenfalls substituiertem Phenylalkyl, dessen geradkettige oder verzweigte Alkylkette 1 bis 4 Kohlenstoffatome aufweist,
oder R₂ und R₃ zusammen mit dem sie tragenden Stickstoffatom ein heterocyclisches System bilden, ausgewählt aus: 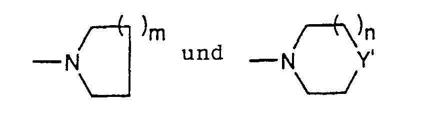 worin:
m eine ganze Zahl mit Werten von 0, 1, 2, 3 oder 4,
n eine ganze Zahl mit Werten von 0, 1 oder 2 darstellen und
Y' ausgewählt ist aus Sauerstoff, Schwefel und der Gruppe
〉N-R₄ ,
R₄ ausgewählt ist aus:
- Wasserstoff,
- Alkyl und
- gegebenenfalls substituiertem -(CH₂)_{σ}-Phenyl, worin σ eine ganze Zahl mit Werten von 0, 1, 2, 3 oder 4 darstellt,
• x und y gleichartige oder verschiedene ganze Zahlen darstellen, die jeweils unabhängig voneinander die Werte 0, 1, 2, 3 oder 4 besitzen können,
• A:
* entweder eine Gruppe der Formel (a) darstellt: 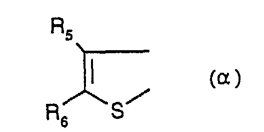 und in dieser Weise zusammen mit dem sie tragenden heterocyclischen System ein Thieno[2,3-d]pyrrolo[1,2-a]pyrrol der allgemeinen Formel (I_{α}) bildet: 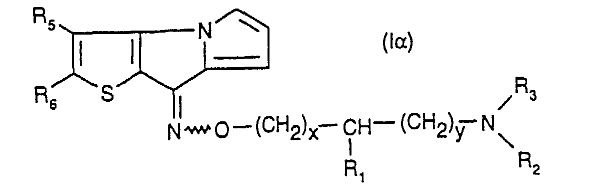 in der R₁, R₂, R₃, x und y die oben angegebenen Bedeutungen besitzen und R₅ und R₆, die gleichartig oder verschieden sein können, unabhängig voneinander eine Gruppe bedeuten ausgewählt aus:
- Wasserstoff,
- Alkyl,
- Hydroxy,
- Alkoxy,
- Halogen,
- Trilfuormethyl,
- Alkoxycarbonyl,
- gegebenenfalls substituiertem -(CH₂)ₚ-Phenyl, worin p für eine ganze Zahl mit Werten von 0, 1, 2, 3 oder 4 steht, und
- gegebenenfalls substituiertem -O-(CH₂)_{p'}-Phenyl, worin p' für eine ganze Zahl mit Werten von 0, 1, 2, 3 oder 4 steht,
* oder eine Gruppe der Formel (β) darstellt: 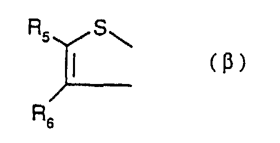 und in dieser Weise zusammen mit dem sie tragenden heterocyclischen System ein Thieno[3,2-d]pyrrolo[1,2-a]pyrrol der allgemeinen Formel (I_{β}) bildet: 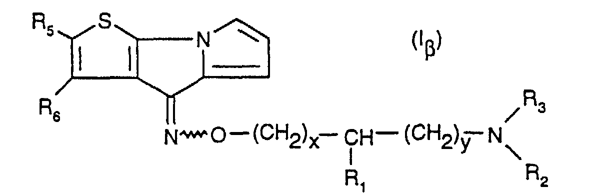 in der R₁, R₂, R₃, R₅, R₆, x und y die oben angegebenen Bedeutungen besitzen,
* oder eine Gruppe der Formel (γ) bedeutet: 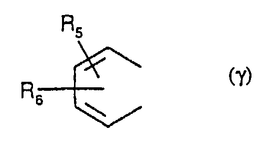 und in dieser Weise mit dem sie tragenden heterocyclischen System ein Pyrrolo[1,2-a]indol der allgemeinen Formel (I_{γ}] bildet: 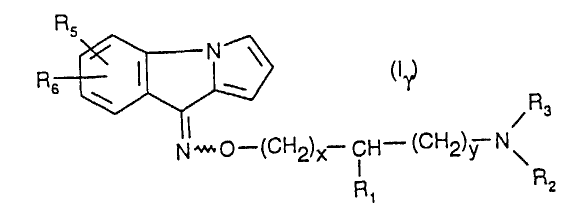 in der R₁, R₂, R₃, R₅, R₆, x und y die oben angegebenen Bedeutungen besitzen,
- deren cis- oder trans-Isomere im Bereich des Oximethers,
- deren Enantiomere oder Diastereoisomere und
- deren Hydrate und/oder deren pharmazeutisch annehmbare Additionssalze mit einer Säure oder Base,
wobei es sich versteht, wenn nichts anderes angegeben ist, daß:
- die Begriffe "Alkyl" und "Alkoxy" geradkettige oder verzweigte Gruppen mit 1 bis 6 Kohlenstoffatomen darstellen,
- der Begriff "Alkenyl" eine ungesättigte geradkettige oder verzweigte Kohlenstoff-haltige Gruppe mit 2 bis 6 Kohlenstoffatomen darstellt,
- der Begriff "Cycloalkyl" ein cyclisches Kohlenstoff-haltiges System darstellt mit 3 bis 8 Kettengliedern,
- die Begriffe "gegebenenfalls substituiertes Indanyl", "gegebenenfalls substituiertes Phenyl", "gegebenenfalls substituiertes Phenoxy", "gegebenenfalls substituiertes Phenylalkyl", "gegebenenfalls substituiertes -(CH₂]_{σ}-Phenyl", "gegebenenfalls substituiertes -(CH₂)ₚ-Phenyl", "gegebenenfalls substituiertes -O-(CH₂)_{p'}-Phenyl" und "gegebenenfalls substituiertes Phenylalkoxy" bedeuten, daß der Phenylrest gegebenenfalls durch einen oder mehrere gleichartige oder verschiedene Substituenten ausgewählt aus Alkyl, Alkoxy, Hydroxy, Halogen, Trifluormethyl, Nitril und Nitro substituiert sein kann.

2. Verbindungen nach Anspruch 1, worin A eine Gruppe der Formel (α) darstellt: 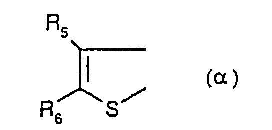 und in dieser Weise mit dem sie tragenden heterocyclischen System ein Thieno[2,3-d]pyrrolo[1,2-a]pyrrol der allgemeinen Formel (I_{α} ) bildet: 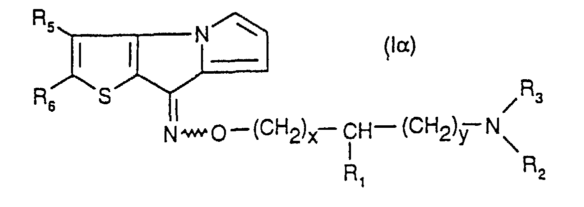 in der R₁, R₂, R₃, x und y die in Anspruch 1 angegebenen Bedeutungen besitzen und R₅ und R₆, die gleichartig oder verschieden sein können, unabhängig voneinander eine Gruppe darstellen ausgewählt aus:
- Wasserstoff,
- Alkyl,
- Hydroxy,
- Alkoxy,
- Halogen,
- Trifluormethyl,
- Alkoxycarbonyl,
- gegebenenfalls substituiertem -(CH₂)ₚ-Phenyl, worin p für eine ganze Zahl mit Werten von 0, 1, 2, 3 oder 4 steht,
- gegebenenfalls substituiertem -O-(CH₂)_{p'}-Phenyl, worin p' für eine ganze Zahl mit Werten von 0, 1, 2, 3 oder 4 steht,
- deren cis- oder trans-Isomere im Bereich des Oximethers,
- deren Enantiomere oder Diastereoisomere und
- deren Hydrate und/oder deren pharmazeutisch annehmbare Additionssalze mit einer Säure oder Base,
wobei es sich versteht, daß, wenn nichts anderes angegeben ist,
- die Begriffe "Alkyl" und "Alkoxy" für geradkettige oder verzweigte Gruppen mit 1 bis 6 Kohlenstoffatomen stehen,
- die Begriffe "gegebenenfalls substituiertes -(CH₂)ₚ-Phenyl" und "gegebenenfalls substituiertes -O-(CH₂)_{p'}-Phenyl" bedeuten, daß der Phenylrest gegebenenfalls durch einen oder mehrere gleichartige oder verschiedene Substituenten ausgewählt aus Alkyl, Alkoxy, Hydroxy, Halogen, Trifluormethyl, Nitril und Nitro substituiert sein kann.

3. Verbindungen nach Anspruch 1, worin A eine Gruppe der Formel (β) darstellt: 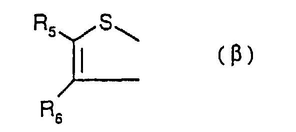 und in dieser Weise mit dem sie tragenden heterocyclischen System ein Thieno[3,2-d]pyrrolo[1,2-a]pyrrol der allgemeinen Formel (I_{β} ) bildet: 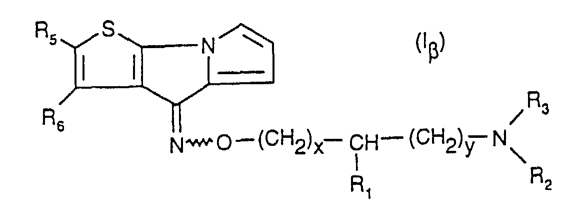 in der R₁, R₂, R₃, x und y die in Anspruch 1 angegebenen Bedeutungen besitzen und R₅ und R₆, die gleichartig oder verschieden sein können, unabhängig voneinander eine Gruppe darstellen ausgewählt aus:
- Wasserstoff,
- Alkyl,
- Hydroxy,
- Alkoxy,
- Halogen,
- Trifluormethyl,
- Alkoxycarbonyl,
- gegebenenfalls substituiertem -(CH₂)ₚ-Phenyl, worin p für eine ganze Zahl mit Werten von 0, 1, 2, 3 oder 4 steht,
- gegebenenfalls substituiertem -O-(CH₂)_{p'}-Phenyl, worin p' für eine ganze Zahl mit Werten von 0, 1, 2, 3 oder 4 steht,
- deren cis- oder trans-Isomere im Bereich des Oximethers,
- deren Enantiomere oder Diastereoisomere und
- deren Hydrate und/oder deren pharmazeutisch annehmare Additionssalze mit einer Säure oder Base,
wobei es sich versteht, daß, wenn nichts anderes angegeben ist,
- die Begriffe "Alkyl" und "Alkoxy" für geradkettige oder verzweigte Gruppen mit 1 bis 6 Kohlenstoffatomen stehen,
- die Begriffe "gegebenenfalls substituiertes -(CH₂]ₚ-Phenyl" und "gegebenenfalls substituiertes -O-(CH₂)_{p'}-Phenyl" bedeuten, daß der Phenylrest gegebenenfalls durch einen oder mehrere gleichartige oder verschiedene Substituenten ausgewählt aus Alkyl, Alkoxy, Hydroxy, Halogen, Trifluormethyl, Nitril und Nitro substituiert sein kann.

4. Verbindungen nach Anspruch 1, worin A eine Gruppe der Formel (γ) darstellt: 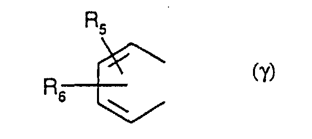 und in dieser Weise mit dem sie tragenden heterocyclischen System ein Pyrrolo[1,2-a]indol der allgemeinen Formel (I_{γ}) bildet: 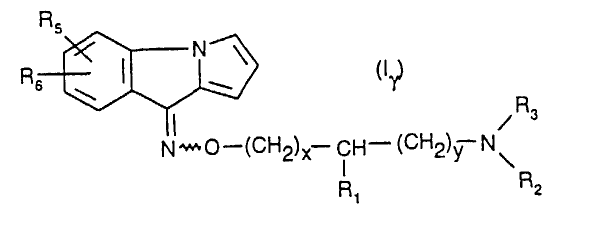 in der R₁, R₂, R₃, x und y die in Anspruch 1 angegebenen Bedeutungen besitzen und R₅ und R₆, die gleichartig oder verschieden sein können, unabhängig voneinander eine Gruppe bedeuten ausgewählt aus:
- Wasserstoff,
- Alkyl,
- Hydroxy,
- Alkoxy,
- Halogen,
- Trifluormethyl,
- Alkoxycarbonyl,
- gegebenenfalls substituiertem -(CH₂)ₚ-Phenyl, worin p für eine ganze Zahl mit Werten von 0, 1, 2, 3 oder 4 steht,
- gegebenenfalls substituiertem -O-(CH₂)_{p'}-Phenyl, worin p' für eine ganze Zahl mit Werten von 0, 1, 2, 3 oder 4 steht,
- deren cis- oder trans-Isomere im Bereich des Oximethers,
- deren Enantiomere oder Diastereoisomere und
- deren Hydrate und/oder deren pharmazeutisch annehmbare Additionssalze mit einer Säure oder Base,
wobei es sich versteht, daß, wenn nichts anderes angegeben ist,
- die Begriffe "Alkyl" und "Alkoxy" für geradkettige oder verzweigte Gruppen mit 1 bis 6 Kohlenstoffatomen stehen,
- die Begriffe "gegebenenfalls substituiertes -(CH₂)ₚ-Phenyl" und "gegebenenfalls substituiertes -O-(CH₂)_{p'}-Phenyl" bedeuten, daß der Phenylrest gegebenenfalls durch einen oder mehrere gleichartige oder verschiedene Substituenten ausgewählt aus Alkyl, Alkoxy, Hydroxy, Halogen, Trifluormethyl, Nitril und Nitro substituiert sein kann.

5. Verbindung nach irgendeinem der Ansprüche 1 und 2, nämlich 3-Methyl-8-{[3-(N,N-dimethylamino)-1-phenyl-n-prop-1-yloxy]-imino}-thieno[2,3-d]pyrrolo[1,2-a]pyrrol der nachstehenden Formel 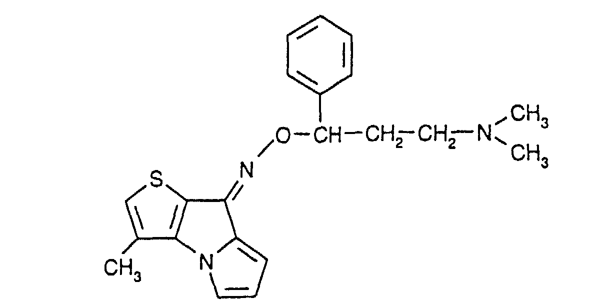 sowie deren Z- und E-Isomere und deren Enantiomere in isolierter Form oder in Form einer Mischung sowie ihre pharmazeutisch annehmbaren Additionssalze mit einer Säure oder Base.

6. Verbindung nach irgendeinem der Ansprüche 1 und 2, nämlich 3-Methyl-8-{[2-(N-benzylamino)-ethyloxy]-imino}-thieno[2,3-d]pyrrolo[1,2-a]pyrrol der nachstehenden Formel 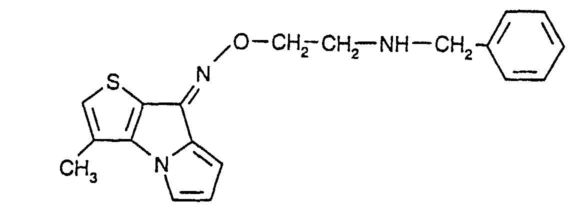 sowie deren Z- und E-Isomere in isolierter Form oder in Form einer Mischung und deren pharmazeutisch annehmbaren Additionssalze mit einer Säure.

7. Verbindung nach irgendeinem der Ansprüche 1 und 2, nämlich 3-Methyl-8-{[2-(N,N-dimethylamino)-n-prop-1-yloxy]-imino}-thieno[2,3-d]pyrrolo[1,2-a]pyrrol der nachstehenden Formel 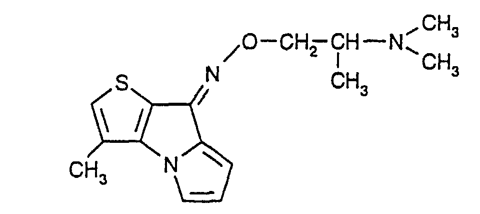 sowie deren Z- und E-Isomere und deren Enantiomere in isolierter Form oder in Form einer Mischung und deren pharmazeutisch annehmbaren Additionssalze mit einer Säure.

8. Verbindung nach irgendeinem der Ansprüche 1 und 3, nämlich 8-{[2-(N,N-Dimethylamino)-ethyloxy]-imino}-thieno(3,2-d]pyrrolo[1,2-a]pyrrol der nachstehenden Formel 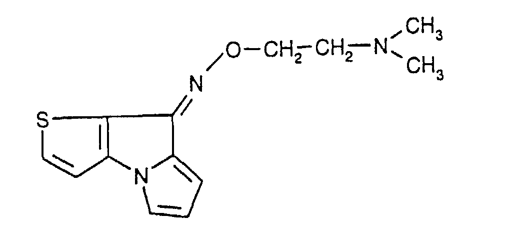 sowie deren Z- und E-Isomere in isolierter Form oder in Form einer Mischung und deren pharmazeutisch annehmbare Additionssalze mit einer Säure.

9. Verbindung nach irgendeinem der Ansprüche 1 und 4, nämlich 9-{[2-(N,N-Dimethylamino)-n-prop-1-yloxy]-imino}-pyrrolo[1,2-a]indol der nachstehenden Formel 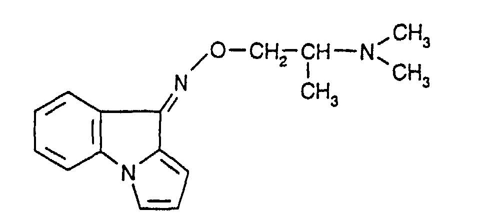 sowie deren Z- und E-Isomere und deren Enantiomere in isolierter Form oder in Form einer Mischung und deren pharmazeutisch annehmbare Additionssalze mit einer Säure.

10. Verfahren zur Herstellung der Verbindungen der Formel (I) nach Anspruch 1, dadurch gekennzeichnet, daß man eine Verbindung der Formel (II): 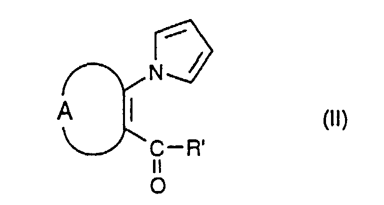 in der A die in Anspruch 1 angegebenen Bedeutungen besitzt und R' eine Hydroxygruppe, eine Alkoxygruppe oder eine Gruppe der Formel 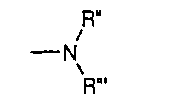 darstellt, worin R" und R"', die von H verschieden sind, Alkylgruppen darstellen können oder gemeinsam mit dem sie tragenden Stickstoffatom ein cyclisches Amin ausgewählt aus Pyrrolidin, Piperidin oder Morpholin bilden können, in Gegenwart oder in Abwesenheit eines Lösungsmittels mit Phosphoroxidchlorid cyclisiert, so daß man nach der Cyclisierung ein tricyclisches Keton der Formel (III) erhält: 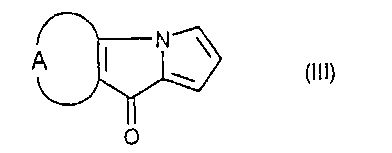 in der A die oben angegebenen Bedeutungen besitzt, welches man anschließend mit Hydroxylamin umsetzt, so daß man das Oxim der Formel (IV) erhält: 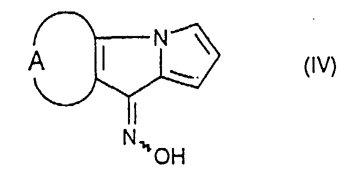 in der A die oben angegebenen Bedeutungen besitzt, welches man erforderlichenfalls und gewünschtenfalls in die E- und Z-Isomeren auftrennen kann, bevor man es mit einer Verbindung der Formel (V) umsetzt: 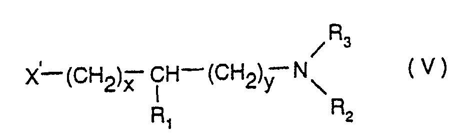 in der R₁, R₂, R₃, x und y die in Anspruch 1 angegebenen Bedeutungen besitzen und X' eine austretende Gruppe darstellt, so daß man die Verbindung der Formel (I) erhält: 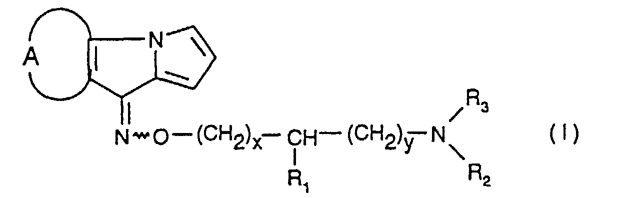 in der A, R₁, R₂, R₃, x und y die oben angegebenen Bedeutungen besitzen, welche man erforderlichenfalls und gewünschtenfalls in die Z- und E-Isomeren und in ihre eventuellen Enantiomeren und/oder Diastereoisomeren auftrennen und/oder mit einer Säure oder Base in ihre pharmazeutisch annehmbaren Salze überführen kann.

11. Verfahren zur Herstellung der Verbindungen der Formel (I) nach Anspruch 1, dadurch gekennzeichnet, daß man eine Verbindung der Formel (II): 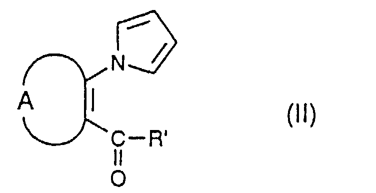 in der A die in Anspruch 1 angegebenen Bedeutungen besitzt und R' eine Hydroxygruppe, eine Alkoxygruppe oder eine Gruppe der Formel 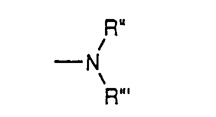 darstellt, worin R" und R"', die von H verschieden sind, Alkylgruppen darstellen können oder gemeinsam mit dem sie tragenden Stickstoffatom ein cyclisches Amin bilden können ausgewählt aus Pyrrolidin, Piperidin oder Morpholin, in Gegenwart oder in Abwesenheit eines Lösungsmittels mit Phosphoroxidchlorid umsetzt, so daß man nach der Cyclisierung ein tricyclisches Keton der Formel (III) erhält: 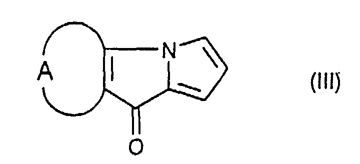 in der A die oben angegebenen Bedeutungen besitzt, welches man anschließend mit einem Amin der allgemeinen Formel (VI) umsetzt: 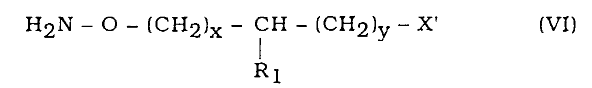 in der R₁, x und y die in Anspruch 1 angegebenen Bedeutungen besitzen und X' eine austretende Gruppe darstellt, so daß man eine Verbindung der Formel (VII) erhält: 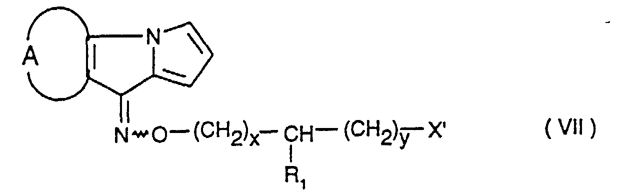 in der A, R₁, X', x und y die oben angegebenen Bedeutungen besitzen, welche man:
a) entweder mit einem Amin der allgemeinen Formel (VIII): 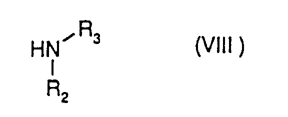 in der R₂ und R₃ de in Anspruch 1 angegebenen Bedeutungen besitzen, umsetzen kann, so daß man die Verbindung der Formel (I) erhält: 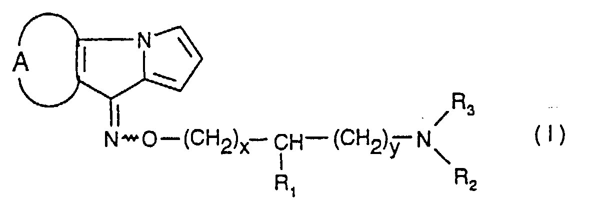 in der A, R₁, R₂, R₃, x und y die oben angegebenen Bedeutungen besitzen, welche man erforderlichenfalls oder gewünschtenfalls in die Z- und E-Isomeren und ihre eventuellen Enantiomeren oder Diastereoisomeren auftrennen und/oder mit einer Säure oder Base in ihre pharmazeutisch annehmbaren Salze umwandeln kann,
b) oder mit Kaliumphthalimid umsetzen kann, so daß man das Phthalimid der Formel (IX) erhält: 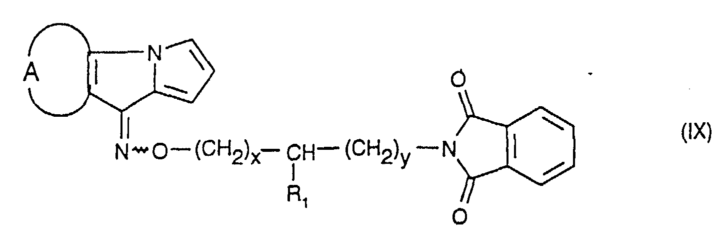 in der A, R₁, x und y die oben angegebenen Bedeutungen besitzen, welches man anschließend in Gegenwart von Hydrazin hydrolysiert zur Bildung der Verbindung der Formel (I_{A}): 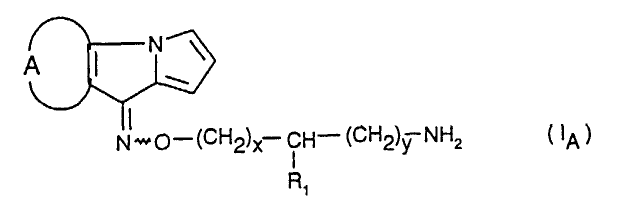 in der A, R₁, x und y die oben angegebenen Bedeutungen besitzen, einem Sonderfalls der Verbindungen der Formel (I), worin R₂ = R₃ = H, welche man erforderlichenfalls und gewünschtenfalls in die Z- und E-Isomeren und ihre eventuellen Enantiomeren oder Diastereoisomeren trennen und/oder mit einer Säure oder Base in ihre pharmazeutisch annehmbaren Salze umwandeln kann,
welche Verbindung der allgemeinen Formel (I_{A}) man gewünschtenfalls mit einer Verbindung der Formel (X):
X' - R₅ (X)
in der X' die oben angegebenen Bedeutungen besitzt und R₅ die gleiche Bedeutung besitzt wie R₂ mit der Ausnahme, daß R₅ weder Wasserstoff noch gegebenenfalls substituiertes Phenyl darstellt, alkylieren kann, so daß man die Verbindung der Formel (I_{B}) erhält: 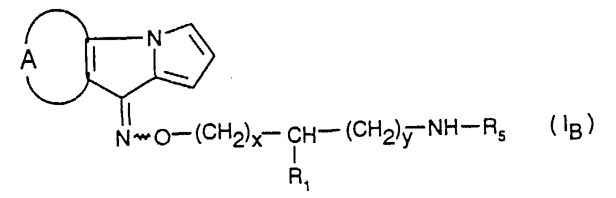 in der A, R₁, R₅, x und y die oben angegebenen Bedeutungen besitzen, einem Sonderfall der Verbindungen der Formel (I), die man erforderlichenfalls und gewünschtenfalls in die Z- und E-Isomeren und ihre eventuellen Enantiomeren oder Diastereoisomeren auftrennen und/oder mit einer Säure oder Base in die pharmazeutisch annehbmaren Salze umwandeln kann.

12. Verfahren zur Herstellung der Verbindungen der Formel (I_{C}): 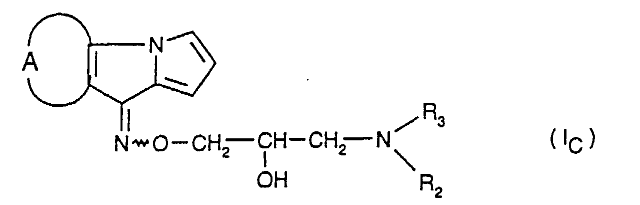 einem Sonderfall der Verbindungen der allgemeinen Formel (I) nach Anspruch 1, worin A, R₂ und R₃ die in Anspruch 1 angegebenen Bedeutungen besitzen, worin x = y = 1 und R₁ eine Hydroxygruppe darstellen, dadurch gekennzeichnet, daß man eine Verbindung der Formel (II): 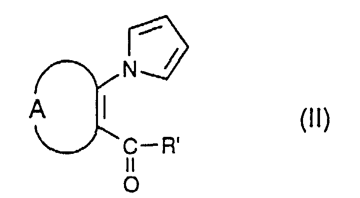 in der A die oben angegebenen Bedeutungen besitzt und R' eine Hydroxygruppe, eine Alkoxygruppe oder eine Gruppe der Formel: 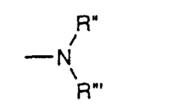 darstellt, worin R" und R"', die von H verschieden sind, Alkylgruppen bedeuten oder gemeinsam mit dem sie tragenden Stickstoffatom ein cyclisches Amin ausgewählt aus Pyrrolidin, Piperidin oder Morpholin bilden,
in Gegenwart oder in Abwesenheit eines Lösungsmittels mit Phosphoroxidchlorid cyclisiert, so daß man nach der Cyclisierung ein tricyclisches Keton der Formel (III) erhält: 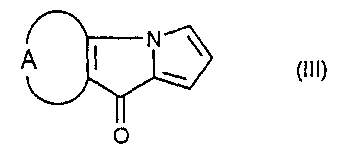 in der A die oben angegebenen Bedeutungen besitzt, welches man anschließend mit Hydroxylamin umsetzt, so daß man das Oxim der allgemeinen Formel (IV) erhält: 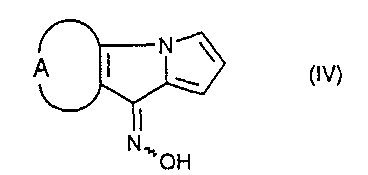 in der A die oben angegebenen Bedeutungen besitzt, und welches man erforderlichenfalls und gewünschtenfalls in die E- und Z-Isomeren auftrennen kann, bevor man es nach der Metallierung mit einem Metallhydrid, mit einem Epihalogenhydrin der Formel (XI): 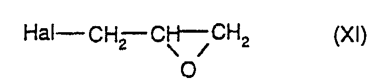 in der HaI ein Halogenatom darstellt, umsetzen kann, so daß man nach der Reaktion der in dieser Weise gebildeten Zwischenverbindung mit einem Amin der Formel (VIII): 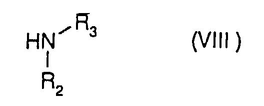 in der R₂ und R₃ die oben angegebenen Bedeutungen besitzen, eine Verbindung der oben definierten Formel (I_{C}) erhält, welche man erforderlichenfalls und gewünschtenfalls in die Z- und E-Isomeren und die eventuellen Enantiomeren oder Diastereoisomeren auftrennen und/oder mit einer Säure oder Base in die pharmazeutisch annehbmaren Salze umwandeln kann.

13. Pharmazeutische Zubereitung enthaltend als Wirkstoff mindestens eine Verbindung der Formel (I) nach Anspruch 1 in reiner Form oder in Form einer Mischung der Isomeren oder eines ihrer pharmazeutisch annehmbaren Additionssalze mit einer Säure oder einer Base in Kombination mit einem oder mehreren pharmazeutisch annehmbaren Trägermaterialien oder Bindemitteln.

14. Pharmazeutische Zubereitung nach Anspruch 13 zur Vorbeugung und Behandlung von Angst, Depressionen, Stress, Psychosen, zwanghaften Triebstörungen der Schizophrenie, Störungen des Zentralnervensystems, der Migräne, Gedächtnisstörungen, Verhaltensstörungen und Störungen der Nahrungsaufnahme, Alkoholismus, Schmerzen sowie zur Vorbeugung und Behandlung des Erbrechens und von Störungen der Entleerung des Magen-Darm-Trakts.

## Claims

1. Compounds of the general formula (I): 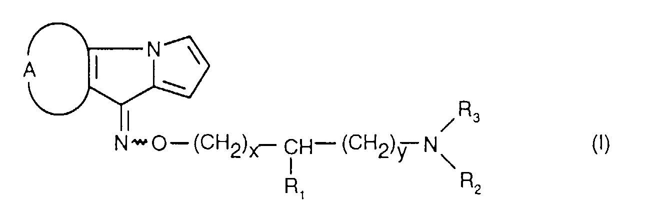 wherein
• R₁ is selected from :
- hydrogen,
- alkyl,
- alkenyl,
- cycloalkyl,
- cycloalkylalkyl of which the straight or branched alkyl chain has from 1 to 4 carbon atoms,
- hydroxy,
- alkoxy,
- optionally substituted phenyl,
- optionally substituted phenylalkyl of which the straight or branched alkyl chain has from 1 to 4 carbon atoms,
- optionally substituted phenoxy, and
- optionally substituted phenylalkoxy of which the straight or branched alkyl chain has from 1 to 4 carbon atoms,
or R₁ forms with R₂ and the chain 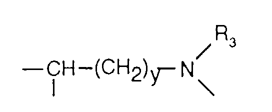 carrying them a nitrogen-containing ring system having from 5 to 8 ring members,
• R₂ and R₃ are each selected, independently of the other, from :
- hydrogen,
- alkyl,
- alkenyl,
- cycloalkyl,
- optionally substituted indanyl,
- cycloalkylalkyl of which the straight or branched alkyl chain has from 1 to 4 carbon atoms,
- optionally substituted phenyl, and
- optionally substituted phenylalkyl of which the straight or branched alkyl chain has from 1 to 4 carbon atoms,
or R₂ and R₃ form together with the nitrogen atom carrying them a heterocyclic system selected from: 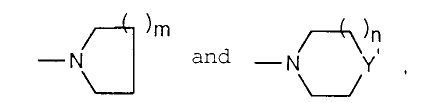 wherein :
m is an integer of which the value may be 0, 1, 2, 3, or 4,
n is an integer of which the value may be 0, 1, or 2,
Y' is selected from oxygen, sulphur and the group
〉N-R₄ ,
R₄ being selected from :
- hydrogen,
- alkyl, and
- optionally substituted -(CH₂)_{σ}-phenyl, wherein σ is an integer of which the value may be 0, 1, 2, 3, or 4,
• x and y represent identical or different integers of which the values may each, independently of the other, be 0, 1, 2, 3, or 4,
• A represents :
* a group of formula (α) : 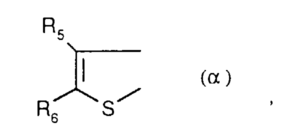 and thus forms with the heterocyclic system carrying it a thieno[2,3-d]pyrrolo[1,2-a]pyrrole of the general formula (I_{α}) : 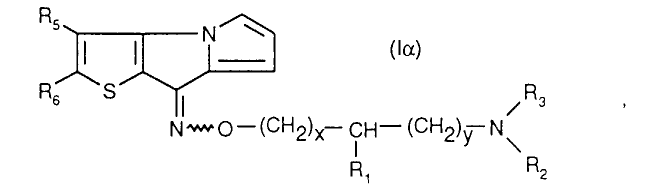 wherein R₁, R₂, R₃, x, and y are as defined hereinbefore and R₅ and R₆, which are identical or different, each represents, independently of the other, a group selected from :
- hydrogen,
- alkyl,
- hydroxy,
- alkoxy,
- halogen,
- trifluoromethyl,
- alkoxycarbonyl,
- optionally substituted -(CH₂)ₚ-phenyl wherein p is an integer of which the value may be 0, 1, 2, 3, or 4, and
- optionally substituted -O-(CH₂)ₚ'-phenyl wherein p' is an integer of which the value may be 0, 1, 2, 3, or 4,
* a group of formula (β) : 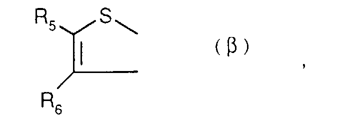 and thus forms with the heterocyclic system carrying it a thieno[3,2-d]pyrrolo[1,2-a]pyrrole of the general formula (I_{β}) : 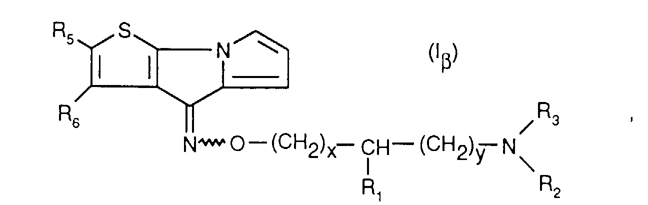 wherein R₁, R₂, R₃, R₅, R₆, x and y are as defined hereinbefore,
* a group of formula (γ) : 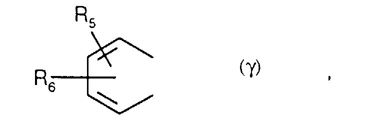 and thus forms with the heterocyclic system carrying it a pyrrolo[1,2-a]indole of the general formula (Iγ) : 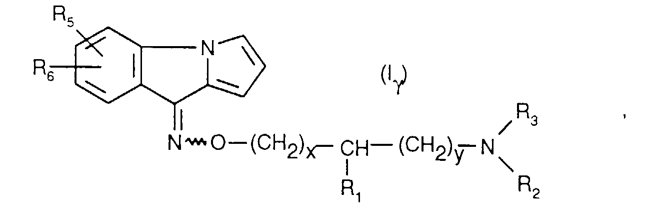 wherein R₁, R₂, R₃, R₅, R₆, x and y are as defined hereinbefore,
- their cis or trans isomers in respect of the oxime ether,
- enantiomers or diastereoisomers thereof, and
- hydrates thereof and/or pharmaceutically acceptable addition salts thereof with an acid or a base,
it being understood, unless specified otherwise, that:
- the terms "alkyl" and "alkoxy" represent straight-chain or branched groups having from 1 to 6 carbon atoms,
- the term "alkenyl" represents a straight-chain or branched unsaturated carbon-containing group having from 2 to 6 carbon atoms,
- the term "cycloalkyl" represents a carbon ring system having from 3 to 8 ring members,
- the expressions "optionally substituted indanyl", "optionally substituted phenyl", "optionally substituted phenoxy", "optionally substituted phenylalkyl", "optionally substituted -(CH₂)_{σ}-phenyl", "optionally substituted -(CH₂)ₚ-phenyl", "optionally substituted -O-(CH₂)ₚ'-phenyl" and "optionally substituted phenylalkoxy" denote that the phenyl radical may optionally be substituted by one or more identical or different substituents selected from alkyl, alkoxy, hydroxy, halogen, trifluoromethyl, nitrile and nitro.

2. Compounds according to claim 1 wherein A represents a group of formula (α) : 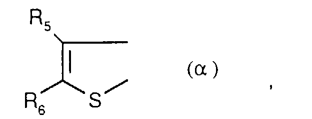 and thus forms with the heterocyclic system carrying it a thieno[2,3-d]pyrrolo[1,2-a]pyrrole of the general formula (I_{α}) : 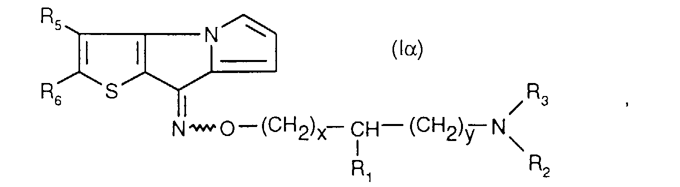 wherein R₁, R₂, R₃, x, and y are as defined in claim 1 and R₅ and R₆, which are identical or different, each represents, independently of the other, a group selected from :
- hydrogen,
- alkyl,
- hydroxy,
- alkoxy,
- halogen,
- trifluoromethyl,
- alkoxycarbonyl,
- optionally substituted -(CH₂)ₚ-phenyl wherein p is an integer of which the value may be 0, 1, 2, 3, or 4, and
- optionally substituted -O-(CH₂)ₚ'-phenyl wherein p' is an integer of which the value may be 0, 1, 2, 3, or 4,
- their cis or trans isomers in respect of the oxime ether,
- enantiomers or diastereoisomers thereof, and
- hydrates thereof and/or pharmaceutically acceptable addition salts thereof with an acid or a base,
it being understood, unless specified otherwise, that:
- the terms "alkyl" and "alkoxy" represent straight-chain or branched groups having from 1 to 6 carbon atoms,
- the expressions "optionally substituted -(CH₂)ₚ-phenyl" and "optionally substituted -O-(CH₂)ₚ'-phenyl" denote that the phenyl radical may optionally be substituted by one or more identical or different substituents selected from alkyl, alkoxy, hydroxy, halogen, trifluoromethyl, nitrile and nitro.

3. Compounds according to claim 1 wherein A represents a group of formula (β) : 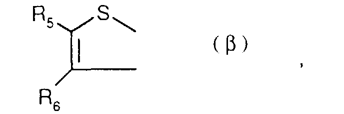 and thus forms with the heterocyclic system carrying it a thieno[3,2-d]pyrrolo[1,2-a]pyrrole of the general formula (I_{β}) : 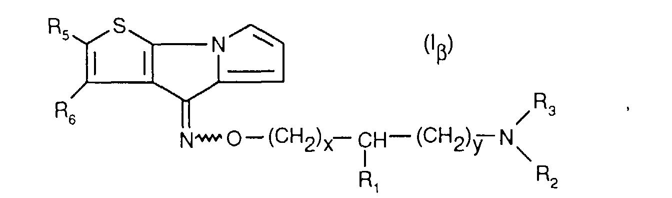 wherein R₁, R₂, R₃, x and y are as defined in claim 1 and R₅ and R₆, which are identical or different, each represents, independently of the other, a group selected from :
- hydrogen,
- alkyl,
- hydroxy,
- alkoxy,
- halogen,
- trifluoromethyl,
- alkoxycarbonyl,
- optionally substituted -(CH₂)ₚ-phenyl wherein p is an integer of which the value may be 0, 1, 2, 3, or 4, and
- optionally substituted -O-(CH₂)ₚ'-phenyl wherein p' is an integer of which the value may be 0, 1, 2, 3, or 4,
- their cis or trans isomers in respect of the oxime ether,
- enantiomers or diastereoisomers thereof, and
- hydrates thereof and/or pharmaceutically acceptable addition salts thereof with an acid or a base,
it being understood, unless specified otherwise, that :
- the terms "alkyl" and "alkoxy" represent straight-chain or branched groups having from 1 to 6 carbon atoms,
- the expressions "optionally substituted -(CH₂)ₚ-phenyl" and "optionally substituted -O-(CH₂)ₚ'-Phenyl" denote that the phenyl radical may optionally be substituted by one or more identical or different substituents selected from alkyl, alkoxy, hydroxy, halogen, trifluoromethyl, nitrile and nitro.

4. Compounds according to claim 1 wherein A represents a group of formula (γ) : 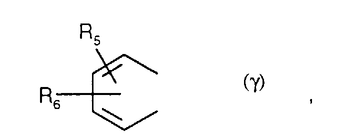 and forms with the heterocyclic system carrying it a pyrrolo[1,2-a]indole of the general formula (Iγ) : 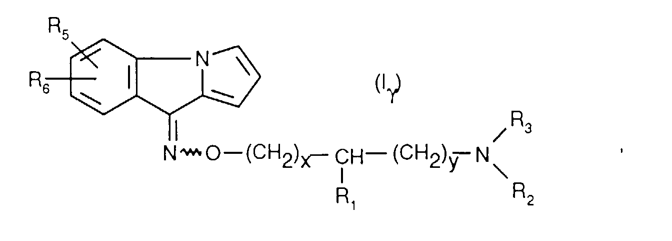 wherein R₁, R₂, R₃, x, and y are as defined in claim 1 and R₅ and R₆, which are identical or different, each represents, independently of the other, a group selected from :
- hydrogen,
- alkyl,
- hydroxy,
- alkoxy,
- halogen,
- trifluoromethyl,
- alkoxycarbonyl,
- optionally substituted -(CH₂)ₚ-phenyl wherein p is an integer of which the value may be 0, 1, 2, 3, or 4, and
- optionally substituted -O-(CH₂)ₚ'-phenyl wherein p' is an integer of which the value may be 0, 1, 2, 3, or 4,
- their cis or trans isomers in respect of the oxime ether,
- enantiomers or diastereoisomers thereof, and
- hydrates thereof and/or pharmaceutically acceptable addition salts thereof with an acid or a base,
it being understood, unless specified otherwise, that:
- the terms "alkyl" and "alkoxy" represent straight-chain or branched groups having from 1 to 6 carbon atoms,
- the expressions "optionally substituted -(CH₂)ₚ-phenyl" and "optionally substituted -O-(CH₂)ₚ'-phenyl" denote that the phenyl radical may optionally be substituted by one or more identical or different substituents selected from alkyl, alkoxy, hydroxy, halogen, trifluoromethyl, nitrile and nitro.

5. Compound according to either claim 1 or claim 2, which is 3-methyl-8-{[3-(N,N-dimethylamino)-1 -phenyl-n-prop-1-yloxy]imino}thieno[2,3-d]pyrrolo[1,2-a]pyrrole for which the formula is shown below, and also its Z and E isomers and its enantiomers, isolated or in the form of a mixture, and pharmaceutically acceptable addition salts thereof with an acid.

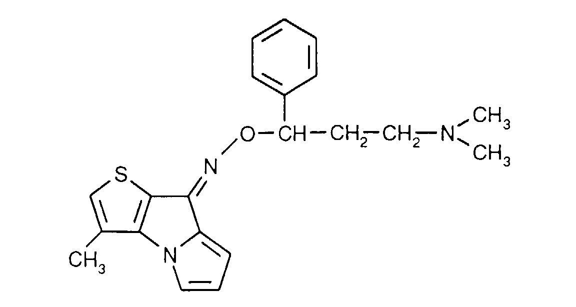

6. Compound according to either claim 1 or claim 2 which is 3-methyl-8-{[2-(N-benzylamino)ethoxy]imino}thieno[2,3-d]pyrrolo[1,2-a]pyrrole for which the formula is shown below, and also its Z and E isomers, isolated or in the form of a mixture, and pharmaceutically acceptable addition salts thereof with an acid.

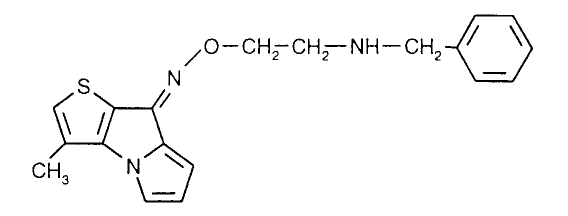

7. Compound according to either claim 1 or claim 2, which is 3-methyl-8-{[2-(N,N-dimethylamino)-n-prop-1-yloxy]imino}thieno[2,3-d]pyrrolo[1,2-a]pyrrole for which the formula is shown below, and also its Z and E isomers and its enantiomers, isolated or in the form of a mixture, and pharmaceutically acceptable addition salts thereof with an acid.

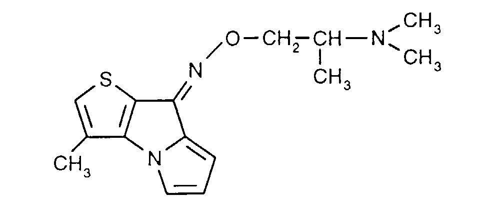

8. Compound according to either claim 1 or claim 3, which is 8-{[2-(N,N-dimethylamino)ethoxy]imino}thieno[3,2-d]pyrrolo[1,2-a]pyrrole for which the formula is shown below, and also its Z and E isomers, isolated or in the form of a mixture, and pharmaceutically acceptable addition salts thereof with an acid.

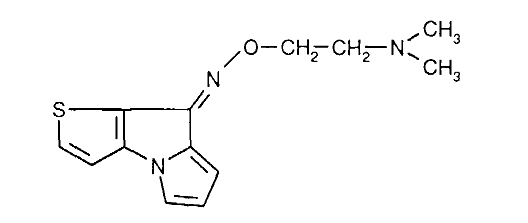

9. Compound according to either claim 1 or claim 4, which is 9-{[2-(N,N-dimethylamino)-n-prop-1-yloxy]imino}pyrrolo[1,2-a]indole for which the formula is shown below, and also its Z and E isomers and its enantiomers, isolated or in the form of a mixture, and pharmaceutically acceptable addition salts thereof with an acid.

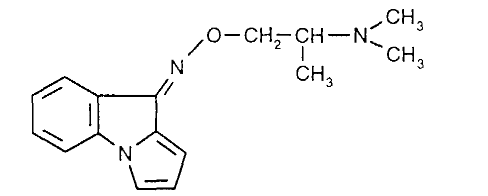

10. Process for the preparation of compounds of formula (I) according to claim 1, characterised in that a compound of formula (II) : 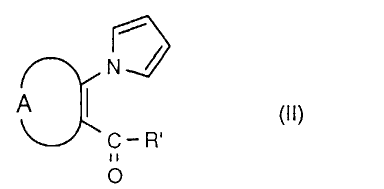 wherein A is as defined in claim 1 and R' represents a hydroxy, alkoxy or 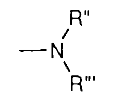 radical wherein R" and R"' are other than H and may be alkyls or may form together with the nitrogen atom carrying them a cyclic amine selected from pyrrolidine, piperidine and morpholine, is cyclised with phosphorus oxychloride in the presence or absence of a solvent,
to obtain, after cyclisation, a tricyclic ketone of formula (III) : 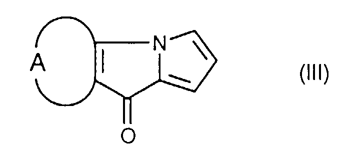 wherein A is as defined hereinbefore, which is then reacted with hydroxylamine to obtain an oxime of formula (IV) : 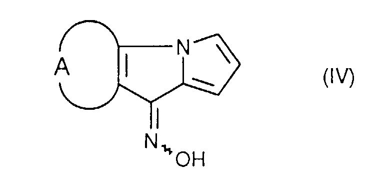 wherein A is as defined hereinbefore, which may, if necessary and if desired, be separated into its E and Z isomers before being reacted with a compound of formula (V) : 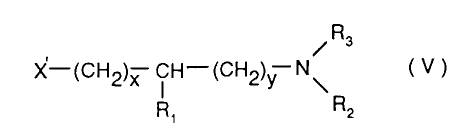 wherein R₁, R₂, R₃, x and y are as defined in claim 1 and X' represents a leaving group to yield a compound of formula (I): 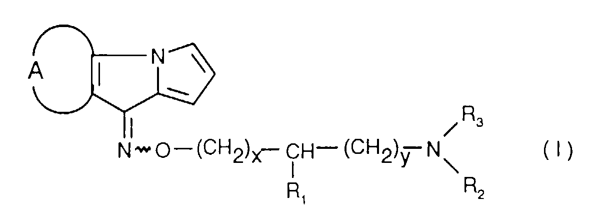 wherein A, R₁, R₂, R₃, x and y are as defined hereinbefore, which may, if necessary and if desired, be separated into its Z and E isomers and into its possible enantiomers or diastereoisomers, and/or be converted into a salt with a pharmaceutically acceptable acid or base.

11. Process for the preparation of compounds of formula (I) according to claim 1, characterised in that a compound of formula (II) : 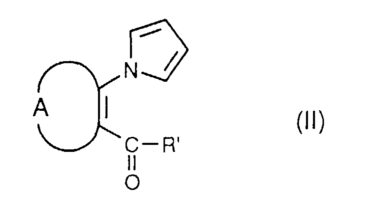 wherein A is as defined in claim 1 and R' represents a hydroxy, alkoxy or 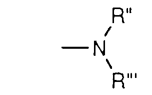 radical wherein R" et R"' are other than H and may represent alkyls or may form, together with the nitrogen atom carrying them, a cyclic amine selected from pyrrolidine, piperidine and morpholine, is cyclised with phosphorus oxychloride in the presence or absence of a solvent, to obtain, after cyclisation, a tricyclic ketone of formula (III) : 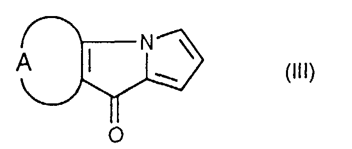 wherein A is as defined hereinbefore, which is then reacted with an amine of the general formula (VI): 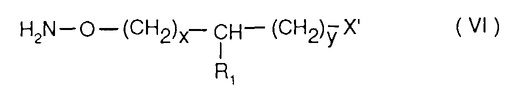 wherein R₁, x and y are as defined in claim 1 and X' represents a leaving group, to obtain a compound of formula (VII): 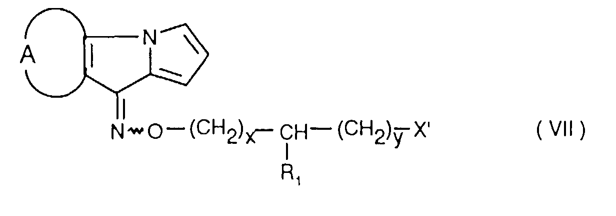 wherein A, R₁, X', x and y are as defined hereinbefore, which may :
a) either be reacted with an amine of the general formula (VIII) : 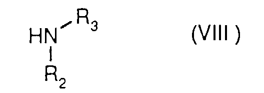 wherein R₂ and R₃ are as defined in claim 1, to yield a compound of formula (I) : 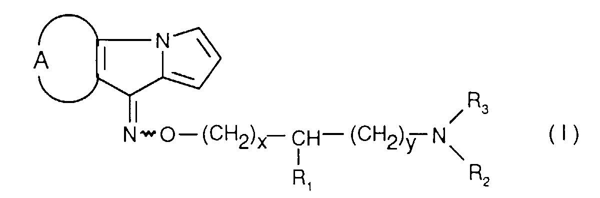 wherein A, R₁, R₂, R₃, x and y are as defined hereinbefore, which may, if necessary and if desired, be separated into its Z and E isomers and into its possible enantiomers or diastereoisomers and/or be converted with an acid or a base into pharmaceutically acceptable salts,
b) or be reacted with potassium phthalimide to obtain a phthalimide of formula (IX) : 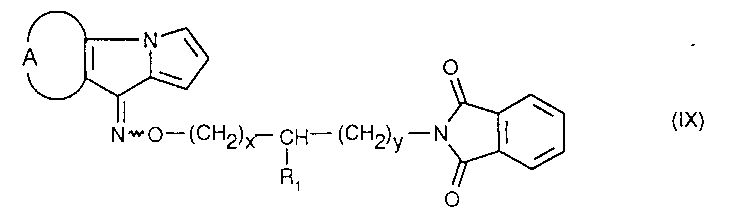 wherein A, R₁, x and y are as defined hereinbefore, which is then hydrolysed in the presence of hydrazine to yield a compound of formula (I_{A}) : 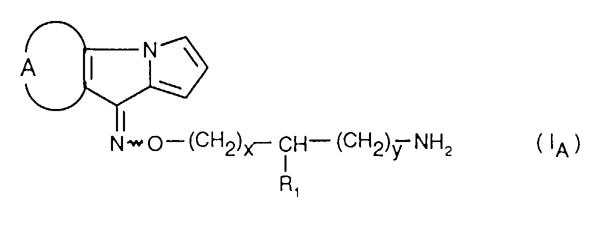 wherein A, R₁, x and y are as defined hereinbefore, a particular case of compounds of formula (I) wherein R₂=R₃=H, which may, if necessary and if desired, be separated into its Z and E isomers and into its possible enantiomers or diastereoisomers, and/or be converted with an acid or a base into pharmaceutically acceptable salts,
which compound of general formula (I_{A}) may, if desired, be alkylated with a compound of formula (X) :
X'-R₅ (X)
wherein X' is as defined hereinbefore and R₅ has the same meaning as R₂, with the proviso that R₅ cannot represent either hydrogen or optionally substituted phenyl, to yield a compound of formula (I_{B}) : 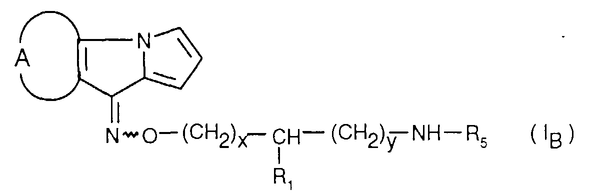 wherein A, R₁, R₅, x and y are as defined hereinbefore, a particular case of compounds of formula (I) which may, if necessary and if desired, be separated into its Z and E isomers and into its possible enantiomers or diastereoisomers and/or be converted with an acid or a base into pharmaceutically acceptable salts.

12. Process for the preparation of compounds of formula (I_{C}) : 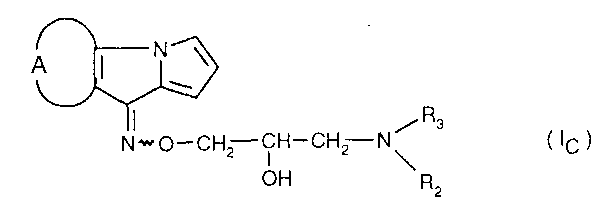 a particular case of compounds of the general formula (I) according to claim 1 wherein A, R₂ and R₃ are as defined in claim 1 with x=y=1 and R₁ representing a hydroxy group, characterised in that a compound of formula (II) : 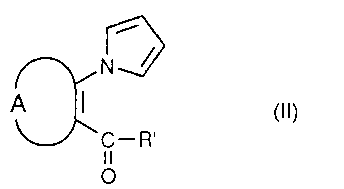 wherein A is as defined hereinbefore and R' represents a hydroxy, alkoxy or 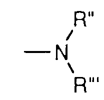 radical wherein R" and R''' are other than H and may be alkyls or may form together, with the nitrogen atom carrying them, a cyclic amine selected from pyrrolidine, piperidine and morpholine, is cyclised with phosphorus oxychloride in the presence or absence of a solvent,
to yield, after cyclisation, a tricyclic ketone of formula (III) : 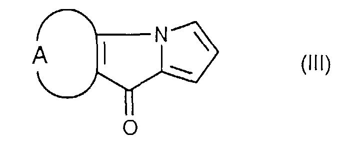 wherein A is as defined hereinbefore, which is then reacted with hydroxylamine to obtain an oxime of the general formula (IV) : 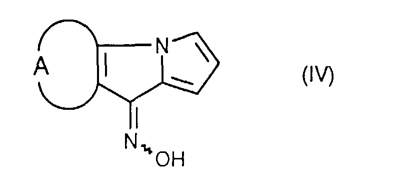 wherein A is as defined hereinbefore, which may, if necessary and if desired, be separated into its E and Z isomers before being reacted, after metallation with a metallic hydride, with an epihalohydrin of formula (XI) : 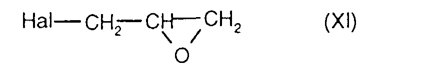 wherein Hal represents a halogen atom, to yield, after reaction of the so-formed intermediate compound with an amine of formula (VIII) : 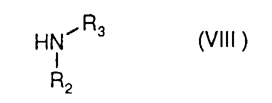 wherein R₂ and R₃ are as defined hereinbefore, a compound of formula (I_{C}) as defined hereinbefore, which may, if necessary and if desired, be separated into its Z and E isomers and into its possible enantiomers or diastereoisomers, and/or be converted with an acid or a base into pharmaceutically acceptable salts.

13. Pharmaceutical composition comprising as active ingredient at least one of the compounds of formula (I) according to claim 1, in pure form or in the form of a mixture of isomers, or one of the pharmaceutically acceptable addition salts thereof with an acid or a base, in combination with one or more pharmaceutically acceptable excipients or carriers.

14. Pharmaceutical composition according to claim 13, for use in the- prevention and treatment of anxiety, depression, stress, psychoses, obsessive-compulsive disorders of schizophrenia, disorders of the central nervous system, migraine, memory disorders, eating behaviour disorders, alcoholism and pain, and also in the prevention and treatment of vomiting and disorders of gastric emptying.
